(19) 

Europäisches Patentamt

European Patent Office

Office européen des brevets

(11) **EP 3 981 419 B1**

(12) **EUROPEAN PATENT SPECIFICATION**

(45) Date of publication and mention of the grant of the patent:
**24.06.2026 Bulletin 2026/26**

(21) Application number: **20200721.7**

(22) Date of filing: **08.10.2020**

(51) International Patent Classification (IPC):
**C07K 14/78** (2006.01)    **C07K 14/435** (2006.01)
**A61P 41/00** (2006.01)    **A61K 38/00** (2006.01)
**C07K 19/00** (2006.01)

(52) Cooperative Patent Classification (CPC):
**A61P 41/00; C07K 14/00; C07K 14/78;** A61K 38/00;
C07K 14/43518; C07K 2319/00; C07K 2319/20;
C07K 2319/50; C07K 2319/60

(54) **BIOULTRABOND - A NOVEL SEALANT, GLUE OR FILLER FOR BIOLOGICAL TISSUES**

BIOULTRABOND - EIN NEUARTIGES DICHTMITTEL, KLEBEMITTEL UND FÜLLMITTEL FÜR BIOLOGISCHE GEWEBE

BIOULTRABOND - NOUVEAU PRODUIT D'ÉTANCHÉITÉ, DE COLLE OU DE REMPLISSAGE POUR LES TISSUS BIOLOGIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**13.04.2022 Bulletin 2022/15**

(73) Proprietor: **Albert-Ludwigs-Universität Freiburg 79098 Freiburg (DE)**

(72) Inventors:
• **SCHILLER, Stefan**
**79292 Pfaffenweiler (DE)**
• **RESCH, Anna**
**79115 Freiburg (DE)**
• **HUBER, Matthias**
**79111 Freiburg (DE)**

(74) Representative: **Grindl, Wolfgang**
**Mitscherlich PartmbB**
**Karlstraße 7**
**80333 München (DE)**

(56) References cited:
**WO-A1-2018/229341**    **WO-A2-2013/030840**

• **GALANDE ET AL: "The third dimension of gene regulation: organization of dynamic chromatin loopscape by SATB1", CURRENT OPINION IN GENETICS & DEVELOPMENT, CURRENT BIOLOGY LTD, XX, vol. 17, no. 5, 24 October 2007 (2007-10-24), pages 408 - 414, XP022323000, ISSN: 0959-437X, DOI: 10.1016/ J.GDE.2007.08.003**
• **YI-NAN ZHANG ET AL: "A Highly Elastic and Rapidly Crosslinkable Elastin-Like Polypeptide-Based Hydrogel for Biomedical Applications", ADVANCED FUNCTIONAL MATERIALS, vol. 25, no. 30, 1 July 2015 (2015-07-01), DE, pages 4814 - 4826, XP055509197, ISSN: 1616-301X, DOI: 10.1002/adfm.201501489**

Remarks:
The complete document including Reference Table(s) and the Sequence Listing(s) can be downloaded from the EPO website

## Description

Technical Field

The present invention pertains to a novel sealant, glue or filler for biological tissues based on a biodegradable, cross-linkable fusion protein having the formula (I) ULD-(L)$_n$-ULD, wherein ULD is an ubiquitin-like-domain, forming tetrameric protein structures, and (L) is a linker sequence, preferably a peptide or protein sequence having 5-1000 aa. The particular advantage of the current system lies in its great variability and low toxicity towards the recipient biological tissue, preferably a mammalian, e.g. a human, or any animal species. Moreover, the novel tissue sealant allows providing sealants, glues or fillers with high elasticity, adhesiveness and high resistance, which can be applied to a (wet) biological tissue within minutes. The present invention also pertains to medical uses of the novel biological tissue sealant, glue or filler and to the use of said fusion protein in methods of treatment.

Background of the invention

Abrasions, lacerations, or perforations to body tissues resulting from injuries, inflammation, or surgical procedures require invasive treatment, including autologous or allogenic grafting and/or suturing. As these procedures pose risks, such as graft rejection, immune response and infections, there is an unmet medical need for alternative strategies to fill and seal tissue defects of the skin, eye, or inner organs. Great advances to meet this need have been made in the field of biomaterial engineering, resulting in sophisticated (adhesive) hydrogels. This led to the approval of a variety of hydrogel-based tissue adhesives, consisting of biological materials like fibrin and thrombin (e.g. Tisseel, Evicel, among others), collagen (e.g. TachoSil, Vitagel), albumin (e.g. BioGlue), gelatin (GRF: gelatin-resorcinol-formaldehyde/glutaraldehyde, Gelatin methacrylate (GelMA)) or synthetic polymers such as cyanoacrylates (e.g. TissuGlue, CoSeal, OcuSeal, and many more) (see Ge, L. & Chen, S., Polymers (Basel). 12, 939 (2020)). WO2018/229341A1 discloses an adhesive having a repetitive region flanked by two other "non-silk" regions. These regions could be ubiquitin-like domain proteins such as from SMT3.

However, currently available sealants present significant drawbacks. While cyanoacrylate glues result in glassy patches with low elasticity (see Yang, J. et al., Adv. Funct. Mater. 30, 1-27 (2020), Barton, M. J. et al., Neurosurg. Rev. 37, 585-595 (2014)) and are associated with significant toxicity (see Barton, M. J. et al., Neurosurg. Rev. 37, 585-595 (2014); Duarte, A. P. et al., Prog. Polym. Sci. 37, 1031-1050 (2012)), gelatin-resorcinol-formaldehyde, urethane pre-polymers and fibrin glues show low adhesion to wet surfaces (see Duarte, A. P. et al., Prog. Polym. Sci. 37, 1031-1050 (2012); Park, H. C. et al., Expert Rev. Ophthalmol. 6, 631-655 (2011)). In addition, fibrin sealants often exhibit poor mechanical properties, can require effortful preparation and bear a residual risk of transmitting viral infections (see Duarte, A. P. et al., Prog. Polym. Sci. 37, 1031-1050 (2012); Park, H. C. et al., Expert Rev. Ophthalmol. 6, 631-655 (2011)). Collagen- and polyethylene glycol (PEG)-based adhesives swell upon polymerization with a risk of tissue compression (see Duarte, A. P. et al., Prog. Polym. Sci. 37, 1031-1050 (2012); Pandey, N. et al., Biomater. Sci. 8, 1240-1255 (2020)). Further issues include long or unpredictable preparation and setting times, as well as batch-to-batch differences in quality, in component concentrations and component sources (see Duarte, A. P. et al., Prog. Polym. Sci. 37, 1031-1050 (2012); Park, H. C. et al., Expert Rev. Ophthalmol. 6, 631-655 (2011)). Moreover, biodegradation without release of toxic monomers or cross-linker molecules (as happens in the case of urethane pre-polymers, cyanoacrylates and formaldehyde- or glutaraldehyde-containing systems) is desirable (see Duarte, A. P. et al., Prog. Polym. Sci. 37, 1031-1050 (2012); Pandey, N. et al., Biomater. Sci. 8, 1240-1255 (2020); Rose, J. B. et al., Materials (Basel). 7, 3106-3135 (2014)). An optimal sealant for ophthalmologic applications should also be transparent and rapidly cross-linkable in wet conditions. To date, solely the PEG hydrogels OcuSeal and ReSure are approved for sealing of clear corneal incisions, while fibrin and cyanoacrylate glues are widely used off-label in ophthalmologic surgeries (see Tong, A. Y. et al., Curr. Opin. Ophthalmol. 29, 14-18 (2018)).

To our knowledge, none of the currently available adhesives are able to overcome all of the challenges mentioned above, thus further research is warranted.

In sum, there is still a high medical need in the art to provide alternative strategies to fill, glue and seal tissue defects of the skin, tissues, eye, or inner organs, both in humans and animals.

Detailed Disclosure of the invention

In this invention, strongly adhesive ULD-based hydrogel systems are provided, generally termed ULD-(L)$_n$-ULD, and preferably as defined according to any of formulae (I), (Ia), (Ib), (Ic), (Id), (Ie) and/or (If) below, which meet numerous requirements for glues. In addition to their remarkable adhesion to wet surfaces, the inventive hydrogels provide elasticity, transparency, easy handling and rapid light-induced cross-linking. These inventive ULD-based hydrogel systems (ULD-(L)$_n$-ULD) are protein-only hydrogels and preferably of entirely human origin (particularly in case of human applications), both of which addresses and overcomes former issues with regard to immunologically adverse reactions to such current materials. Moreover, the inventive ULD-based hydrogel systems allow adjusting the material behavior from isochorus characteristics to shrinking (or if necessary expansion) upon application to the tissue. Such a tissue is typically a wet tissue. Likewise, properties of the inventive ULD-based hydrogel can be easily adapted by adapting the linker sequence $(L)_n$, e.g. by introducing a different protein or peptide sequence or by modifying protein or peptide sequences, e.g. as described herein as a linker $(L)_n$. Properties of the inventive ULD-based hydrogel can also be adapted by modifying e.g. the entire fusion protein, e.g. at the ULD domains, e.g. by introducing a corresponding modification as described herein.

The elegance of the inventive system lies within the liberty to choose the linker between ULD tails freely, forming ULD-$(L)_n$-ULD building blocks and offering limitless possibilities to incorporate specific amino acids to further enhance biocompatibility and to tailor biodegradability and cell instructing behavior, e.g. by incorporation of specific peptides or epitopes promoting cell adhesion or proteinase-mediated degradation. To avoid bond failure, optimal hydrogel sealant's modulus (Young's modulus) can be adapted to match the substrate. The inventive fusion protein is modular, versatile and customizable both on a chemical (biointeractive, bioinstructive) and mechanical level. Mechanical and physicochemical properties can be easily tailored by creating any ULD-$(L)_n$-ULD building block or mixing different building blocks, choosing the desired protein concentration, and adjusting cross-linking parameters. Different (elastic) protein building blocks, such as collagen, elastin, elastin-like-proteins (ELPs), fibrin, HSA, fibronectin, recombinant resilin, spider silk, mEGFP, GFP, SpyCatcher protein, etc., might be chosen as linker peptides $(L)_n$ to address a multitude of applications. For example, by choosing the linker sequences $(L)_n$ from human sequences, it is possible to endow the inventive hydrogels with exclusively human-derived protein sequences and thus to minimize, if not exclude, adverse immune reactions. Similarly, such systems may be adapted for all known animal species, which require treatment of abrasions, lacerations, or perforations to body tissues and organs resulting from injuries, inflammation, or surgical procedures require invasive treatment, including autologous or allogenic grafting and/or suturing.

Subsequent optimization of such linker sequences $(L)_n$ might aim at mimicking the natural extracellular matrix or adjusting resilience and tensile strength, e.g. if elastin/ELPs, resilin or spidersilk is used as a linker, or visibility, e.g. if fluorescent protein domains, of mEGFP or GFP are used as a linker, or modular equipment - non-covalent or covalent - with functional molecules, e.g. if HSA, SpyTag/SpyCatcher protein, a SNAP-tag, isopep-tag, HUH-tag, Snoop-tag, Dog-tag, Sdy-tag, etc. and their oligomers are used as a linker, or cell interactions, e.g. if ELPs, particularly charged ELPs are used as a linker, etc. With such ULD-$(L)_n$-ULD building blocks (ULD-$(L)_n$-ULD fusion proteins), there is no need for organic solvents, avoiding toxic traces and enabling the material to maintain its functionality and cross-linking ability in the aqueous environment of body tissues.

Expression of ULD-$(L)_n$-ULD fusion proteins as described herein is possible using microbial expression systems, such as *E. coli* or *S. cerevisiae*. If larger protein quantities are required for future productions, large-scale bio-fermentation and inverse transition cycling might be exploited to scale up expression and purification yields at relatively low cost.

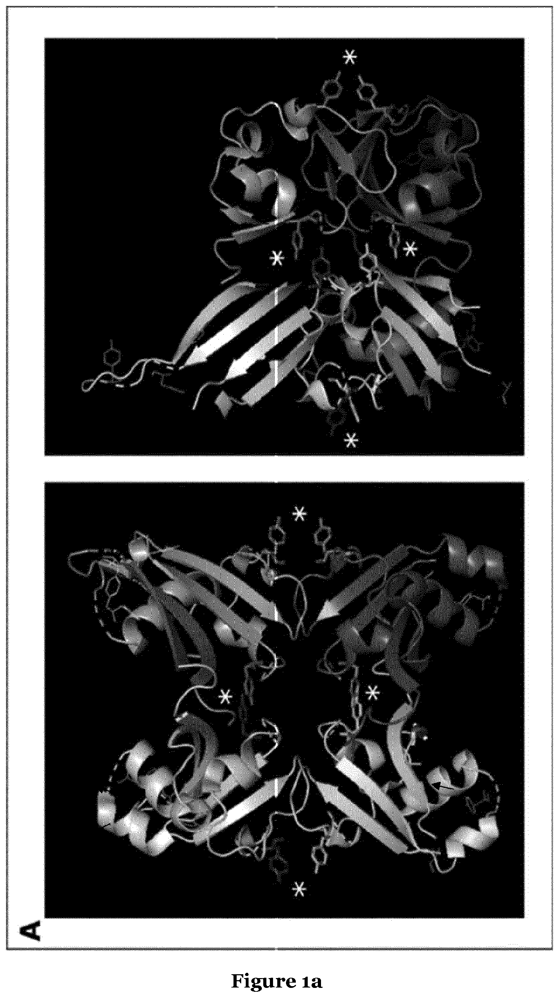

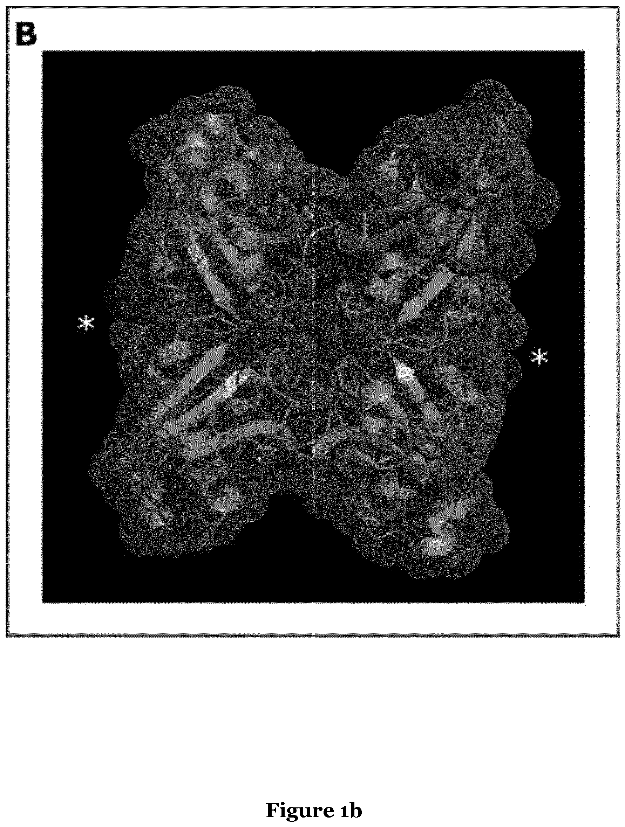

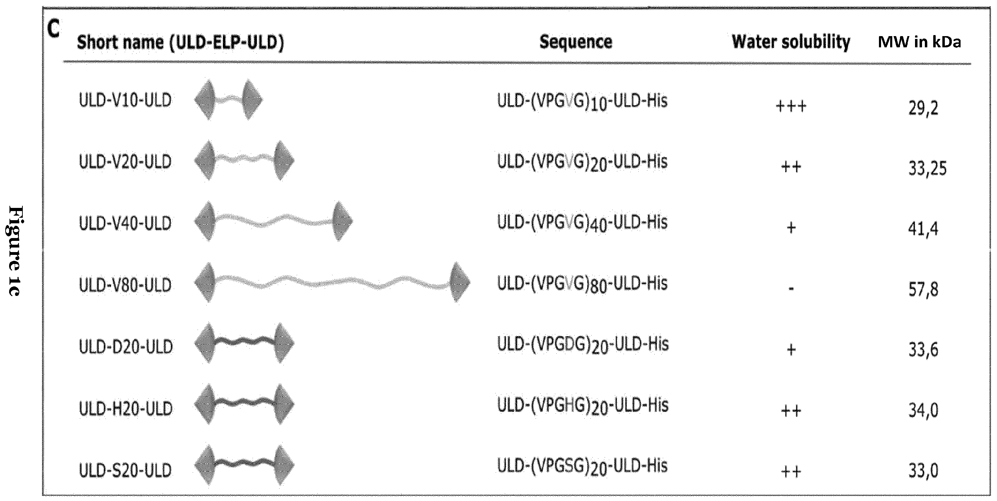

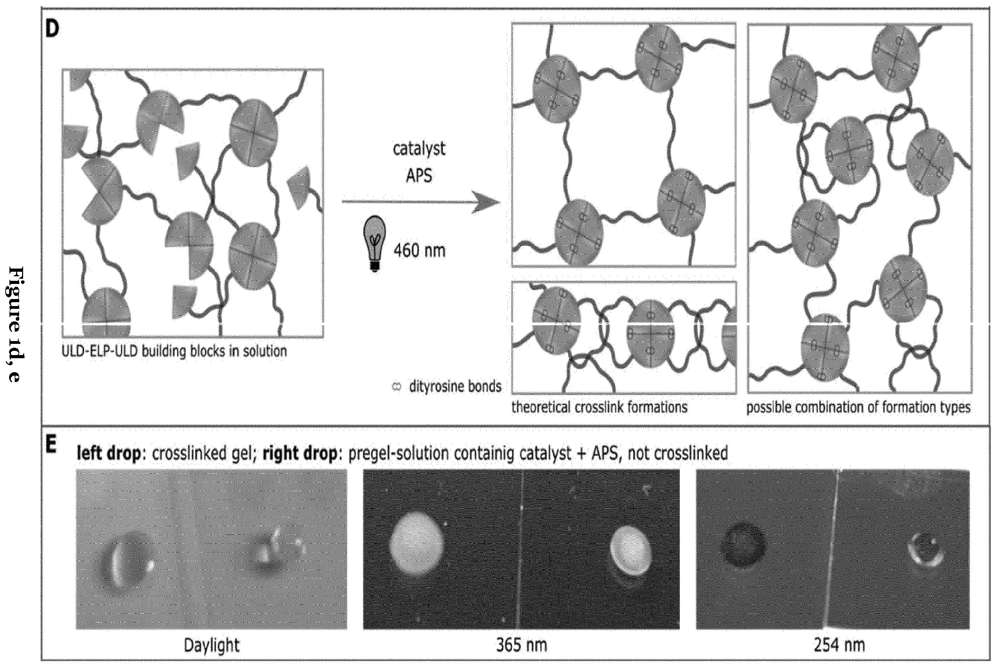

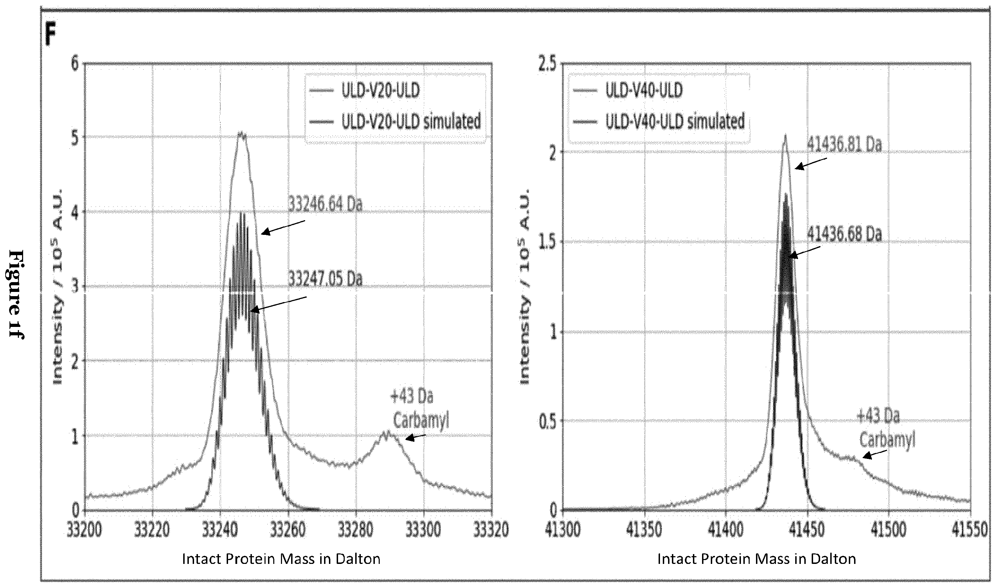

The inventive hydrogel system consists of a versatile single building block formed by a fusion protein of a peptidic or proteinogenic linker sequence $(L)_n$ flanked at both the N- and the C-terminus of the peptidic or proteinogenic linker sequence with an Ubiquitin-like domain (ULD) of the human global gene organizer SATB1 (Special AT-rich sequence-binding protein 1) (see Wang, Z. et al., Nucleic Acids Res. 40, 4193-4202 (2012)). The Ubiquitin-like domain (ULD) at the N- and the C-terminus are preferably identical. ULD exhibits a strong tendency to form homo-tetramers, even under harsh conditions (see Wang, Z. et al., Nucleic Acids Res. 40, 4193-4202 (2012)). These ULD tetramers thereby consist either of four distinct fusion proteins or a combination with internal loops within one fusion protein that could be covalently cross-linked (see Figure 1 b, d). Taking advantage of the spontaneous ULD tetramerization, the invention therefore allows formation of stabilized ULD tetramers by inducing intramolecular and intermolecular covalent cross-links, e.g. between parts of the ULD domains and/or the linker domains, e.g. cross-links of amino acids, such as inherent tyrosine residues in-between ULD tetramer subunits.

According to a first embodiment the invention as described and defined herein pertains to a fusion protein having the following formula (I):

$$\text{ULD-}(L)_n\text{-ULD}$$

ULD   is an ubiquitin-like-domain, preferably selected from any of SEQ ID NOs: 1-18 or a sequence, having at least 75% sequence identity to any of SEQ ID NOs: 1-18, preferably having at least 80 %, at least 85 %, at least 90 %, at least 95 % or even at least 99 % sequence identity to any of SEQ ID NOs: 1-18; Preferably both ULD sequences in formula (I) are identical to each other;

(L)   is a linker sequence, preferably a peptide or protein sequence having 5-1000 aa;

n is an integer selected from 1-100.

Human ULD consists of 102 amino acids (N-terminal domain of SATB1, **SEQ ID NO: 1)** and has been reported to exhibit a strong tendency to form homo-tetramers as a consequence of hydrophobic interactions and hydrogen bonds within the tetramer interfaces. In addition to the work reported by Wang et al. (see Wang, Z. et al., Nucleic Acids Res. 40, 4193-4202 (2012), Wang, Z. et al., J. Biol. Chem. 289, 27376-27385 (2014)) the inventors confirmed the absence of ULD monomers in water via SDS-PAGE, MS and DLS. At the commonly used urea concentration of $4\frac{mol}{L}$ (4 M urea), ULD tetramers were predominant. Notably, the ability of ubiquitin to form stable tetramers is maintained in fusion proteins and inventors confirmed this finding by MS and DLS. As the cross-linking system used in the current invention is exclusively based on ULD, the inventors surprisingly found that there is a large freedom to choose the linker between both ULD tails without impeding the cross-linking mechanism.

The ubiquitin sequence as used in the current invention in formula (I) is preferably an ubiquitin-like-domain, more preferably selected from SEQ ID NO: 1 or a sequence, having at least 75% sequence identity to SEQ ID NO: 1, preferably at least 75%, at least 80 %, at least 85 %, at least 90 %, at least 95 % or even at least 99 % sequence identity to SEQ ID NO: 1. In the context of a human to be treated, preferably the entire sequence of the fusion peptide ULD-$(L)_n$-ULD according to formula (I) is human or based on human sequences.

Additional or alternatively to such human ubiquitin-like-domain sequences in formula (I), according to SEQ ID NO: 1 sequences for the ubiquitin-like-domain can be derived from fish, amphibian, avian, such as chicken, birds, mammals, such as human, dog, cat, horse, sheep, cow, pig, guinea pig, rabbit, rodents, etc., all of which exhibit at least 75% sequence identity with SEQ ID NO: 1 (N-terminal domain of human SATB1), e.g.: any of SEQ ID NOs: 2-18 as depicted in the following:

- **SEQ ID NO:2** (dog, Canis lupus familiaris, DNA-binding protein SATB1, NCBI Reference XP_022264105.1, N-terminal sequence, 100% sequence identity to SEQ ID NO: 1);

- **SEQ ID NO: 3** (dog, Canis lupus familiaris, DNA-binding protein SATB2 isoform X1, NCBI Reference: XP_005640560.1, N-terminal sequence, 78% sequence identity to SEQ ID NO: 1);

- **SEQ ID NO:4** (cat, *Felis catus,* DNA-binding protein SATB1 isoform X4, NCBI Reference XP_011284516.1, N-terminal sequence, 100% sequence identity to SEQ ID NO: 1);

- **SEQ ID NO: 5** (*Equus caballus,* DNA-binding protein SATB1 isoform X1, NCBI Reference: XP_014587330.2, N-terminal sequence, 100% sequence identity to SEQ ID NO: 1);

- **SEQ ID NO: 6** (pig, *Sus scrofa,* DNA-binding protein SATB1, NCBI Reference: XP_003358373.2, N-terminal sequence, 100% sequence identity to SEQ ID NO: 1);

- **SEQ ID NO: 7** (pig, *Sus scrofa,* DNA-binding protein SATB2 isoform X1, NCBI Reference: XP_020931727.1, N-terminal sequence, 78% sequence identity to SEQ ID NO: 1);

- **SEQ ID NO: 8** (cow, *Bos taurus,* DNA-binding protein SATB1, NCBI Reference: NP_001095510.1, N-terminal sequence, 100% sequence identity to SEQ ID NO: 1);

- **SEQ ID NO: 9** (cow, *Bos taurus,* DNA-binding protein SATB2, NCBI Reference: NP001192999.1, N-terminal sequence, sequence identity 78% to SEQ ID NO: 1);

- **SEQ ID NO: 10** (sheep, *Ovis aries,* DNA-binding protein SATB1, NCBI-reference: XP_027817855.1, N-terminal sequence, 100% sequence identity to SEQ ID NO: 1);

- **SEQ ID NO: 11** (guinea pig (*Cavia porcellus,* DNA-binding protein SATB1 isoform X1, NCBI Reference: XP_005008358.1, N-terminal sequence, 100% sequence identity to SEQ ID NO: 1);

- **SEQ ID NO: 12** (rabbit (*Oryctolagus cuniculus,* DNA-binding protein SATB1, NCBI-reference: XP_008264272.1, N-terminal sequence, 100% sequence identity to SEQ ID NO: 1);

- **SEQ ID NO: 13** (rodent, prairie vole, *Microtus ochrogaster,* DNA-binding protein SATB1 isoform X1, NCBI-reference: XP_005368803.1, N-terminal sequence, sequence identity 100 % to SEQ ID NO: 1);

- **SEQ ID NO: 14** (Chicken, *Gallus gallus,* DNA-binding protein SATB1, NCBI Reference: NP_001186573.1, N-terminal sequence, 98% sequence identity to SEQ ID NO: 1);

- **SEQ ID NO: 15** (Alligator, (amphibian) *Alligator mississippiensis,* DNA-binding protein SATB2, NCBI Reference: KYO26889.1, N-terminal sequence, 98% sequence identity to SEQ ID NO: 1);

- **SEQ ID NO: 16** (Fish, *Oncorhynchus kisutch engl.* coho salmon, DNA-binding protein SATB1-like isoform X1, NCBI Reference: XP_031642577.1, N-terminal sequence, 95 % sequence identity to SEQ ID NO: 1);

- **SEQ ID NO: 17** (Fish, *Oncorhynchus mykiss, eng.* rainbow trout, dt. Regenbogenforelle, DNA-binding protein SATB1-like isoform X6, NCBI Reference: XP_021450053.1, N-terminal sequence, 95%Seq. Identity to SEQ ID NO: 1);

- **SEQ ID NO: 18** (Fish, *Oncorhynchus mykiss,* DNA-binding protein SATB1-like, NCBI Reference: XP_021417777.1, N-terminal sequence, 81% Seq. Identity to SEQ ID NO: 1).

Hence, ubiquitin-like-domain sequences ULD in formula (I) may comprise a sequence according to any of SEQ ID NO: 1-18, or a sequence having at least 75%, preferably at least 80% or at least 85%, more preferably at least 90%, even more preferably at least 95 % or most preferably at least 99 % sequence identity to any of **SEQ ID NOs: 1-18.** More specifically, ubiquitin-like-domain sequences ULD in formula (I) may comprise a sequence according to any of **SEQ ID NO: 1**, or a sequence, having at least 75%, preferably at least 80% or at least 85%, more preferably at least 90%, even more preferably at least 95 % or most preferably at least 99 % sequence identity to **SEQ ID NO: 1.** Such ULD sequences preferably comprise any of **SEQ ID NOs: 2-18**, or a sequence having at least 75%, preferably at least 80% or at least 85%, more preferably at least 90%, even more preferably at least 95 % or most preferably at least 99 % sequence identity to any of **SEQ ID NOs: 2-18.**

In order to determine the percentage to which two sequences (amino acid sequences as defined herein or nucleic acid sequences) are identical, the sequences can be aligned in order to be subsequently compared to one another. Therefore, e.g. gaps can be inserted into the sequence of the first sequence and the component at the corresponding position of the second sequence can be compared. If a position in the first sequence is occupied by the same component as is the case at a position in the second sequence, the two sequences are identical at this position. The percentage to which two sequences are identical is a function of the number of identical positions divided by the total number of positions. The percentage to which two sequences are identical can be determined using a mathematical algorithm. A preferred, but not limiting, example of a mathematical algorithm which can be used is the algorithm of Karlin et al. (1993), PNAS USA, 90:5873-5877 or Altschul et al. (1997), Nucleic Acids Res., 25:3389-3402. Such an algorithm is integrated in the BLAST program. Sequences which are identical to the sequences of the present invention to a certain extent can be identified by this program. Any further algorithm may be used suitable to identify a homology or identity between two sequences.

According to the general formula (I) the inventive fusion protein further comprises a linker (L), which connects the two ubiquitin-like-domain as defined in formula (I). The linker (L) is therefore flanked at both the N- and the C-terminal end by an ubiquitin-like-domain as defined above.

According to one embodiment, the linker (L) is a polypeptide or protein sequence, having an amino acid number of between 5-1000 aa. Such a polypeptide or protein sequence may generally be selected from any possible peptide, protein or amino acid sequence having a length of between 5-1000 aa, 10-1000 aa, or 15-1000 aa, e.g. between 25 and 1000 aa, preferably 25-1000 aa, e.g. 25-1000 aa, etc.

According to one more specific embodiment, the linker (L) is a polypeptide or protein sequence exemplarily selected from proteins comprising, but not limited thereto,

- collagen (as encoded by any of DNA sequences **SEQ ID NO: 19 or 21**, or as defined by any of protein sequences according to **SEQ ID NO: 20 or 22),**

- elastin (as encoded by DNA sequence **SEQ ID NO: 23**, or as defined by protein sequence **SEQ ID NO: 24),**

- fibrinogen (as encoded by DNA sequence **SEQ ID NO: 25**, or as defined by protein sequence **SEQ ID NO: 26),**

- HSA (as encoded by DNA sequence **SEQ ID NO: 27**, or as defined by protein sequence **SEQ ID NO: 28),**

- fibronectin (as encoded by DNA sequence **SEQ ID NO: 29**, or as defined by protein sequence **SEQ ID NO: 30),**

- recombinant resilin (10x) (as encoded by DNA sequence **SEQ ID NO: 31,** or as defined by protein sequence **SEQ ID NO: 32),**

- spider silk (10x) (as encoded by DNA sequence **SEQ ID NO: 33,** or as defined by protein sequence **SEQ ID NO: 34),**

- mEGFP (as encoded by DNA sequence **SEQ ID NO: 35,** or as defined by protein sequence **SEQ ID NO: 36),**

- SpyCatcher protein (as encoded by DNA sequence **SEQ ID NO: 37,** or as defined by protein sequence **SEQ ID NO: 38),** or

- Green Fluorescent Protein (GFP) (as encoded by DNA sequence **SEQ ID NO: 39,** or as defined by protein sequence **SEQ ID NO: 40),**

- fluorescent proteins, such as mCherry (fluorescent protein), signal peptides, protease recognition sequences, such as TEVrec protease recognition sequence, a recognition sequence for protease Factor XA, recognition sequence for protease Thrombin, etc., or peptides or epitopes promoting cell adhesion or proteinase-mediated degradation, etc.,

- mixtures of such linker sequences, preferably as described herein, such as mCHR-TEVree-mEGFP (mCHR = monomeric mCherry (fluorescent protein; TEVrec = TEVrec protease recognition sequence; mEGFP) (as encoded e.g. by DNA sequence **SEQ ID NO: 41** or as defined by protein sequence **SEQ ID NO: 42),**

Encompassed are particularly as linker sequence (L) a protein sequence of any of **SEQ ID NO: 20, 22, 24, 26, 28, 30, 32, 34, 36, 38, 40, or 42** or a sequence, having at least 75%, preferably at least 80% or at least 85%, more preferably at least 90%, even more preferably at least 95 % or most preferably at least 99 % sequence identity to such a protein sequence of any of SEQ ID NO: 20, 22, 24, 26, 28, 30, 32, 34, 36, 38, 40, or 42. Alternatively, such protein sequences may be encoded by a nucleic acid sequence according to any of **SEQ ID NOs: 19, 21, 23, 25, 27, 29, 31, 33, 35, 37, 39, or 41** or a sequence, having at least 75%, preferably 80% or at least 85%, more preferably at least 90%, even more preferably at least 95 % or most preferably at least 99 % sequence identity to a nucleic acid sequence according to any of SEQ ID NOs: 19, 21, 23, 25, 27, 29, 31, 33, 35, 37, 39, or 41. In view of linker sequences (L) according to a protein sequence of any of SEQ ID NO: 20, 22, 24, 26, 28, 30, 32, 34, 36, 38, 40, or 42 the repeat number n of linker sequence (L) in formula (L) is preferably as defined above, likewise preferably 1-20, more preferably 1-10, even more preferably 1-5, e.g. 1-4, 1-3, 1-2 or even 1.

According to a further specific embodiment, the linker sequence (L) may be selected from an elastin-like-protein (ELP), preferably as defined below. In the context of the present invention, such an elastin-like-protein (ELP) is preferably based on ELP amino acid sequences derived from mammals, birds, fish, or amphibian, preferably human, avian, dog, cat, horse, sheep, cow, pig, rabbit or rodents. If the corresponding sequence of a species is used (preferably both with regard to ULD sequence and linker sequence (L) as defined herein), this ensures high biocompatibility, superior elasticity, facile production and versatility in the corresponding species. Hence, preferably sequences from the same or at least a related species are used which is to be treated. In the context of a human, preferably the entire sequence of the fusion peptide ULD-(L)$_n$-ULD according to formula (I) is human or based on human sequences.

The genetically engineered elastin-like-proteins (ELP) typically consist of the repetitive pentamer sequence Val-Pro-Gly-(Xaa)$_m$-Gly, the pentamer sequence typically repeated n-times as defined above for formula (I) and then defined within formula (I) typically as (Val-Pro-Gly-(Xaa)$_m$-Gly)$_n$. (Val-Pro-Gly-(Xaa)$_m$-Gly) can be also written in the one-letter amino acid code as (VPGX$_m$G), wherein V = Val, P = Pro, G = Gly and X = Xaa. The pentamer sequence VPGVG, forming basis for sequence VPGX$_m$G/Val-Pro-Gly-(Xaa)$_m$-Gly, is present in nearly all animal species and is in case of humans derived from the human extracellular matrix protein tropoelastin. Any proteinogenic amino acid, preferably except for proline, can be introduced at the 4$^{th}$ position (Xaa) or (X) without a major impact on the polypeptide's secondary structure. Such an amino acid (Xaa) or (X) may be either present as one amino acid aa or as a repetition of the same amino acid aa. Such a repeat is then defined by integer m, which is a number from 1 to 5, preferably 1-4, more preferably 1-3 or 2, most preferably 1. Within the repeat (Xaa)$_m$ (or (X)$_m$) each amino acid can be selected independently from an aa as defined for Xaa below, i.e. each aa can be different or the same aa or a mixture thereof may be provided. Due to the variability, the physicochemical properties of ELPs can be easily varied and adjusted, e.g. to surroundings requirements, while structural molecular integrity and main behavioral characteristics, such as principle elasticity and durability, are maintained at the same time. According to a particularly preferred embodiment, the ELP sequence is human.

Accordingly, the elastin-like-protein (ELP), used as linker sequence (L), may preferably have the following generic sequence:

(Val-Pro-Gly-(Xaa)$_m$-Gly) **(SEQ ID NO: 43)**

wherein

Xaa    is independently from each other selected from Glu, Asp, Arg, Gly, Ala, Val, Lys, His, Ser, Thr, Asn, Gln, Tyr, Cys, Phe, Ile, Leu, Met or Trp; or is a non-naturally occurring amino acid, or is a mixture thereof.

m    is an integer from 1 to 5, preferably 1-4, more preferably 1-3 or 2, most preferably 1.

Typically, SEQ ID NO: 43 is repeated n-times as generally defined above for linker sequence (L) in formula (I).

Accordingly, the inventive fusion protein according to formula (I) may be formulated more specifically as Formula (Ia), if an elastin-like-protein (ELP) is used as a linker sequence (L):

$$\text{ULD-(Val-Pro-Gly-(Xaa)}_m\text{-Gly)}_n\text{-ULD} \qquad \text{formula (Ia)}$$

wherein

ULD    is as defined above;

n    is an integer as defined above, preferably selected from 1-100, likewise preferably from 3-90, more preferably from 3-80, even more preferably from 3-65 or from 3-45, e.g. from 10-120, from 15-90, from 15-80, from 15 to 65, 15 to 45 or from 20 to 40;

m    is an integer as defined above, preferably selected from 1 to 5, preferably 1-4, more preferably 1-3, even more preferably 1-2, most preferably 1;

Xaa    is independently selected from Glu, Asp, Arg, Gly, Ala, Val, Lys, His, Ser, Thr, Asn, Gln, Tyr, Cys, Phe, Ile, Leu, Met or Trp; or is a non-naturally occurring amino acid, or is a mixture thereof

More preferably, Xaa = Val, His, Asp, Arg, Ser, most preferably Xaa = Val

Preferably, each repeat unit (Val-Pro-Gly-(Xaa)$_m$-Gly) according to **SEQ ID NO: 43** and as embedded above in the Formula (Ia) as (Val-Pro-Gly-(Xaa)$_m$-Gly)$_n$ may be selected independently from each other, which allows preparation of fusion peptides according to the invention with an alternating linker sequence (ELP).

According to one embodiment, the elastin-like-protein (Val-Pro-Gly-(Xaa)$_m$-Gly) **(SEQ ID NO: 43)** may be selected from at least one or more of the peptide sequences according to any of **SEQ ID NO: 44 to 65,** more preferably as listed in Table 1 in the following:

Table 1

| | |
|---|---|
| Val-Pro-Gly-**Glu**-Gly | **SEQ ID NO: 44** |
| Val-Pro-Gly-**Asp**-Gly | **SEQ ID NO: 45** |
| Val-Pro-Gly-**Arg**-Gly | **SEQ ID NO: 46** |
| Val-Pro-Gly-**Val**-Gly | **SEQ ID NO: 47** |
| Val-Pro-Gly-**Lys**-Gly | **SEQ ID NO: 48** |
| Val-Pro-Gly-**His**-Gly | **SEQ ID NO: 49** |
| Val-Pro-Gly-**Ser**-Gly | **SEQ ID NO: 50** |
| Val-Pro-Gly-**Thr**-Gly | **SEQ ID NO: 51** |
| Val-Pro-Gly-**Asn**-Gly | **SEQ ID NO: 52** |
| Val-Pro-Gly-**Gln**-Gly | **SEQ ID NO: 53** |
| Val-Pro-Gly-**Tyr**-Gly | **SEQ ID NO: 54** |
| Val-Pro-Gly-**Cys**-Gly | **SEQ ID NO: 55** |
| Val-Pro-Gly-**Phe**-Gly | **SEQ ID NO: 56** |
| Val-Pro-Gly-**Ile**-Gly | **SEQ ID NO: 57** |
| Val-Pro-Gly-**Leu**-Gly | **SEQ ID NO: 58** |
| Val-Pro-Gly-**Met**-Gly | **SEQ ID NO: 59** |
| Val-Pro-Gly-**Trp**-Gly | **SEQ ID NO: 60** |
| Val-Pro-Gly-**Ala**-Gly | **SEQ ID NO: 61** |
| Val-Pro-Gly-**Gly**-Gly | **SEQ ID NO: 62** |
| Val-Pro-Gly-**Asp**-Gly-Val-Pro-Gly-**Ser**-Gly-Val-Pro-Gly-**Tyr**-Gly | **SEQ ID NO: 63** |
| Val-Pro-Gly-**Val**-Gly-Val-Pro-Gly-**Arg**-Gly-Val-Pro-Gly-**Tyr**-Gly | **SEQ ID NO: 64** |
| Val-Pro-Gly-**Val**-Gly-Val-Pro-Gly-**Val**-Gly-Val-Pro-Gly-**Tyr**-Gly | **SEQ ID NO: 65** |

Preparation of the ELP amino acid sequences as linker (L) and $(L)_n$ can occur as outlined in EP 2 668 962, EP 2854863, US 1,0428,137 or WO 2013/178627, the process of which facilitates the generation and rapid extension of a first set of repetitive ELP pentamers and the subsequent modular insertion between two ULD domains. The generated repeat numbers n of the pentamer motif (Val-Pro-Gly-(Xaa)$_m$-Gly) **(SEQ ID NO: 43)**, or according to any of **SEQ ID NO: 44-62 or 63-65,** typically varies from n = 1-100 as depicted above for formula (I) or (Ia), preferably from 3-90, more preferably from 3-80, even more preferably from 3-65 or from 3-45, e.g. from 10-120, from 15-90, from 15-80, from 15 to 65, from 15 to 45 or from 20 to 40, for different ULD-(Val-Pro-Gly-(Xaa)$_m$-Gly)$_n$-ULD variants and m = 1-5 as defined above. As mentioned above, the pentamer motif can be varied within the whole ULD-$(L)_n$-ULD fusion peptide by changing Xaa between the above single pentamer motifs according to any of SEQ ID NO: 44-65, each of which may be contained in the single repeats of $(L)_n$. The pentamer motifs with differing Xaa can occur within one ULD-$(L)_n$-ULD fusion peptide either randomly or blockwise or in a further regular manner. An exemplary "tri-block" for (L) is e.g. "VPGVG-VPGRG-VPFYG", "VPGDG-VPGSG-VPGYG" or "VPGVG-VPGVG-VPGYG", each of which is depicted in **SEQ ID NO:s 63, 64 and 65,** each of which serves as an example, without being limited thereto, as to how building blocks according to any of SEQ ID NO's: 44-62 can be can be formed and combined with each other and varied accordingly. Each variation of Xaa within the number of repeats of $(L)_n$ is possible and envisaged.

The inventive fusion protein according to formula (I) or (Ia) may also comprise a fusion peptide linker (FPL), which may be inserted between the N-terminal ULD domain and the linker $(L)_n$, and/or the between the C-terminal ULD domain and the linker $(L)_n$, the linker $(L)_n$ being any linker as defined above, e.g. (Val-Pro-Gly-(Xaa)$_m$-Gly)$_n$. Such an optional fusion peptide linker (FPL) may be used to increase the distance between the linker $(L)_n$, and/or the N- and C-terminal ULD sequences, preferably to increase the degree of freedom of the ULD sequence(s) and/or the linker $(L)_n$, alternatively to increase stability or folding of the ULD domain(s) and/or the linker $(L)_n$, to increase expression efficiency, to improve biological activity, to enable targeting, to introduce cleavage sites or to alter the pK, etc.

The inventive fusion protein according to formula (I) or (Ia) can therefore be formulated also as Formulae (Ib) or (Ic):

ULD-(FPL)-$(L)_n$-(FPL)-ULD,　　　　　　Formula (Ib),

or

ULD-(FPL)-(Val-Pro-Gly-(Xaa)$_m$-Gly)$_n$-(FPL)-ULD,　　　　　　Formula (Ic)

wherein

ULD　　is as defined in claim 1

(L)　　is as defined in claim 1

n　　is an integer and is as defined above for formula (I) or (Ia), preferably selected from 1-100, likewise preferably from 3-90, more preferably from 3-80, even more preferably from 3-65 or from 3-45, e.g. from 10-120, from 15-90, from 15-80, from 15 to 65, 15 to 45 or from 20 to 40;

m　　is an integer as defined above, preferably selected from 1 to 5, preferably 1-4, more preferably 1-3 or 2, most preferably 1;

(FPL)　　is an optional amino acid sequence and independently from each other comprises 1 to 30 amino acids, and is more preferably selected from any of **SEQ ID NO: 66 to 85,** or a sequence, having at least 75% sequence identity, preferably at least 80 %, at least 85 %, at least 90 %, at least 95 % or even at least 99 % sequence identity to a nucleic acid sequence according to any of SEQ ID NO: 66 to 85;

Such a fusion peptide linker (FPL) as optionally used in the current invention independently of each other may comprise or consist of one of the following sequences, selected from DPMSSS **(SEQ ID NO: 66),** GGREASS **(SEQ ID NO: 67**), (GGGGS)$_{1-4}$ **(SEQ ID NO: 68),** (Gly$_{1-5}$,), preferably Gly$_1$, Gly$_2$, Gly$_3$, Gly$_4$ or Gly$_5$ **(SEQ ID NO: 69),** (Gly)$_6$ **(SEQ ID NO: 70),** (Gly)s **(SEQ ID NO: 71),** (EAAAK)$_{1-3}$ **(SEQ ID NO: 72),** A(EAAAK)$_4$ALEA(EAAAK)$_4$A **(SEQ ID NO: 73),** PAPAP **(SEQ ID NO: 74),** AEAAAKEAAAKA **(SEQ ID NO: 75**), disulfide, VSQTSKLTR↓AETVFPDV **(SEQ ID NO: 76),** PLG↓LWA **(SEQ ID NO: 77**), RVL↓AEA **(SEQ ID NO: 78),** EDVVCC↓SMSY **(SEQ ID NO: 79),** GGIEGR↓GS **(SEQ ID NO: 80),** TRHRQPR↓GWE **(SEQ ID NO: 81),** AGNRVRR↓SVG **(SEQ ID NO: 82),** RRRRRRR↓R↓R **(SEQ ID NO: 83),** or GFLG↓LE **(SEQ ID NO: 84),** or ENLYFQX, and X=G or S **(SEQ ID NO: 85,** TEV protease recognition/cleavage site), etc; (↓ indicates a potential cleavage site), or a sequence, having at least 75% sequence identity, preferably at least 80 %, at least 85 %, at least 90 %, at least 95 % or even at least 99 % sequence identity to a nucleic acid sequence according to any of SEQ ID NO: 66 to 85. For fusion peptide linker sequences see also Xiaoying Chen et al., Adv Drug Deliv Rev. 2013 October 15; 65(10): 1357-1369. doi:10.1016/j.addr.2012.09.039).

Moreover, the inventive fusion protein according to any of formulae (I), (Ia), (Ib) or (Ic), may comprise a modification (Mod), such as a tag for purification or interaction, a protease cleavage site, chemical cleavage sites, peptides or epitopes promoting cell adhesion, signal proteins, fluorescent proteins (such as mGFP, GFP, etc), etc. Such a modification may be located generally between any of elements ULD and $(L)_n$, or may be located at the N-terminal end of the fusion protein according to any of formulae (I), (Ia), (Ib) or (Ic), at the C-terminal end of the fusion protein according to any of formulae (I), (Ia), (Ib) or (Ic), or both at the N-terminal end and the C-terminal end. Each such modification may be selected independently from each other.

For example, the inventive fusion protein according to any of formulae (I), (Ia), (Ib) and/or (Ic) may comprise, e.g. a purification tag (or any other modification) at the C-terminal end and/or a peptide or epitope promoting cell adhesion at the N-terminal end, a purification tag (or any other modification) solely at the N-terminal or C-terminal end, etc. Likewise, the combination of two or more modifications at the N- and/or C-terminal end of the fusion protein according to any of formulae (I), (Ia), (Ib) or (Ic), are possible, e.g. a protease cleavage site and a tag for purification or interaction, both at the N-terminal or C-terminal end, etc., or a (m)GFP at the C-terminal end and a His-tag at the N-terminal end, a signal protein at the C-terminal end and a His-tag at the N-terminal end, etc. Such a modification may also be a fusion peptide linker sequence as defined above.

Particularly preferable the inventive fusion protein according to any of formulae (I), (Ia), (Ib) and/or (Ic) may be modified by incorporating a tag for purification or interaction, preferably selected from a His6-tag (His6), a His10-tag (His10), a FLAG-tag, a Halo-tag, a NE-tag, ALFA-tag, a Calmodulin-tag, a polyglutamate-tag, an Avi-tag, a C-tag, a polyarginine-tag, an E-tag, SBP-tag, TC-tag, V5-tag, VSV-tag, a FLAG-tag, a T7-tag, a HA-tag, a Strep-Tag, a MYC tag, SpyTag/SpyCatcher protein, a SNAP-tag, isopep-tag, HUH-tag, a Spot-tag, Snoop-tag, Dog-tag, Sdy-tag, etc. Further tags for purification or interaction are known to a skilled person and may be adapted and used as suitable in the fusion protein as defined above. Such tags for purification and interaction may be located at the N-terminal end and/or at the C-terminal end of the fusion protein according to any of formulae (I), (Ia), (Ib) and/or (Ic). According to one preferred embodiment such a tag for purification or interaction can be cleaved off from the remaining fusion protein according to any of formula (I), (Ia), (Ib) and/or (Ic), e.g. by incorporating a further protease cleavage site between the fusion protein according to any of formulae (I), (Ia), (Ib) and/or (Ic) and the tag for purification or interaction to be cleaved off. In this context, a tag for purification is typically a tag that allows purification of the fusion protein after expression. A tag for interaction is typically to be understood as a protein sequence, which allows forming a (non)-covalent or even a covalent bond to a specific counterpart. The above groups meet at least one of both requirements.

Protease cleavage sites in the context of the present invention may comprise protease recognition sequences, preferably cleavage sites or recognition sequences for endo- or exo-proteases, preferably selected from a recognition sequences for protease Factor XA, a TEV protease recognition sequence, recognition sequence for protease Thrombin, or any further protease cleavage site as known to a skilled person, e.g. as obtainable by data bases such as ExPASy Proteomics Server - PeptideCutter page etc.

Chemical cleavage sites in the context of the present invention may comprise sites cleavable by hydroxylamine. Such sites are preferably amino acids N-G (asparagine-glycine). Chemical cleavage sites may comprise sites cleavable by CNBr, metal catalyzed cleavage sequences, acids such as TFA, HCl, formic acid, iodosobenzoic acid, NTCB (2-nitro-5-thiocyanobenzoic acid), etc.

One example showing such modifications is depicted in SEQ ID NO: 116 (DNA sequence) and SEQ ID NO: 117 (protein sequence), showing an exemplary partial sequence of any of formulae (I), (Ia), (Ib) or (Ic), comprising an ULD sequence having N-terminally a (starting) methionine (which can be omitted), then a fusion peptide linker DPMSSS (SEQ ID NO: 66), then a ULD sequence (SEQ ID NO: 1), then again a fusion peptide linker GGREASS (SEQ ID NO: 67), followed by a His$_6$-tag. Such a ULD fragment may be used e.g. C-terminally to a ULD-$(L)_n$ fragment of any of formulae (I), (Ia), (Ib) and/or (Ic).

Exemplary formulae (Id), (Ie) and (If) are depicted in the following, wherein (Mod) is an optional modification as described above:

$$(Mod)\text{-}ULD\text{-}(FPL)\text{-}(L)_n\text{-}(FPL)\text{-}ULD\text{-}(Mod), \qquad \text{Formula (Id)}$$

$$(Mod)\text{-}(Mod)\text{-}ULD\text{-}(FPL)\text{-}(L)_n\text{-}(FPL)\text{-}ULD\text{-}(Mod)\text{-}(Mod), \qquad \text{Formula (Ie)}$$

$$(Mod)\text{-}ULD\text{-}(Mod)\text{-}(FPL)\text{-}(L)_n\text{-}(FPL)\text{-}(Mod)\text{-}ULD\text{-}(Mod), \qquad \text{Formula (If)}$$

(Mod) is an optional amino acid sequence between 1 to 300 aa, e.g. between 1-250 aa. (Mod) represents a further modification and is selected independent from each other. More preferably, (Mod) is selected from modification as described above, typically a tag for purification or interaction as defined herein above, even more preferably selected from a protease cleavage site, a chemical cleavage site, peptides or epitopes promoting cell adhesion, signal proteins, fluorescent proteins (such as mGFP, GFP, etc), etc., as defined herein.

Each ULD, (FPL), L and n is preferably as described above.

The fusion proteins according to any of formulae (I), (Ia), (Ib), (Ic), (Id), (Ie) and/or (If) may furthermore be cross-linked. Although ULD tetramers are highly stable under physiological conditions, the physical (non-covalent) cross-links between tetramer subunits (i.e. each ULD as defined herein in the context of any of the inventive formulae) might beneficially increase binding strength, e.g. to further to endow a hydrogel with sufficient mechanical strength for the desired application. The inventors therefore decided to also allow cross-linking the preexisting ULD tetramers of the fusion protein according to any of formulae (I), (Ia), (Ib), (Ic), (Id), (Ie) and/or (If) to introduce covalent bonds into the interfaces of the ULD tetramer subunits. The invention particularly allows formation of stabilized ULD tetramers by inducing intramolecular and intermolecular covalent cross-links, e.g. between the ULD domains and/or the linker domains, e.g. cross-links of amino acids, such as cross-linking inherent tyrosine residues in-between ULD tetramer subunits and formation of dityrosine bonds. Such cross-linked hydrogels provide sufficient flexibility but also mechanical strength and resistance for in *vivo* applications. Likewise, cross-links can be introduced between the different linker sequences $(L)_n$ as described above. Most preferably, however, (covalent) cross-links are formed between interfaces of ULD tetramer subunits.

Generally, cross-linking reactions as described above can be carried out by any suitable cross-linking method, e.g. chemical cross-linking reactions, photochemical cross-linking reactions, catalytic induction of dityrosine formation, cross-linking reactions using a cross-linking catalyst, cross-linking reactions via peroxidases, laccases, transaminases, or alternatively via any further cross-linking method. Photochemical cross-linking reactions (photochemically inducing intramolecular and intermolecular covalent cross-links), cross-linking reactions using a cross-linking catalyst, and catalytic induction of dityrosine formation are preferred. Most preferably, photochemical cross-linking is carried out.

Cross-linking reactions (as well as cross-linking agents or a cross-linking catalyst) in the context of the present invention typically concern cross-linking reactions allowing to form covalent bonds between and within proteins, e.g. photochemical cross-linking reactions (via redox or radical mechanisms e.g. C-C, C-O, C-N, C-P, C-B, C-S cross-links), isopeptide bond formation, transglutaminase reaction, active ester-like ester or peptide bond forming reactions, aldehyde/ketone-amine reactions, amin-amin cross-linker, cross-linking two amino acids carrying amino groups, aldehyde-nucleophile (e.g. amines) reactions, reactions forming disulfides or utilizing sulfhydryl cross-linker, amin-sulfhydryl cross-linker, sulfhydryl reactive cross-linker, aromatic ketones, arylazides, diazirines and tag-dependent cross-links e.g. via SpyTag/SpyCatcher protein, a SNAP-tag, isopep-tag, HUH-tag, a Spot-tag, Snoop-tag, Dog-tag, Sdy-tag etc. Cross-linking agents in the context of the present invention also include and are preferably selected from cross-linking catalysts, which catalyze the formation of cross-links between amino acids, formation of amino acid cross-links forming e.g. dityrosines by cross-linking of two closely neighbored tyrosine residues. Such cross-linking catalysts may be selected from the group comprising or consisting of riboflavin, riboflavin phosphate, Tris(bipyridine)ruthenium(II) chloride (Ru(II)bpy), 2-hydroxy-4'-(2'-hydroxyethoxy)-2-methylpropiophenone, methylene blue, phenylbis(2,4,6-trimethylbenzoyl)phosphine oxide, multifunctional aldehydes and ketones e.g. glutaraldehyde, haloacetyles, Pyridyl disulfides, active esters (e.g. NHS-ester, Maleimide cross-linker, peroxidase, laccase, transglutaminase, chlorophyll, etc. Cross-linking reactions may preferably include e.g. a photochemical cross-linking reaction using as a cross-linking catalyst at least one of riboflavin, riboflavin phosphate, Tris(bipyridine)ruthenium(II) chloride (Ru(II)bpy), 2-hydroxy-4'-(2'-hydroxyethoxy)-2-methylpropiophenone, phenylbis(2,4,6-trimethylbenzoyl)phosphine oxide, methylene blue, preferably in the presence of ammonium persulfate (APS) as a (further) cross-linking catalyst.

A specific local distribution of the formation of cross-links, e.g. between ULD tetramer subunits and not between different linker sequences $(L)_n$, can also be induced, e.g. by photochemical formation of dityrosine bonds between tyrosines of different ULD tetramer subunits and avoidance of crosslinks between tyrosines in the linker sequence $(L)_n$, e.g. when using elastin-like protein sequences (ELP) as defined above are used lacking tyrosines. Likewise the number of dityrosine bonds in the linker sequence $(L)_n$ can also be specifically adapted (and hence the strength of crosslinking in the linker sequence $(L)_n$), e.g. when using correspondingly selected elastin-like protein sequences (ELP) as defined above, by inserting a limited number of tyrosines into the entire linker sequence $(L)_n$ via amino acid Xaa, depending on the envisaged strength of the bond between the entire linker sequences $(L)_n$. The inventive fusion protein system is therefore highly flexible.

Notably, exploiting inherent tyrosine residues as cross-linking targets makes the system independent from subsequent chemical introduction of cross-linking sites, which is a subject to variability. Combined with the genetically encoded amino acid composition of the linker sequence (L), this approach results in highly uniform hydrogels. It is also noted that a potential de-coupling of the variable ELP linker from the cross-linking mechanism provides myriad possibilities, e.g. incorporation of polar, charged, or lipophilic amino acids and combinations thereof to also adjust the corresponding hydrogel properties with regard to the desired requirements.

In this context, the crystal structure of the ULD tetramer (see Figure 1A) revealed that each of the four interfaces between the ULD tetramer subunits harbors cross-linkable groups, most preferably two neighboring tyrosine residues which can be photochemically cross-linked with cross-linking catalysts such as riboflavin, riboflavin phosphate, Tris(bipyridine)ruthenium(II) chloride (Ru(II)bpy), 2-hydroxy-4'-(2'-hydroxyethoxy)-2-methylpropiophenone, or phenylbis(2,4,6-trimethylbenzoyl)phosphine oxide, along with an oxidant e.g. ammonium persulfate (APS) as a further cross-linking catalyst (see Figure 1) to enhance the reaction.

Accordingly, following a preferred embodiment of the invention, the fusion proteins according to any of formulae (I), (Ia), (Ib), (Ic), (Id), (Ie) and/or (If), preferably the ULD tetramers of the fusion protein according to any of formulae (I), (Ia), (Ib), (Ic), (Id), (Ie) and/or (If), are photochemically cross-linked, using typically riboflavin, riboflavin phosphate, Tris(bipyridine)ruthenium(II) chloride (Ru(II)bpy), 2-hydroxy-4'-(2'-hydroxyethoxy)-2-methylpropiophenone, or phenylbis(2,4,6-trimethylbenzoyl)phosphine oxide as a cross-linking catalyst, preferably in the presence of ammonium persulfate (APS) as a further cross-linking catalyst.

Photochemical cross-linking is preferably carried out at a wavelength of between 275 and 550 nm, preferably between 400 and 500 nm, most preferably at 460 nm.

Likewise preferably, photochemical cross-linking is preferably carried out at an illumination energy of between 2 to 3000 $J/cm^2$, preferably at an illumination energy of between 2 to 2500 $J/cm^2$, more preferably between 5 to 2000 $J/cm^2$, or even at an illumination energy of between 5 to 90 $J/cm^2$. Such an illumination energy may be adapted for the different therapies as required. For example for ophthalmic surgeries, without being limited thereto, photochemical cross-linking is preferably (but not limited thereto) carried out at an illumination energy of between 2 to 120 $J/cm^2$, preferably at an illumination energy of between 5 to 120 $J/cm^2$, more preferably between 5 to 100 $J/cm^2$, or even at an illumination energy of between 5 to 90 $J/cm^2$. Photochemical cross-linking is preferably carried out using e.g. an LED device, such as a portable "LED-pen".

The cross-linking times preferably vary between a few seconds and various minutes, e.g. between 5 seconds to 10 minutes, preferably between 5 and 360 seconds, more preferably between 5 and 240 seconds, 10 to 240 seconds or 10 to 180 seconds, even more preferably between 10 to 120 seconds and most preferably between 10 to 60 or even 10 to 30 seconds.

According to one embodiment, the inventive fusion protein according to any of formulae (I), (Ia), (Ib), (Ic), (Id), (Ie) and/or (If), or a combination thereof, and a herein defined cross-linking agent or cross-linking catalyst may be present in a weight ratio of about 1: 0.000001 to 1:3, e.g. between 1:0.00001 to 1:2, 1:0.0001: to 1:1, or, preferably 1:0.00001: to 1:1. APS may be further added to any such a protein:cross-linking catalyst ratio.

The inventive fusion protein according to any of formulae (I), (Ia), (Ib), (Ic), (Id), (Ie) and/or (If) may also be present as a combination of at least two of the fusion proteins according to any of formulae (I), (Ia), (Ib), (Ie) and/or (If) or a combination of a fusion proteins according to formula (I), (Ia), (Ib), (Ic), (Id), (Ie) and/or (If) with another network forming protein.

Such a fusion protein mixture may be used to specifically adapt the properties and the elastic modulus of the resulting fusion protein after cross-linking.

The inventive fusion protein according to any of formulae (I), (Ia), (Ib), (Ic), (Id), (Ie) and/or (If) or a fusion protein combination thereof may exhibit a Young's modulus of between 5 kPa and 10 MPa, preferably between 5 kPa and 5 MPa, more preferably between 5 kPa and 1 MPa, e.g. between 10 kPa and 400 kPa, between 300 kPa and 250 kPa, even more preferably between 10 kPa and 200 kPa or between 10 kPa and 180 kPa, wherein each of the upper/lower limits can be combined with each other. Determination of Young's moduli may occur e.g. via nanoindentation (calculated based on Hertz fit method). Therefore, preferably hydrogel pads are prepared and for each hydrogel pad, nanoindentation is performed at different spots, e.g. 1 to 20. Errors are calculated on basis of standard deviations of the measurements within one hydrogel pad.

As a general example, a fusion protein combination of at least two of the fusion proteins according to any of formulae (I), (Ia), (Ib), (Ic), (Id), (Ie) and/or (If) can be e.g. a combination of at least two fusion proteins of formula (I) and/or (Ib), (I) and/or (Ic), (I) and/or (Id), (Ia) and/or (Ib), (Ia) and/or (Ic), etc.

As an example, such a fusion protein combination of at least two inventive fusion proteins may be selected from a first fusion protein according to formula (I) and/or (Ib), wherein the linker (L) of the first fusion protein is selected from any amino acid as defined above, or collagen, elastin, fibrin, HSA, fibronectin, or an ELP sequence, etc. as mentioned above, and a second fusion protein according to formula (I) and/or (Ib), wherein linker (L) of the second fusion protein is selected from any amino acid as defined above, or collagen, elastin, fibrin, HSA, fibronectin, or an ELP sequence, etc. as mentioned above, wherein the linker (L) of the second fusion protein is different to the linker (L) of the first fusion protein. Likewise or in addition, the integer n of the second fusion protein may be or may not be identical to the integer n of the first fusion protein. As an example, the integer n of the second fusion protein may be different to the one of the first fusion protein wherein the linker (L) of the second fusion protein is identical to the one of the first fusion protein. Likewise, the integer n of both fusion proteins may be identical while the linker (L) is different between the first and the second fusion protein.

According to a further example, the inventive fusion protein combination may comprise as a first fusion protein at least two fusion proteins of formula (Ia) and/or (Ic), wherein the linker (L) is an elastin-like-protein (ELP) sequence (Val-Pro-Gly-Xaa-Gly)$_n$ as defined above. Similarly, as defined before, the elastin-like-protein (ELP) sequence (Val-Pro-Gly-Xaa-

Gly)$_n$ may be equal or different in the first and the second fusion protein. Likewise, the integer n may be equal or different in the first and the second fusion protein.

According to a further example, the inventive fusion protein combination may comprise as a first fusion protein at least one fusion protein of formula (I) and/or (Ib), combined with a second fusion protein being at least one fusion protein of formula (Ia) and/or (Ic).

Further examples can be formed accordingly.

According to any of the above-mentioned combinations, the fusion protein combination comprising at least two fusion proteins according to any of formulae (I), (Ia), (Ib), (Ic), (Id), (Ie) and/or (If) may comprise the fusion proteins in different ratios. As an example, the first fusion protein and the second fusion protein may be present in a ratio of between 1000:1-1:1000, e.g. between 500:1-1:500 between 400:1-1:400, between 300:1-1:300, between 200:1-1:200, preferably between 100:1-1:100, more preferably between 10:1-1:10, e.g. from 9:2-2:9, 8:3-3:8, 7:4-4:7, 6:3-3:6, 1:1, 1:2-2:1, etc.

According to one further embodiment, the fusion proteins of the combination as defined above may comprise the same or different elastic moduli (Young's modulus). The elastic modulus (Young's modulus) is thereby preferably as defined for each of such fusion proteins as defined in general for fusion proteins above.

As an example, the fusion protein combination may comprise at least two fusion proteins according to any of formulae (I), (Ia), (Ib), (Ic), (Id), (Ie) and/or (If), wherein the first fusion protein has a Young's modulus of between 5 kPa and 10 MPa, e.g. between 5 kPa and 5 MPa, between 5 kPa and 1 MPa, e.g. between 10 kPa and 400 kPa, between 300 kPa and 250 kPa, more preferably between 10 kPa and 200 kPa or between 10 kPa and 180 kPa. The Young's modulus of the first and the second fusion protein may be the same or different, wherein any of the above ranges may be applied for each of the first and/or second fusion protein.

Thereby, the Young's modulus of the fusion proteins in the combination, e.g. of the first and the second fusion protein, preferably differs from each other by 1 kPa-1 MPa, preferably 1-500 kPa, more preferably 1-200 kPa, e.g. by 1-10 kPa, 1-20 kPa, 1-30 kPa, 1-40 kPa, 1-50 kPa, 1-60 kPa, 1-70 kPa, 1-80 kPa, 1-90 kPa, 1-100 kPa, 1-110 kPa, 1-120 kPa, 1-130 kPa, 1-140 kPa, 1-150 kPa, 1-160 kPa, 1-170 kPa, 1-180 kPa, 1-190 kPa or 1-200 kPa, more preferably by 1-10 kPa, 1-20 kPa, 1-30 kPa, 1-40 kPa, 1-50 kPa, 1-60 kPa, 1-70 kPa, 1-80 kPa, 1-90 kPa or 1-100 kPa, e.g. 1-10 kPa, 1-50 kPa or 1-100 kPa.

According to a further embodiment, the inventive fusion protein according to any of formulae (I), (Ia), (Ib), (Ic), (Id), (Ie) and/or (If) is intended for use as a medicament, as a surgical or medical tool, preferably for use as a (biodegradable) adhesive, tissue glue, filler or sealant for biological tissues, as a vascular sealant, in tissue augmentation, in tissue repair, as hemostatic agents, etc. and may be used in a variety of open, endoscopic, and laparoscopic surgical procedures. The inventive fusion protein as described herein can also be used, e.g. in the before mentioned context, for treating medical conditions that require a coating or sealing layer to prevent the leakage of gases, liquid or solids, preferably of such a biological tissue, more preferably in the context of sealing and/or replacing a suture. The inventive fusion protein can also be used in any tissue engineering application where synthetic gel matrices are currently being utilized, typically in support of or in replacement of naturally occurring tissue. Such a tissue is typically a wet tissue. A wet tissue in the context of the present invention typically means a tissue that has about a natural moisture content as can be found in a living tissue.

Biological tissues that could be treated in the context of the present invention preferably embrace any (wet) tissue of a human or an animal, typically selected from mammals, birds, fish, or amphibian, preferably human, avian, dog, cat, horse, sheep, cow, pig, rabbit or rodents, most preferably humans. Moreover, biological tissues as defined herein to be treated in the context of the present invention typically concerns any such (wet) tissue that requires treatment due to a lesion, surgery, or other defects in the corresponding tissue, e.g. due to abrasions, lacerations, or perforations to such body tissues resulting from injuries, inflammation, or surgical procedures, and/or biological tissues that require invasive treatment, including autologous or allogenic grafting and/or suturing. Such (wet) biological tissues may occur e.g. in ophthalmologic diseases or surgery, heart diseases or surgery, intestinal diseases or surgery, liver diseases or surgery, kidney diseases or surgery, skin diseases or surgery, beauty surgery, etc.

(Wet) biological tissues as defined herein preferably comprise the skin, eyes, inner organs, such as kidney, liver, spleen, heart, the intestinal system, the endocrine system, the respiratory system, small vessels, tendons, ligament, joint capsules, nerves, dermal tissue, eye tissue, coronary tissue, muscle tissue, bone, cartilage tissue, etc.

Moreover, the inventive fusion protein according to any of formulae (I), (Ia), (Ib), (Ic), (Id), (Ie) and/or (If) can be used for treating medical conditions or supporting surgical procedures that require the formation of a suture, wherein the suture can be formed either by the inventive fusion protein itself or a conventional surgical suture, which is then preferably supported and/or sealed with the inventive fusion protein. The suture is preferably applied in the context of a biological tissue as mentioned before. The inventive fusion protein according to any of formulae (I), (Ia), (Ib), (Ic), (Id), (Ie) and/or (If) is therefore preferably used for treating, filling, connecting and/or sealing tissue lesions or surgical lesions, abrasions, lacerations, or perforations (or replacing tissue in tissue defects) in a patient in need thereof, typically as a coating or sealing layer to prevent the leakage of gases, liquid or solids, and/or for supporting or even replacing conventional surgical sutures that require additional mechanical support to increase the resistance of the new suture.

A patient in need thereof in the context of the present invention is preferably selected from mammals, birds, fish, or amphibian, preferably human, avian, dog, cat, horse, sheep, cow, pig, rabbit or rodents, etc.

As discussed already above, the elegance of the inventive fusion protein according to any of formulae (I), (Ia), (Ib), (Ic), (Id), (Ie) and/or (If) lies within the liberty to choose the linker between ULD tails freely, forming ULD-linker-ULD building blocks and offering limitless possibilities to incorporate specific amino acids to further enhance biocompatibility and to tailor biodegradability and cell instructing behavior, e.g. by incorporation of specific peptides or epitopes promoting cell adhesion or proteinase-mediated degradation, etc. The inventive fusion protein can be comprised of human protein sequences solely without the need for other hydrogel/bio-glue forming molecular entities. It is accessed recombinantly minimizing the risk for pathogens. The ULD domains used in the inventive fusion protein form stable tetramers, which can be cross-linked and the interface regions of the ULD domains and provide highly stable anchors in the tissue to be treated. Due to the presence of the linker peptide (L) both N-terminally and C-terminally flanked with a ULD domain, each of the ULD domains typically linked in a ULD tetramer to other ULD domains, prevents the existence of "loose" ends of the linker peptide (L) and facilitates the generation of a stable network in the biological tissue to be treated, already prior to cross-linking the ULD-domains in the formed ULD tetramers. Cross-linking significantly enhances such an adhesion and the "anchoring" in the treated tissue. To avoid bond failure, optimal modulus of the inventive (cross-linked) fusion protein can be adapted as disclosed above to match the substrate more perfectly, if more fine-tuning is required. The inventive fusion protein is therefore modular, versatile and customizable both on a chemical and mechanical level. Mechanical and physicochemical properties can be easily tailored as described herein, which renders the inventive fusion protein highly suitable for use as a medicament or surgical tool, preferably for use as an adhesive, glue, filler or sealant for tissues in such cases as described above.

The inventive fusion protein according to any of formulae (I), (Ia), (Ib), (Ic), (Id), (Ie) and/or (If) may also be used to adhere tissues together such as skin, inner organs, e.g. selected from kidney, liver, spleen, intestine, stomach, small vessels, nerves, dermal tissue, eye tissue, coronary tissue, muscle tissue, cartilage tissue, etc. Such biological tissues preferably concern any tissue of a human or an animal, typically selected from mammals, birds, fish, or amphibian, preferably human, avian, dog, cat, horse, sheep, cow, pig, rabbit or rodents, most preferably humans.

The inventive fusion protein according to any of formulae (I), (Ia), (Ib), (Ic), (Id), (Ie) and/or (If) may be used as an adhesive, glue, filler or sealant either *ex vivo* or in *vivo*.

Ex *vivo* applications would allow e.g. gluing or sealing or otherwise treating (wet) biological tissues with the inventive fusion protein according to any of formulae (I), (Ia), (Ib), (Ic), (Id), (Ie) and/or (If), or a combination thereof, *ex vivo*, wherein the (wet) biological tissue is preferably explanted from a patient to be treated prior to applying the inventive fusion protein to the biological tissue, and optionally re-implanted into the patient after applying the inventive fusion protein to the biological tissue. The patient may be a human or an animal as defined above, e.g. selected from mammals, birds, fish, or amphibian, preferably human, avian, dog, cat, horse, sheep, cow, pig, rabbit or rodents. Moreover, a (wet) biological tissue may be any (wet) biological tissue as defined above derived from such a patient.

Likewise, ex *vivo* applications would allow e.g. gluing or sealing or otherwise treating (wet) biological tissues with the inventive fusion protein according to any of formulae (I), (Ia), (Ib), (Ic), (Id), (Ie) and/or (If), or a combination thereof, for providing material to test biological functions of the (wet) biological tissue glued with the inventive fusion protein.

In *vivo* applications would allow e.g. gluing or sealing or otherwise treating biological tissues with the inventive fusion protein according to any of formulae (I), (Ia), (Ib), (Ic), (Id), (Ie) and/or (If), or a combination thereof, directly in the patient to be treated, e.g. during a surgical operation.

The inventive fusion protein can be used e.g. in following in *vivo* or ex *vivo* applications:

1) by applying the inventive fusion protein according to any of formulae (I), (Ia), (Ib), (Ic), (Id), (Ie) and/or (If), or a combination thereof, to the surface of a first (wet) biological tissue as defined herein, the inventive fusion protein preferably already containing a cross-linker/cross-linking agent or cross-linking catalyst as defined herein in admixture, preferably added readily prior to application of the inventive fusion protein to the first (wet) biological tissue; providing a second (wet) biological tissue as defined herein, that is pressed to or contacted with the first (wet) biological tissue; then cross-linking the fusion protein at the interface region of the first and the second (wet) biological tissue, e.g. chemically or via photochemical cross-linking reaction as described herein, e.g. by illuminating the inventive fusion protein according to any of formulae (I), (Ia), (Ib), (Ic), (Id), (Ie) and/or (If), or a combination thereof, as described herein, thereby gluing both (wet) biological tissues together; or

2) bringing a first biological tissue and a second biological tissue, both as described herein, in close juxtaposition and then applying the inventive fusion protein according to any of formulae (I), (Ia), (Ib), (Ic), (Id), (Ie) and/or (If), or a combination thereof, preferably in the presence of a cross-linking agent or crosslinking catalyst as defined herein, readily added to the inventive fusion protein prior to application, such that both the first and second tissues are contacted with the inventive fusion protein according to any of formulae (I), (Ia), (Ib), (Ic), (Id), (Ie) and/or (If), or a combination thereof, then cross-linking the tissue at the interface region as described above, e.g. chemically or by photochemical cross-linking reactions, thereby gluing both tissues together.

In addition, the inventive fusion protein according to any of formulae (I), (Ia), (Ib), (Ic), (Id), (Ie) and/or (If) can be used to fill spaces in soft and hard biological tissues that are created by disease or surgery. The inventive fusion protein may additionally be used for anchoring or affixing surgical implants, such as in prosthetic fixation, e.g., to fix a mesh to tissue during hernia repair, for ophthalmologic surgery, such as treatment of grey star (cataract disease), etc.

As a specific example, the inventive fusion protein as described herein may be used in *vivo* or *ex vivo* e.g. by applying the inventive fusion protein according to any of formulae (I), (Ia), (Ib), (Ic), (Id), (Ie) and/or (If), or a combination thereof, to a damaged biological tissue or organ, preferably to e.g. seal, glue, fill or adhere, preferably by sutureless bonds:

a) lesions of the skin or inner organs, e.g. selected from kidney, liver, spleen, intestine, stomach, heart, from small vessels, nerves, dermal tissue, eye tissue, coronary tissue, muscle tissue, cartilage tissue, etc.;

b) vascular and or other tissues or organs, preferably to stop or minimize the flow of blood;

c) thoracic tissue to stop or minimize the leakage of air;

d) gastrointestinal tract or pancreatic tissue to stop or minimize the leakage of fecal or tissue contents;

e) bladder or urethra to stop or minimize the leakage of urine;

f) dura to stop or minimize the leakage of cerebrospinal fluid; and/or

g) skin or serosal tissue to stop the leakage of serosal fluid;

h) eye tissue including the cornea, sclera, iris, etc.;

i) support and/or replace cartilage tissue, bone, tendons and ligaments;

j) support and/or replace an intervertebral disc (or intervertebral fibrocartilage);

k) coronary surgery, to fix or seal a coronary artery or a stent to coronary tissue;

l) for beauty surgery, preferably to create suture less or suture free bonds between two tissue elements, etc.

The invention also provides for an in *vivo* or ex *vivo* method for adhering gluing, sealing or adhering biological tissues together, preferably biological tissues as defined above, comprising the steps:

1) providing the inventive fusion protein according to any of formulae (I), (Ia), (Ib), (Ic), (Id), (Ie) and/or (If), or a combination thereof,

2) providing a cross-linking agent or cross-linking catalyst as defined herein,

3) mixing the inventive fusion protein and the cross-linking agent or cross-linking catalyst;

4) applying the mix of step 3) comprising the inventive fusion protein and the cross-linking catalyst and/or cross-linking agent to the surface of a first biological tissue, the first biological tissue preferably as defined herein;

5) pressing or fixing the biological tissue onto a second biological tissue, the second biological tissue preferably as defined herein, such that the interface region of the first and the second biological tissue are in contact with each other via the inventive fusion protein;

6) cross-linking the inventive fusion protein at the interface region of both tissues as described herein, e.g. via chemical or via photochemical cross-linking, thereby gluing the first and the second biological tissue together. Photochemical cross-linking is preferably carried out as described above, e.g. by illuminating the fusion protein using an illumination energy as described above, and preferably at an illumination time as described above. Photo cross-linking can be carried out e.g. using an LED, more preferably using an LED-pen.

Alternatively, the current invention provides an in *vivo* or *ex vivo* method for gluing, sealing or adhering (wet) biological tissues together, preferably (wet) biological tissues as defined above, comprising the steps:

1) bringing in close juxtaposition a first and a second piece of (wet) biological tissue as defined herein, *preferably* selected from small vessels, nerves, dermal tissue, eye tissue, coronary tissue, muscle tissue, cartilage tissue, etc.,

2) applying the inventive fusion protein according to any of formulae (I), (Ia), (Ib), (Ic), (Id), (Ie) and/or (If), or a combination thereof, *preferably* in a pre-gel state, i.e. in the presence of a cross-linking catalyst and/or cross-linking agent as defined herein without yet cross-linking the inventive fusion protein, such that both the first and second tissues are contacted with the inventive fusion protein,

3) then cross-linking the inventive fusion protein at the interface region of the first and the second (wet) biological tissue as described above, e.g. chemically or by photochemical cross-linking, with a cross-linking catalyst and/or cross-linking agent as defined herein, thereby gluing both (wet) biological tissues together.

In any of the above mentioned in *vivo* or *ex vivo* applications, the inventive fusion protein according to any of formulae (I), (Ia), (Ib), (Ic), (Id), (Ie) and/or (If), or a combination thereof, may also be mixed prior to application or crosslinking with the corresponding "pure" protein sequence as defined before for $(L)_n$, i.e. a protein sequence which does not comprise an ULD sequence or any of the further modifications as described for any of formulae (I), (Ia), (Ib), (Ic), (Id), (Ie) and/or (If). Preferably, n is as defined above and (L) is a protein or peptide as defined above. In this case, the inventive fusion protein or a combination thereof may act as a mediator of gluing or sealing and the pure ULD-free protein acts as a filler and modulator of the physicochemical properties, especially if the mixture is used to fill spaces in soft and hard biological tissues or if it necessary to match the mechanical properties of the surrounding tissue.

As an example, the inventive fusion protein according to any of formulae (I), (Ia), (Ib), (Ic), (Id), (Ie) and/or (If), or a combination thereof, may be mixed with the pure proteins $(L)_n$, e.g. wherein n is as defined above and (L) is selected from collagen, elastin, elastin-like-proteins (ELPs), fibrin, HSA, fibronectin, recombinant resilin, spider silk, mEGFP, GFP, SpyCatcher protein, etc. preferably (L) being a protein sequence of any of **SEQ ID NO: 20, 22, 24, 26, 28, 30, 32, 34, 36, 38, 40, or 42** or a sequence, having at least 75%, preferably 80% or 85%, more preferably 90%, even more preferably 95 % or most preferably 99 % sequence identity to such a protein sequence of any of SEQ ID NO: 20, 22, 24, 26, 28, 30, 32, 34, 36, 38, 40, or 42. Alternatively, the inventive fusion protein according to any of formulae (I), (Ia), (Ib), (Ic), (Id), (Ie) and/or (If), or a combination thereof, may be mixed with a pure peptide or protein comprising or consisting of any of SEQ ID NO: 43-65, as depicted above.

The inventive fusion proteins according to any of formulae (I), (Ia), (Ib), (Ic), (Id), (Ie) and/or (If), or a combination thereof, or a mixture of such inventive fusion proteins with pure proteins as defined above, can also be used to fill spaces in soft and hard biological tissues that are created by disease or surgery, particularly in ophthalmic surgery, coronary surgery, tissue surgery, beauty surgery, and surgery of cartilages or surgery of bones. The inventive fusion proteins according to any of formulae (I), (Ia), (Ib), (Ic), (Id), (Ie) and/or (If), or a combination thereof, or a mixture of such inventive fusion proteins with pure proteins as defined above, may additionally be used for anchoring or affixing surgical implants, such as in prosthetic fixation to, e.g., fix a mesh to tissue during hernia repair, for ophthalmologic surgery, such as treatment of grey star (cataract disease), etc.

Applying the inventive fusion protein according to any of formulae (I), (Ia), (Ib), (Ic), (Id), (Ie) and/or (If), or a combination thereof, or a mixture of such inventive fusion proteins with pure proteins as defined above, in the above described methods and uses may occur by any suitable means, e.g. a syringe, an endoscope, or as spray including endoscopic or laparoscopic procedures etc.

Finally, the invention also provides a kit of parts, briefly termed kit, preferably for gluing, sealing or adhering (wet) biological tissues preferably as defined herein together, comprising a fusion protein according to any of formulae (I), (Ia), (Ib), (Ic), (Id), (Ie) and/or (If), or a combination thereof, a cross-linking catalyst and/or cross-linking agent as defined herein. The kit of parts contains preferably a medical device for administering the fusion protein and/or a cross-linking catalyst and/or cross-linking agent as defined herein. The kit of parts may optionally instructions also for use. In case of a photochemical cross-linking reaction, the fusion protein according to any of formulae (I), (Ia), (Ib), (Ic), (Id), (Ie) and/or (If), or a mix thereof, and the cross-linking catalyst and/or cross-linking agent may also be provided together as a premix, wherein the cross-linking agent or cross-linking catalyst is preferably a photochemical cross-linking agent or crosslinking catalyst as defined above. The kit may also comprise an illumination device, such as an LED device or an LED pen, to allow photo-cross-linking the fusion protein as defined herein with the cross-linker as defined herein.

## Description of Figures:

**Figure 1:** Shows exemplary inventive ULD-ELP-ULD (ULD-(VPGX$_m$G)ULD) hydrogels with a versatile and customizable single-building-block cross-linking system.

**A)** Shows an ULD crystal structure according to Wang and co-workers (see supra) as available in the Protein Data Bank (access code 3TUO) with marked tyrosine residues. Left: front view of the ULD tetramer, right: top view. Tyrosine residues located at the interfaces of two subunits are marked (thick sticks; tyrosine residues of the same subunit have the same color). In each of the four interfaces, two tyrosine residues are located in close proximity to each other (marked by an asterisk).

**B)** Visualization of the water-accessible surface of the ULD tetramer, with colored tyrosine residues located within the interfaces. Upon approximation of the ULD tetramer surface that is accessible to water (via PyMol), it appears that only the tyrosine pairs located at the periphery of the ULD tetramer (tyrosine 81 of each ULD monomer) might be accessible to bulky cross-linker molecules. Accessibility of tyrosine pairs located closer to the tetramer core seems to be limited. Hence, covalent cross-linking of tyrosine residues might predominantly rely on the distal tyrosine pairs in two of the interfaces.

**C)** Overview of building blocks of an exemplary ULD-ELP-ULD library:

- ULD-(VPGVG)10-ULD (SEQ ID NO: 86 (protein sequence)),

- ULD-(VPGVG)20-ULD (SEQ ID NO: 87 (DNA sequence) and SEQ ID NO: 88 (protein sequence)),

- ULD-(VPGVG)40-ULD (SEQ ID NO: 89(DNA sequence) and SEQ ID NO: 90 (protein sequence)),

- ULD-(VPGVG)80-ULD (SEQ ID NO: 91 (protein sequence)),
  (all also abbreviated as ULD-V10-ULD, ULD-V20-ULD, ULD-V40-ULD, and ULD-V80-ULD), as well as

- ULD-(VPGDG)20-ULD (SEQ ID NO: 92 (DNA sequence) and SEQ ID NO: 93 (protein sequence)),

- ULD-(VPGHG)20-ULD (SEQ ID NO: 94 (DNA sequence) and SEQ ID NO: 95 (protein sequence)),

- ULD-(VPGSG)20-ULD (SEQ ID NO: 96 (DNA sequence) and SEQ ID NO: 97 (protein sequence)),

**D)** Scheme of the formation of ULD-based cross-links between ULD-ELP-ULD building blocks. Possible arrangements might include mesh wire fence-like patterns, intertwining (with both ULD ends of one single building block contributing to the same ULD tetramer) and a combination thereof.

**E)** Two identical pre-gel-solutions containing 20% ULD-(VPGVG)20-ULD (abbreviated as ULD-V20-ULD) in water, 0.1 mM Ru(II)bpy and 30 mM APS were prepared simultaneously. The left drop of pre-gel-solution was illuminated and cross-linked at 460 nm for 1 minute, the right drop was not illuminated and hence remained liquid. Upon subsequent exposure to UV light, the cross-linked gel drop on the left shows strong blue fluorescence, indicating dityrosine bonds. In contrast, the drop of pre-gel-solution on the right does not fluoresce, demonstrating that no dityrosine bonds are present in the pre-gel-solution prior to illumination and cross-linking at 460 nm.

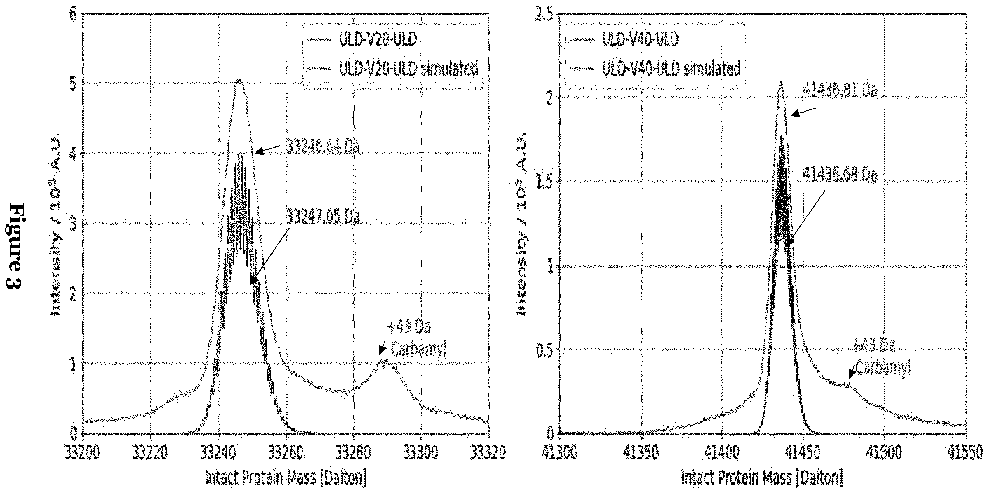

**F)** MS spectra of intact ULD-(VPGVG)20-ULD and ULD-(VPGVG)40-ULD (abbreviated as ULD-V20-ULD and ULD-V40-ULD) confirm the sequence identity of simulated and measured spectra for both proteins. Proteins were measured in 4 M urea and 50 % ethanol, preventing multimerization. The indicated measured and simulated masses only deviate slightly with 4.9 ppm for ULD-V40-ULD and 12.4 ppm for ULD-V20-ULD. The mass difference of +43 Dalton found in both proteins indicates carbamylation of one lysine residue in the ULD part of the fusion protein, as confirmed via LC-MS/MS measurement (see also Figure 3).

**Figure 2:** SDS-PAGE shows high purification yield of ULD-ELP-ULD fusion proteins via FPLC for ULD-(VPGVG) 20-ULD, ULD-(VPGVG)40-ULD and ULD-(VPGVG)80-ULD (ULD-V20-ULD, ULD-V40-ULD, and ULD-V80-ULD), as specific examples.

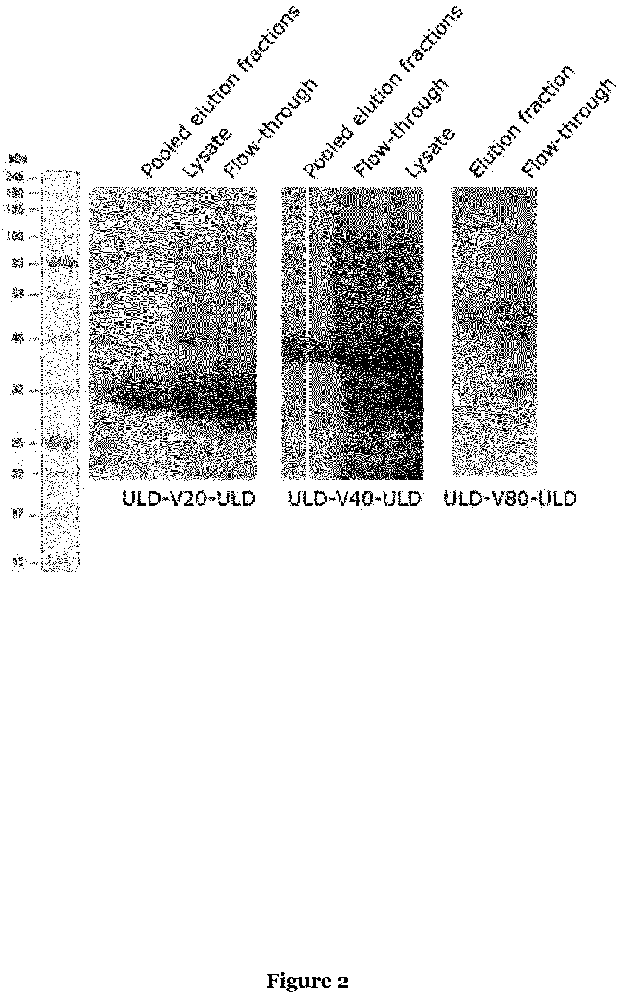

**Figure 3:** MS spectra of intact ULD-(VPGVG)20-ULD and ULD-(VPGVG)40-ULD (ULD-V20-ULD and ULD-V40-ULD) confirm sequence identity and show high purity. The simulated and measured masses deviate to a negligible extent (4.9 ppm for ULD-V40-ULD and 12.4 ppm for ULD-V20-ULD). An additional, small peak / shoulder at a +43 Da shift indicates lysine carbamylation, as confirmed via LC-MS/MS. Longer exposure time to 4 M urea lysis buffer promotes carbamylation, as was seen after evaluation of MS/MS measurements of both the tryptic peptides (see also Figure 2) and the intact proteins after 4 days of exposure in urea buffer (data not shown). Therefore, an uninterrupted purification procedure making use of ammonium containing buffers is recommended to prevent undesired protein modifications[1]. Besides the target fusion proteins, no other peptides were found in the UV 214 nm chromatogram, confirming high-grade target protein purity.

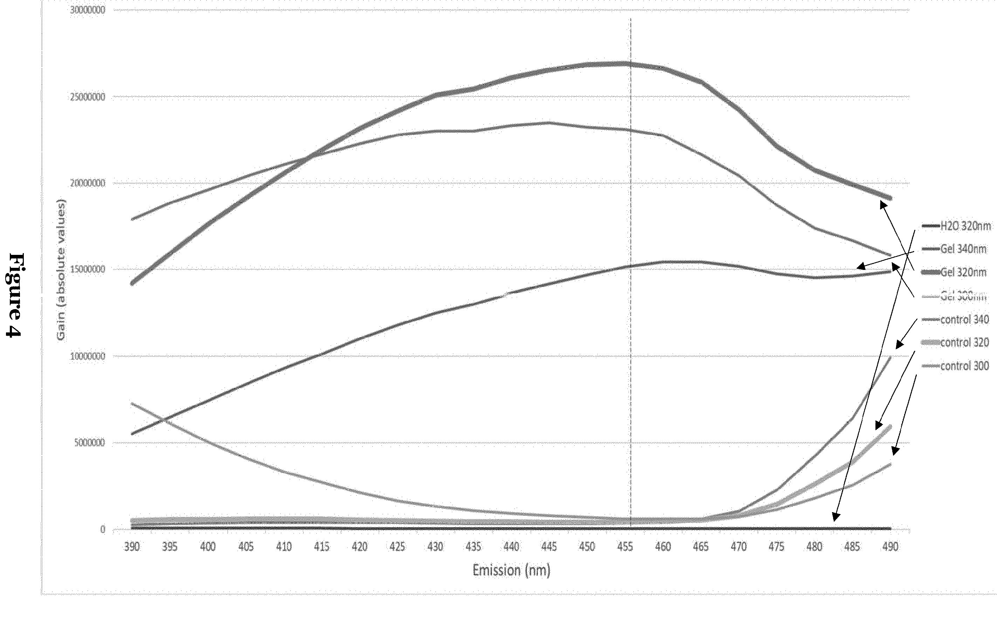

**Figure 4:** Emission of the cross-linked hydrogel and respective controls at different excitation wavelengths. A hydrogel of ULD-(VPGVG)20-ULD (ULD-V20-ULD), 20% protein prepared in water and cross-linked with 2.5 mM riboflavinphosphate / 30 mM APS was used. An uncross-linked pre-gel-solution of identical composition (without cross-linking illumination) served as a control. 320 nm were chosen for excitation determined emission at 455 nm. At these settings, the cross-linked hydrogel (first three lines from top in Figure 4, Gel 320 nm, 300 nm, 340 nm) exhibit an emission maximum. At the same time, the control (identical pre-gel-solution, not cross-linked, third line from bottom at 490 nm) shows an emission minimum. Other excitation wavelengths were also deemed suitable, even though lower in the emission maximum, which remained at about 455 nm. Applying these wavelengths, relative amounts of dityrosine were determined within cross-linked hydrogels to identify targets for adjustment of the hydrogel's properties. Each sample was prepared and measured in triplicates. A single sample was measured at 32 spots within the well. Due to the convex surface of the gel drop, spots at the center of the well were taken into account for analyses and standard deviation.

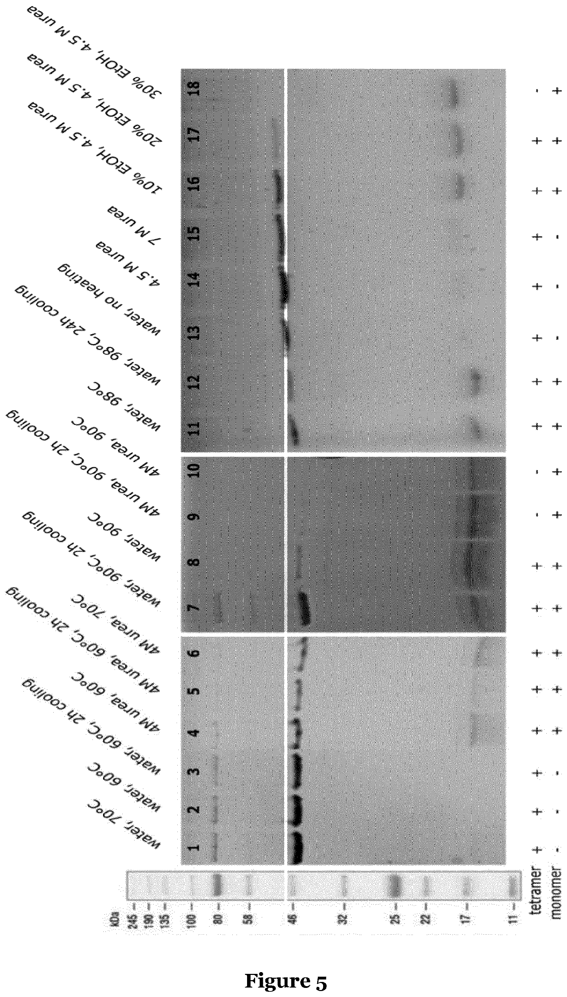

**Figure 5:** Shows ULD tetramers and tetramer disruption with heating or addition of ethanol and urea, visualized via SDS-PAGE.

**Figure 6:** Shows dityrosine cross-link formation in dependence of APS concentration, illumination energy and hydrogel composition. Each sample was measured in triplicates. Error bars indicate standard deviation of triplicates.

**A)** ULD-(VPGVG)20-ULD (ULD-V20-ULD) (20% protein prepared in water, 2.5 mM riboflavinphosphate, 30 mM APS) was illuminated for 15 to 90 seconds.
**B)** ULD-(VPGVG)20-ULD (ULD-V20-ULD) (20% protein prepared in water, 2.5 mM riboflavinphosphate, illuminated with 5.8 J/cm$^2$) was cross-linked at different APS concentrations. Dityrosine formation benefits from rising APS concentrations up to 40 mM, higher APS concentrations seem to be detrimental.
**C)** All hydrogel variants contained 2.5 mM riboflavinphosphate, 30 mM APS and were illuminated at 5.8 J/cm$^2$.

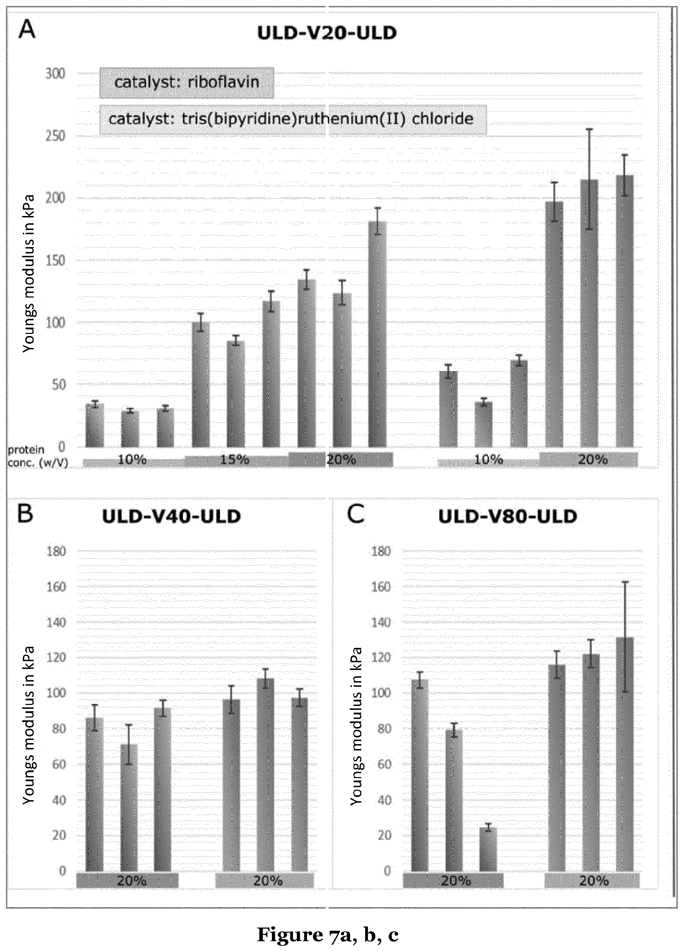

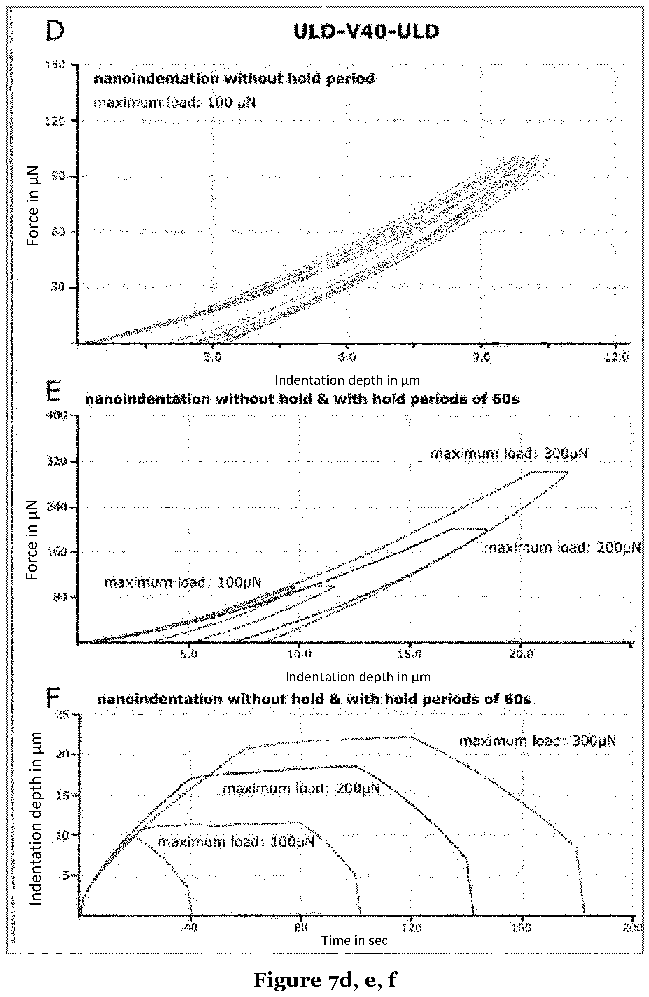

**Figure 7:** Depicts micro-elasticity of ULD-ELP-ULD hydrogels as determined via nanoindentation.

**A-C)** Young's moduli determined via nanoindentation (calculated based on Hertz fit35) for ULD-(VPGVG)20-ULD (ULD-V20-ULD hydrogel), and B) ULD-(VPGVG)40-ULD and ULD-(VPGVG)80-ULD hydrogels (ULD-V40-ULD and ULD-V80-ULD hydrogels) (C). For each hydrogel type, 3 hydrogel pads were prepared independently. All gels were prepared in ddH2O with 10, 15 and/or 20% w/v protein concentration. Moreover, all gels were prepared with 30 mM APS. The first 6 gels in A), and the first 3 gels in B) and C) additionally contained 2.5 mM riboflavinphosphate, whereas the last 6 gels in A), and the last three gels in B) and C) additionally contained 0.1 mM Ru(II)bpy. For each hydrogel pad in A) and B) nanoindentation was performed at 15 different spots. Each bar in A) and B) represents one hydrogel pad with 15 measured spots. Error bars indicate standard deviations of the 15 measurements within one hydrogel pad.
**D-E)** Force-displacement graphs and displacement-time graphs for various measurements of a single sample of ULD-(VPGVG)40-ULD (ULD-V40-ULD), 20 % protein, cross-linked in water with 2.5 mM riboflavinphosphate and 30 mM APS. Nanoindentation measurements were performed in load-controlled mode at a constant loading and unloading rate. Each curve depicts a single measurement (one of 15 measured spots within one hydrogel pad). Positions of single measurements are given by a 5x3 matrix. D) Force-displacement-graphs with maximum load 100 μN, without hold periods. E) Force-displacement curves with differing maximum loads (100 μN, 200 μN, 300 μN), with and without a hold period of 60 seconds. Varying maximum loads and inclusion of a hold period do not affect the hydrogel's small-scale-mechanical behavior considerably.

**F)** Displacement-time-curves for varying maximum loads, with and without a hold period, demonstrating mild creeping behavior of hydrogels during the hold phase.

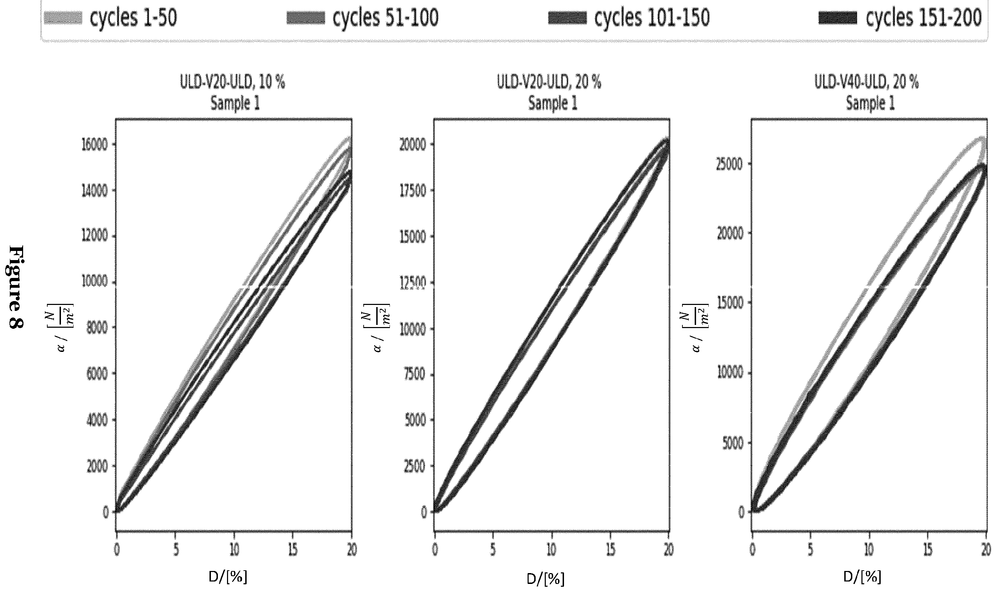

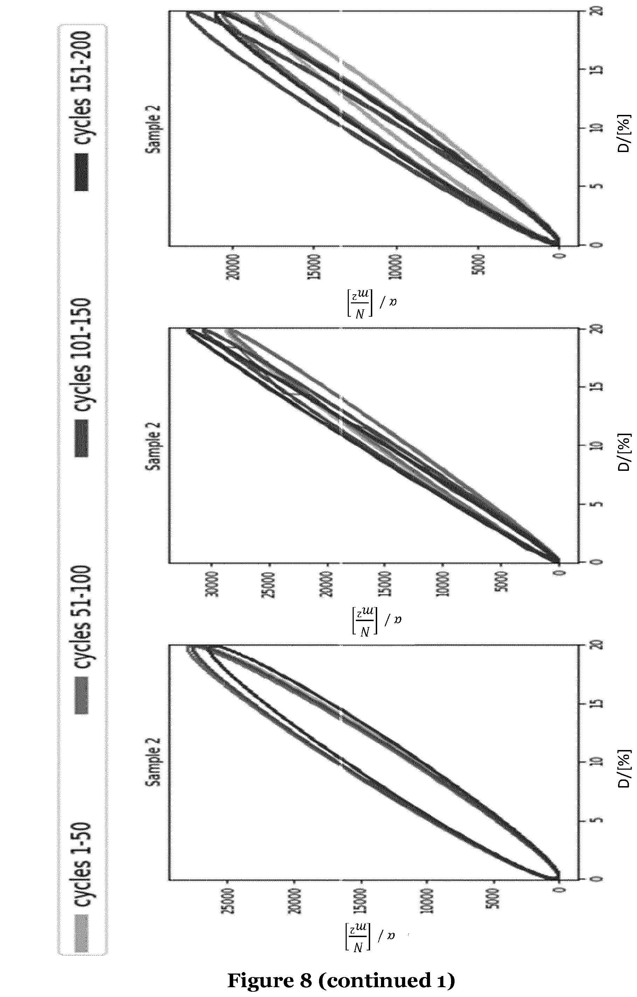

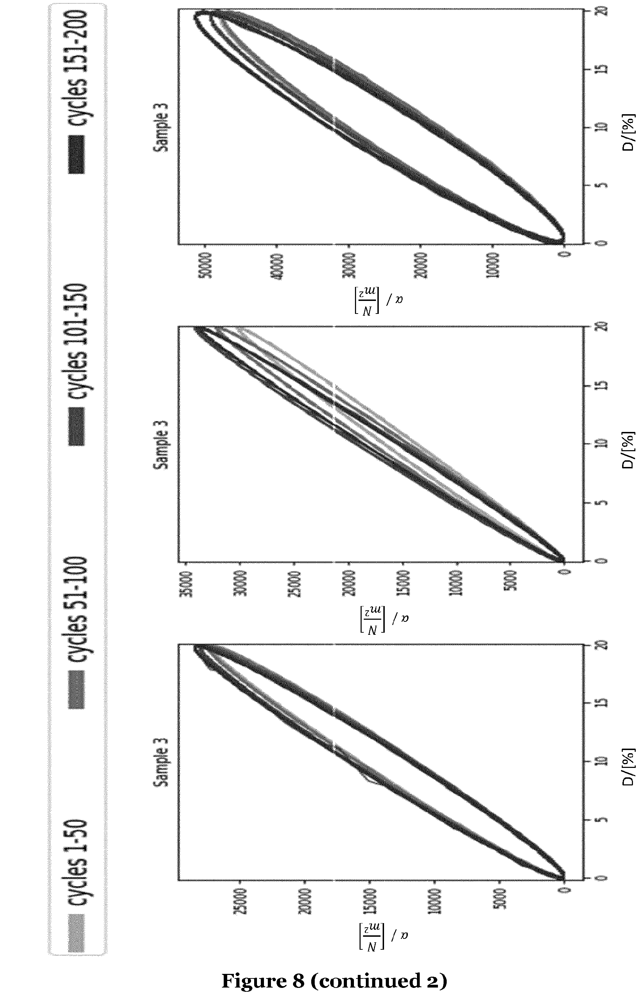

**Figure 8:** Uniaxial tensile stretch data show robust hysteresis, high elasticity and no evidence of fatigue on a macroscopic scale. Left panel: stress-strain curves for ULD-(VPGVG)20-ULD (ULD-V20-ULD), 10 % protein, middle: ULD-V20-ULD, 20 % protein, and right panel: ULD-(VPGVG)40-ULD (ULD-V40-ULD), 20 % protein. Each hydrogel was cross-linked in 4 M urea, with 2.5 mM riboflavinphosphate and 30 mM APS. For each hydrogel type, a film was cast and cut into three slices of approximately 10 mm x 5 mm (each slice = one sample). Measurements were carried out in PBS buffer at 37 °C. Each graph shows measurement data of one hydrogel sample. Each sample underwent cyclic stretching and relaxation with maximum displacement set to 20% at a frequency of 1 Hz for a total of 200 cycles. For each sample, 4 subgroups consisting of 50 cycles each have been isolated from the corresponding dataset and undergone filtering. All stress-strain data points for each subgroup are plotted (200 cycles, 8000 data points), hence no average values and confidence bands are presented. However, as the discrete fourier transform returns a unique power spectral density for each window (i.e. subgroup) it is applied to, it returned slightly varying power spectral densities for each subgroup. This may be due to noise and machine drift, which in turn result in small differences in stress-strain curve slopes.

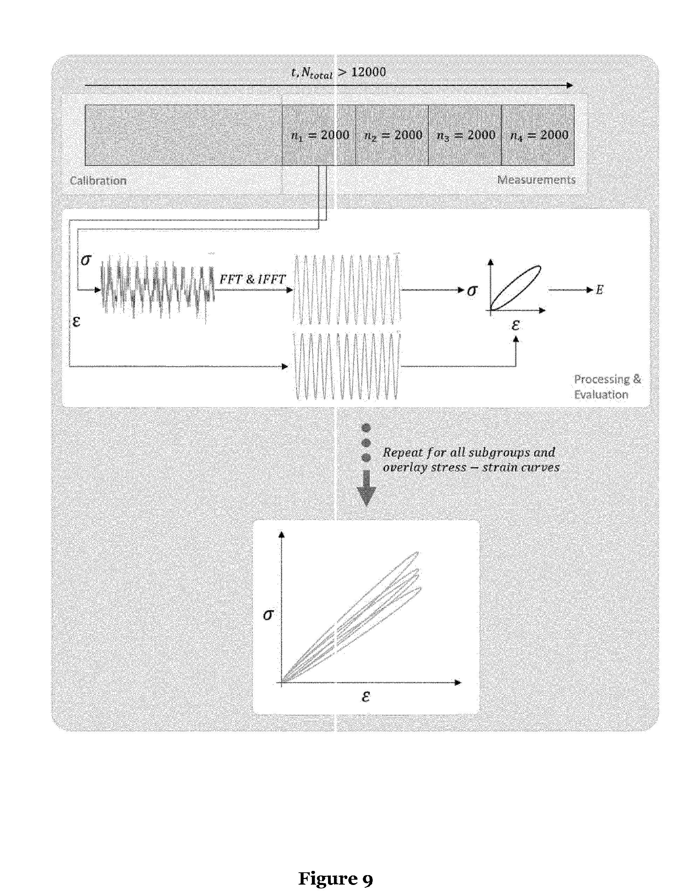

**Figure 9:** Depicts the sequence of data processing. Data sets for each measurement contained at least 12,000 data points, including the data points already recorded during the calibration and equilibration phase. The initial 4000 data points were hence discarded, leaving 4 subgroups of 2000 data points each, corresponding to 50 cycles per subgroup. Recorded force values were denoised via fast fourier transform for each subgroup. Based on denoised force values, the corresponding stress was calculated and plotted as a function of the given displacement (= strain). Thus, for every recorded force-strain data point, the corresponding stress-strain value was plotted accordingly. Young's modulus was calculated based on the slope of the secant of the resulting stress-strain curve for each subgroup of a given sample. Mean Young's moduli (average of Young's moduli of the four subgroups) are shown for each hydrogel sample in Table 2.

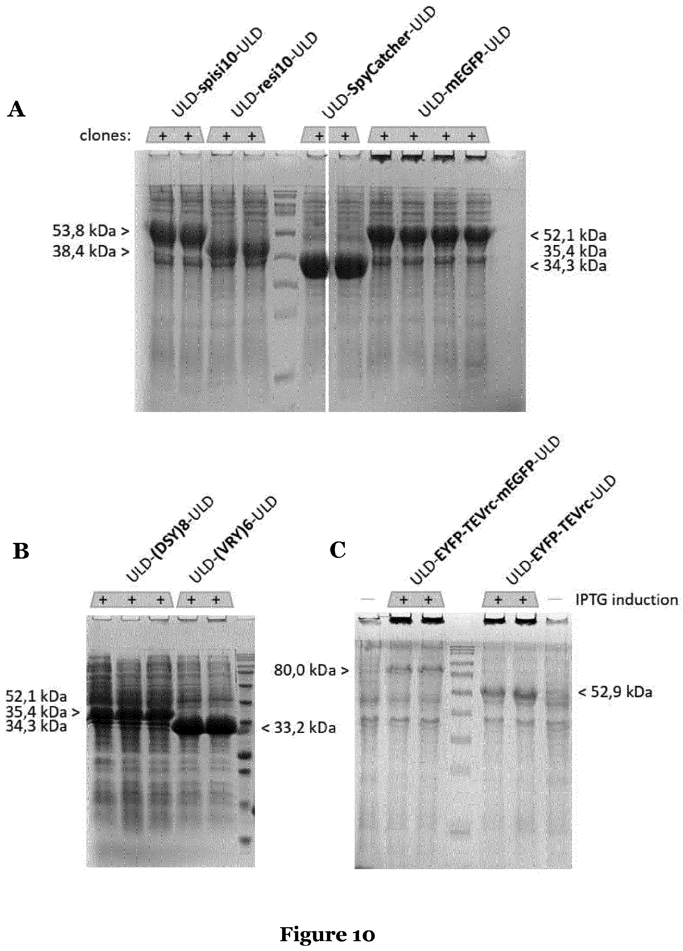

**Figure 10**: **Shows examples of expressed ULD-Linker-ULD protein glue constructs representing different mechano-chemical properties** Demonstration of efficiently expressed different ULD-based protein glues via SDS-PAGE of cloned, sequenced and expressed ULD-Linker-ULD constructs comprising different possible proteinaceous linker sequences. These constructs demonstrate different possible molecular weight linker, linker-length, class and possible complexity and functionality of protein linker (globular, disordered, fibrous, degradability, adapter). From A) to C) two to four different clones of each exemplary construct was expressed via IPTG induction of *E. coli* cells. All constructs were cloned in a high-expressing pET28-NMBL-vector as pET28-NMBL-ULD-Linker-ULD-His construct (see Huber et al., Biomaterials, Volume 35, Issue 31, October 2014, Pages 8767-8779 2014). ULD-spisi10-ULD and ULD-resi10-ULD comprise fibrous linkers sequences. ULD-SpyCatcher-ULD contain an adapter linker sequence that could be complemented with SpyTag functionalized molecules. ULD-mEGFP-ULD construct demonstrate a globular green fluorescence protein as linker for visible functionalization. ULD-(DSY)8-ULD and ULD-(VRY)6-ULD include disordered and photo-crosslinkable linker sequences. Finally, ULD-EYFP-TEVrc-mEGFP-ULD as well as ULD-EYFP-TEVrc-ULD comprise linker sequences that combine fluorescent visibility with possible network degradability properties via TEVprotease recognition sequence motif (TEVrc).

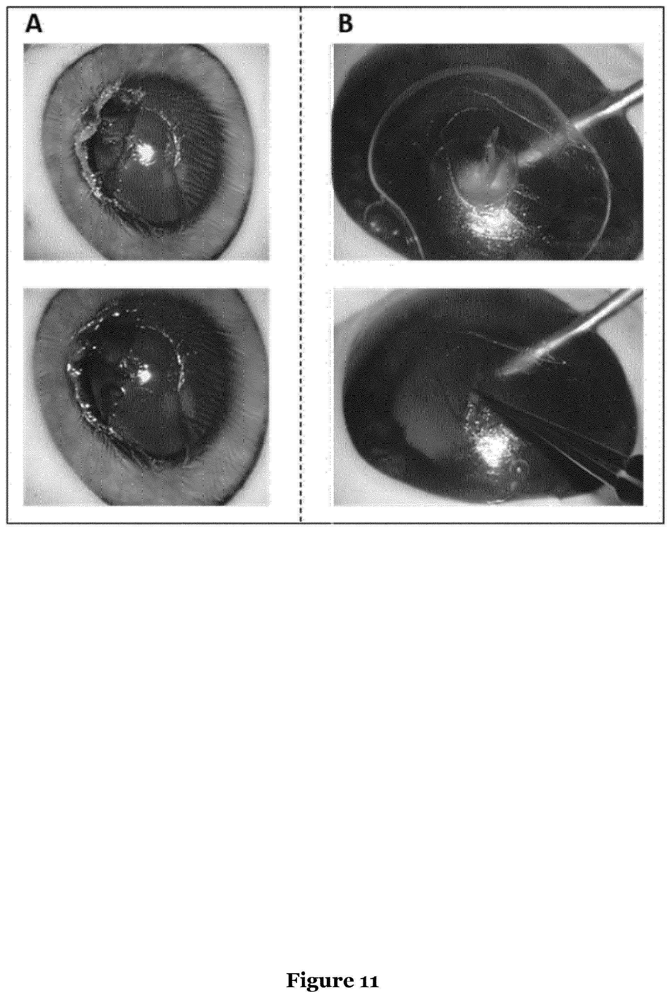

**Figure 11:** **ULD-ELP-ULD hydrogels seal corneal defects effectively and demonstrate extremely high adhesion to corneal surface.** For both experiments shown here, we used extracted porcine eyes and ULD-(VPGVG)40-ULD (ULD-V40-ULD) hydrogels (20% protein, cross-linked with riboflavinphosphate and 30 mM APS, duration of illumination at 460 nm 30 seconds [total illumination energy 5.8 J/cm$^2$]). The pre-gel-solution was prepared freshly before each application and 2-3 $\mu$l were applied into the defect or on top of the incision.

**A)** Eye with inflicted stromal defect, in part de-epithelialized; top: before treatment; bottom: after filling the defect with hydrogel and cross-linking. The defect was incubated with 50 mM riboflavinphosphate for 3 minutes prior to filling it.

**B)** Eye with inflicted full-thickness corneal incision of 2.2 mm length. The eye was filled with a viscous solution of 1 % sodium hyaluronate (Z-HYALIN®) and a small amount of air was inserted through a paracentesis to prevent liquid from leakage. A pre-polymerized hydrogel film (the same hydrogel as was used for filling and sealing defects) with a punch of approximately 5 mm diameter was positioned around the incision and filled with the pre-gel-solution (see Figure 11). Top: Cross-linked hydrogel patch sealing the full-thickness corneal incision up to intraocular pressures of 100 mmHg, both before and after removal of the air. Bottom: The hydrogel patch adheres strongly to the cornea, removal of the patch results in tearing of the hydrogel.

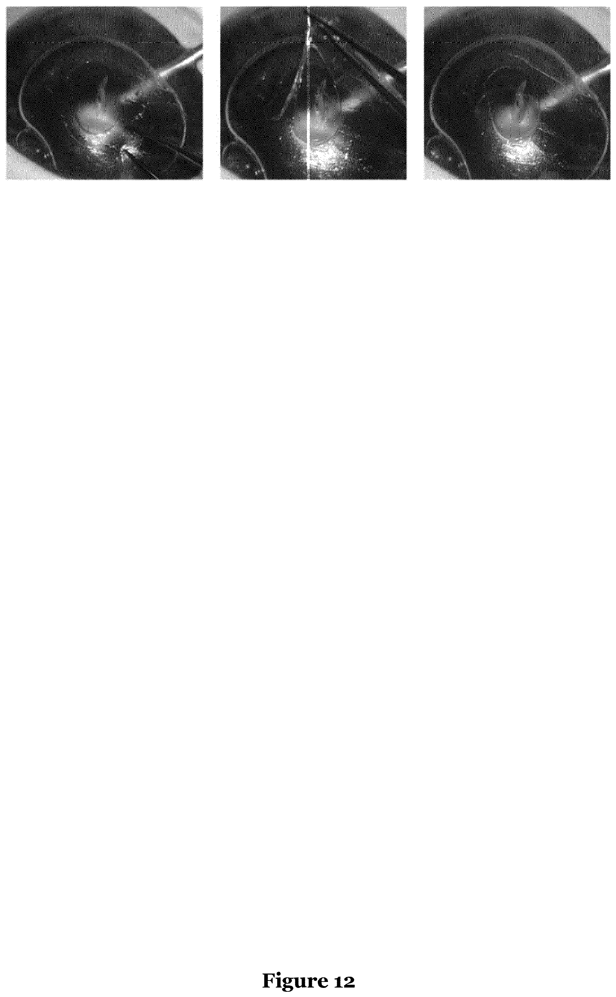

**Figure 12:** Creating adherent hydrogel patches to seal full-thickness corneal incisions, utilizing a hydrogel mold to prevent the liquid solution from flowing away. A pre-polymerized hydrogel film with a punched hole of about 5 mm diameter - serving as a mold - was positioned around the incision and filled with a pre-gel-solution of ULD-(VPGVG)40-ULD (ULD-V40-ULD) (2.5 mM riboflavinphosphate, 30 mM APS). The hydrogel was cross-linked within 30 seconds. The pre-polymerized hydrogel mold was then removed, leaving behind an adherent hydrogel patch sealing the incision.

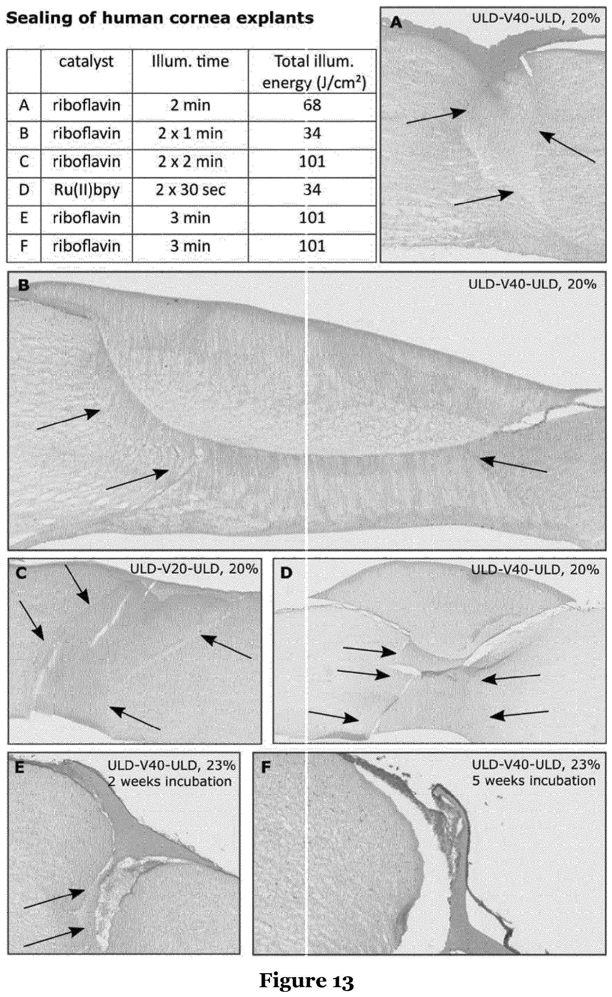

**Figure 13:** ULD-ELP-ULD hydrogels coalesce with human cornea tissue and do not show signs of cytotoxicity. Hematoxylin-eosin (HE)-stained cryotome preparations of human cornea explants. Human donor corneas were cut into strips and resealed with ULD-(VPGVG)20-ULD and ULD-(VPGVG)40-ULD (ULD-V20-ULD and ULD-V40-ULD), as indicated. Black arrows mark the borderline between the cornea and the hydrogel, showing that the two materials coalesce and demonstrating interpenetration of the sealing hydrogel and corneal tissue. For samples shown in B-D, two layers of hydrogel were applied and cross-linked consecutively, showing that the different hydrogel layers conglomerate. Cracks in the hydrogel fillings, as seen in C and D, were partly induced by the cryotome preparation procedure. E and F demonstrate cell survival after cutting and re-sealing procedures as well as promising cell viability thereafter, with appreciable colonization of hydrogel sealings by epithelial cells.

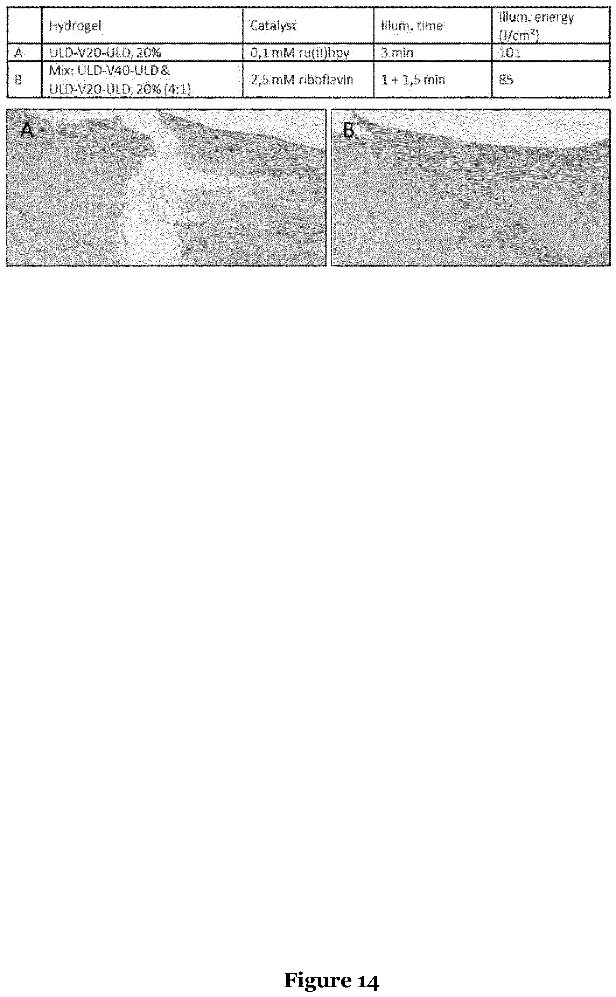

**Figure 14:** Shows results, which suggest that ULD-ELP-ULD gels demonstrate favorable biocompatibility, even when cross-linked with Ru(II)bpy and upon application of high illumination energies (A). Mixtures of ULD-(VPGVG)40-ULD (ULD-V40-ULD) with ULD-(VPGVG)20-ULD (ULD-V20-ULD) present a dense texture, comparable with ULD-(VPGVG)20-ULD (ULD-V20-ULD) based hydrogels (B).

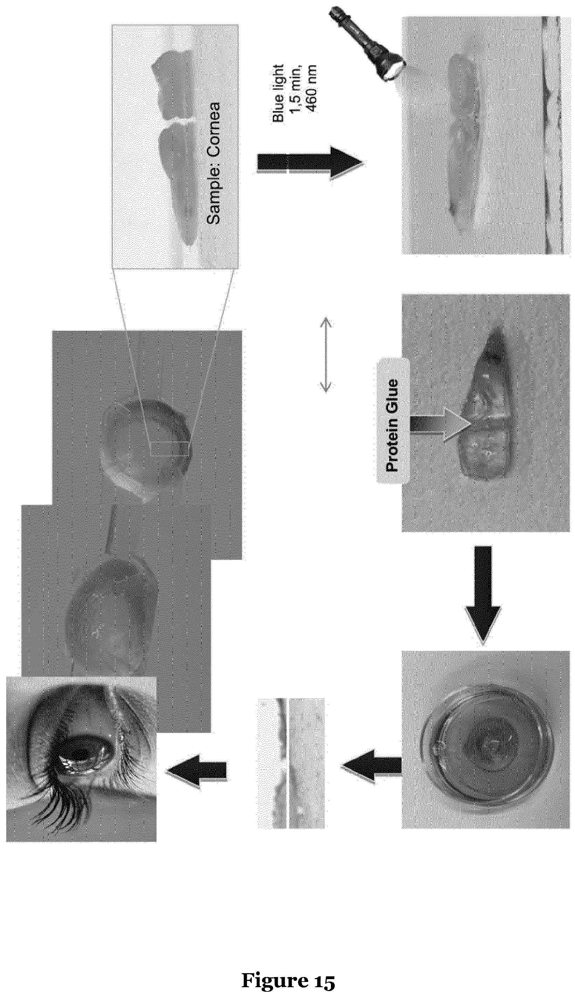

**Figure 15:** Shows the sealing of a lesion in the human cornea with ULD-(VPGVG)40-ULD (2.5 mM riboflavinphosphate, 30 mM APS). The hydrogel was cross-linked under blue light at 460 nm for 1.5 minutes.

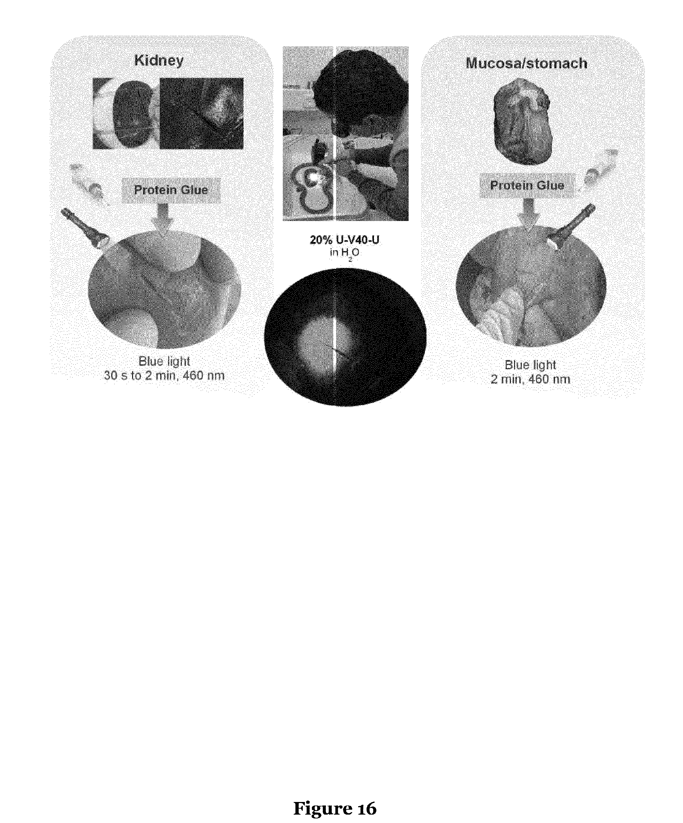

**Figure 16:** Shows the sealing of a lesion in the human kidney and mucosa/stomach with ULD-(VPGVG)40-ULD (2.5 mM riboflavinphosphate, 30 mM APS). The hydrogel was cross-linked under blue light at 460 nm for 2 minutes.

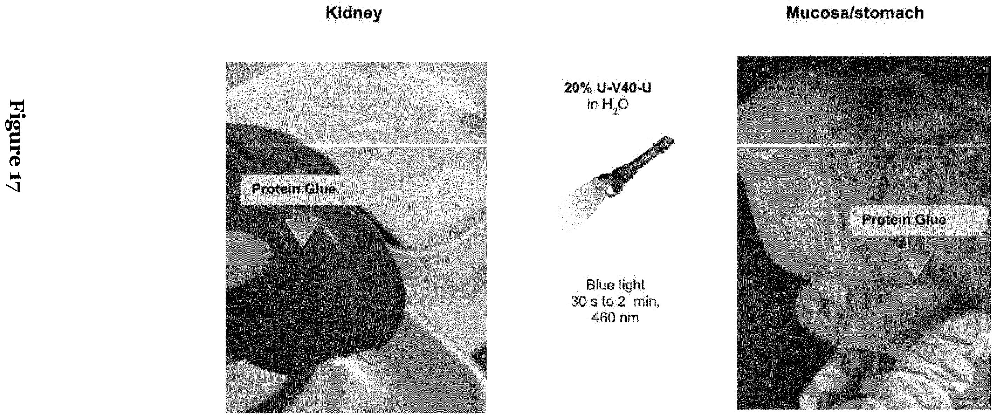

**Figure 17:** Shows the sealing of a lesion in the human kidney and mucosa/stomach with ULD-(VPGVG)40-ULD (2.5 mM riboflavinphosphate, 30 mM APS). The hydrogel was cross-linked under blue light at 460 nm for 30 sec. to 2 minutes.

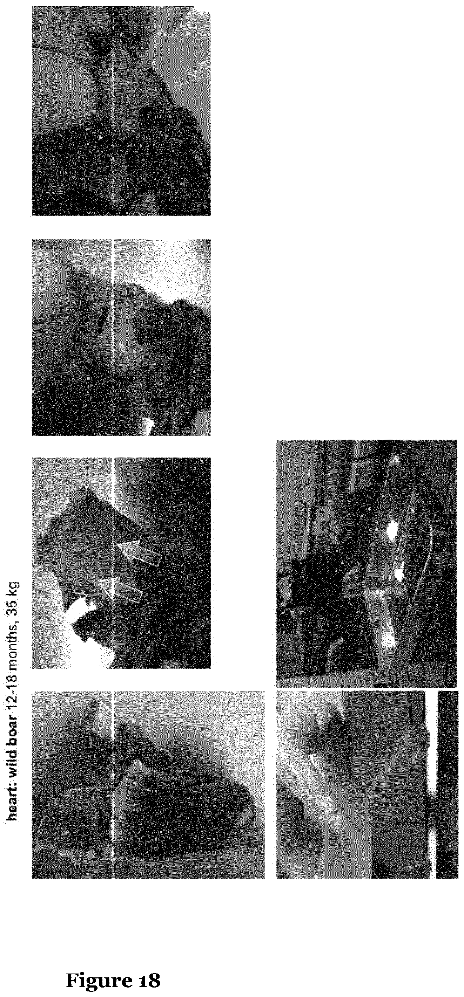

**Figure 18:** Shows the sealing of a heart valve in the heart of a wild boar with ULD-(VPGVG)40-ULD (2.5 mM riboflavinphosphate, 30 mM APS). The hydrogel was cross-linked under blue light at 460 nm for 30 sec. to 2 minutes.

**Experimental data:**

1. Genetic engineering of ELP fusion proteins according to formula (I), (Ia), (Ib) and (Ic)

In a first attempt a preliminary library of ULD-(VPGXG)$_n$-ULD building blocks according to formula (Ia) was created by cloning repetitive ELP sequences of varying lengths and inserting different amino acids at the 4$^{th}$ position (X) of the ELP pentamers, namely V, H, D, R and S:

- ULD-(VPGVG)10-ULD **(SEQ ID NO: 86** (protein sequence)),

- ULD-(VPGVG)20-ULD **(SEQ ID NO: 87** (DNA sequence) and **SEQ ID NO: 88** (protein sequence)),

- ULD-(VPGVG)40-ULD **(SEQ ID NO: 89**(DNA sequence) and **SEQ ID NO: 90** (protein sequence)),

- ULD-(VPGVG)80-ULD **(SEQ ID NO: 91** (protein sequence)),
  (all also abbreviated as ULD-V10-ULD, ULD-V20-ULD, ULD-V40-ULD, and ULD-V80-ULD), as well as

- ULD-(VPGDG)20-ULD **(SEQ ID NO: 92** (DNA sequence) and **SEQ ID NO: 93** (protein sequence)),

- ULD-(VPGHG)20-ULD **(SEQ ID NO: 94** (DNA sequence) and **SEQ ID NO: 95** (protein sequence)),

- ULD-(VPGSG)20-ULD **(SEQ ID NO: 96** (DNA sequence) and **SEQ ID NO: 97** (protein sequence)),

Furthermore, a ULD-(VPGVG)$_{121}$-ULD was produced.

The generated exemplary repeat numbers n (integer n) of the pentamer motif (VPGXG)$_n$ therefore varies from n = 10 to n = 120 for different exemplary ULD-(VPGXG)$_n$-ULD variants (see also Figure 1C).

For the purpose of the inventive Examples and Figures ULD-(VPGXG)$_n$-ULD variants can be exceptionally abbreviated also as "ULD-X$_n$-ULD", wherein X = the respective amino acid aa in one letter code, representing the entire pentamer motif VPGXG. In any of these cases fusion proteins according to formula (I), (Ia), (Ib) and (Ic) are meant.

The recombinant generation of the described cross-linkable fusion protein was generally performed following the mode of preparation as outlined in EP 2 668 962, EP 2 854 863, US 1,0428,137 or WO 2013/17862. The Ubiquitin-like domain (ULD) of the human global gene organizer SATB1 (Special AT-rich sequence-binding protein 1; M97287)(see Zhang X, Chu X, Wang L, Wang H, Liang G, Zhang J, et al. Rational Design of a Tetrameric Protein to Enhance Interactions between Self-Assembled Fibers Gives Molecular Hydrogels. Angew Chemie. 2012;124(18):4464-8., and Wang Z, Yang X, Chu X, Zhang J, Zhou H, Shen Y, et al. The structural basis for the oligomerization of the N-terminal domain of SATB1. Nucleic Acids Res. 2012;40(9):4193-202) was codon optimizied for E.coli expression (see Grote A, Hiller K, Scheer M, Münch R, Nörtemann B, Hempel DC, et al. JCat: A novel tool to adapt codon usage of a target gene to its potential expression host. Nucleic Acids Res. 2005;33(SUPPL. 2):526-31) and for the implementation into the OVTP cloning system (see Huber MC, Schreiber A, Wild W, Benz K, Schiller SM. Introducing a combinatorial DNA-toolbox platform constituting defined protein-based biohybrid-materials. Biomaterials [Internet]. 2014;35(31):8767-79. Available from: http://www.sciencedirect.com/science/article/pii/S0142961214007546). It was subsequently ordered as a synthetic gene from Invitrogene (Thermofisher Scientific).

The ULD sequence was complemented with additional DNA sequences for adapting to the NMBL-linker region of the OVTP system (see above patents and DNA sequence below). The synthesized ULD domain (according to SEQ ID NO: 1) was cloned into a pET28-NMBL-His vector via SacI / EarI / BspQI restriction sites as described in Huber et al. 2014, supra, to generate pET28-NMBL-ULD-His and then hereinafter completed with a compatible Linker-module (L)n or exemplarily (VPGVG)20 to pET28-NMBL-(L)n-ULD-His followed by the addition of another compatible ULD module to pET28-NMBL-ULD-(L)n-ULD-His (or exemplarily pET28-NMBL-ULD-(VPGVG)20-ULD-His).

The Linker module could be seen as a placeholder for all previously described (OVTP compatible) DNA sequences (e.g. HSA, ELPs, recomb. resilin ...). Additionally as intermediate steps before and after the insertion of the (L)n module the implementation of optional FPL "peptide linker sequences" (cf. also corresponding DNA sequences) could be executed. The resulting pET28-NMBL-ULD-(L)n-ULD-His vectors could be directly transformed into E.coli expression cells (e.g. BL21(DE3), BLR etc.) as described in Huber et al. 2014, supra, and expressed and purified as described elsewhere (see e.g. Sambrook J, Russell DW. Molecular Cloning: A Laboratory Manual. 3rd Cold Spring Harbor Laboratory Press. New York. 2001)

Similarly, the following fusion proteins were produced:

ULD-(VPGKG)20-ULD **(SEQ ID NO: 98** (DNA sequence) and **SEQ ID NO: 99** (protein sequence)),

ULD-(VPGRG)20-ULD **(SEQ ID NO: 100** (DNA sequence) and **SEQ ID NO: 101** (protein sequence)),

ULD-(VPGDG-VPGSG-VPGYG)8-ULD **(SEQ ID NO: 102** (DNA sequence) and **SEQ ID NO: 103** (protein sequence)),

ULD-(VPGVG-VPGRG-VPGYG)6-ULD **(SEQ ID NO: 104** (DNA sequence) and **SEQ ID NO: 105** (protein sequence)),

ULD-(mEGFP)-ULD **(SEQ ID NO: 106** (DNA sequence) and **SEQ ID NO: 107** (protein sequence)),

ULD-(mCHR-TEVrec-mEGFP)-ULD **(SEQ ID NO: 108** (DNA sequence) and **SEQ ID NO: 109** (protein sequence)),

ULD-(spisi10)-ULD (**SEQ ID NO: 110** (DNA sequence) and **SEQ ID NO: 111** (protein sequence)),

ULD-(resi10)-ULD (**SEQ ID NO: 112** (DNA sequence) and **SEQ ID NO: 113** (protein sequence)),

ULD-(hsa)-ULD (**SEQ ID NO: 114** (DNA sequence) and **SEQ ID NO: 115** (protein sequence)), etc.

2. Protein expression, purification and quantification

ELP fusion proteins according to any of formulae (I), (Ia), (Ib) and (Ic) were prepared comprising at the C-terminus and the N-terminus ULD sequences according to SEQ ID NO: 1 with a peptide chain, in the context of these examples an ELP chain in-between. Protein expression, purification and quantification were carried out according to the following protocol:

*E. coli* strains BL21(DE3) as prepared according to Example 1 were used for protein expression. Starter cultures of 2YT or Terrific Broth (TB) medium and 40 $\mu$g/mL Kanamycin were inoculated with cells from DMSO stocks. Bacteria were grown while shaking at 250 rpm and 37°C over night. The next morning, 2 mL starter culture was centrifuged for 4 min at 4000 rpm and resuspended in 1 mL fresh 2YT or TB medium as described by Quiroz and Chilkoti (see Quiroz, F. G. & Chilkoti, Nat. Mater. 14, 1164-1171 (2015)). 400 mL fresh medium was inoculated with the resuspended starter culture and bacteria were grown for about 8 hours at 37°C while shaking at 180 rpm. Protein expression was induced by adding IPTG to a final concentration of 1 mM and temperature was reduced to 20 °C. Cells were then harvested after ca. 20 hours by centrifugation at 4000 rpm and 4 °C. For cell lysis, a lysis buffer was used containing 50 mM Tris-HCl, 500 mM NaCl, 4M urea and 20 mM imidazole, pH 7.5, to resuspend the cell pellet. Lysozyme was then added to a final concentration (f.c.) of 0.1 mg/mL, PMSF (f.c. 1 mM) and TCEP (f.c. 1 mM) and the resuspended cells were incubated for 30 min on ice. After 2 times of freezing the suspension in liquid nitrogen, thawing and incubation with DNAse I in between the freezing cycles, the suspension was sonified and centrifuged at 10,000 g and 4 °C for 30 min. The supernatant containing the desired protein was then purified on a HisTrap FF crude column using Äkta FPLC purifier system connected to a fraction collector (F9-R, GE Healthcare). Absorbance was measured at 280 nm and analyzed using Unicorn 6.3 software. The column was equilibrated with Tris-buffer (50 mM Tris-HCl, 500 mM NaCl, 4 M urea, 20 mM imidazole). Following the loading of the lysate, washing was performed (50 mM Tris-HCl, 500 mM NaCl, 4 M urea, 20 mM imidazole). His-tagged proteins were eluted using an elution buffer containing 500 mM imidazole and elution fractions were pooled. For fusion proteins, e.g. ULD-(VPGVG)$_{20}$-ULD and ULD-( VPGVG)$_{40}$-ULD, inventors determined the protein concentration of the pooled elution via absorbance at 280 nm using the spectrophotometer Nanodrop 1,000 (Peqlab Biotechnology GmbH). Beer-Lambert law was applied to calculate the protein concentration using a calibration curve that was previously set up for each protein separately by weighing lyophilized protein and measuring absorbance at different protein concentrations. All other proteins were lyophilized and the required amount was weighed immediately before hydrogel fabrication. For purity analysis and identification, standard SDS-PAGE (Tris/glycine, 10%) was applied (see Figure 2). Following dialysis into 1 mM Tris-HCl buffer pH 8 to remove salts and remaining compounds from His-tag purification, aliquots were prepared with defined protein amounts, followed by lyophilization. Protein aliquots were stored at -20°C.

3. Hydrogel fabrication

Lyophilized protein aliquots as prepared according to Example 2 with known protein quantities were dissolved in MilliQ water, 4M urea or PBS, depending on the protein and the desired application. In case of hydrogel pads for nanoindentation, MilliQ water (for ULD-(VPGVG)$_{20}$-ULD and ULD-(VPGVG)$_{4}$,-ULD) or 4M urea (for ULD-(VPGVG)$_{80}$-ULD and ULD-(VPGVG)$_{120}$-ULD) was used. For corneal sealing experiments, MilliQ water or PBS was used. As soon as the protein was dissolved homogeneously, riboflavinphosphate (stock solution 50 mM) or Tris(bipyridine) ruthenium(II) chloride (Ru(II)bpy; stock solution 10 mM) was added to a final concentration of 2.5 mM riboflavinphosphate or 0.1 mM Ru(II)bpy, followed by brief vortexing and centrifugation, then ammonium persulfate (APS) (stock solution 1 M) was added to a final concentration of 30 mM, again followed by vortexing and centrifugation. For illumination of the pre-gel-solution and subsequent cross-linking, the Prizmatix / Mountain Photonics UHP-T-DI LED was used, a high power LED light source with 5.5 W and a collimated light beam of 460 nm wavelength that is connected to a power control element to adjust the desired intensity. Illumination time, distance to the light source and light intensity were chosen according to the pre-gel-solution's requirements and desired application. The illumination energy per area can be calculated as

$$\frac{E}{A} = I * t * \frac{5.5\,W}{A},$$

where E is the illumination energy, A is the illuminated area, I is the set intensity, and t is the illumination time.

For nanoindentation and dynamic stretch experiments, the pre-gel solution was casted between two microscope slides and illuminated from both sides. To prevent sticking to the glass, the microscope slides were coated with a tetrafluoroethylene hexafluoropropylene copolymer foil.

4. PyMol Measurement

The crystallographic ULD structures prepared in Examples 1-3 was accessed via the Protein Data Bank with accession code 3TUO[2] and loaded into PyMOL v2.3.3, Schrodinger, LLC. Using the dot representation the estimated solvent accessible protein surface was visualized by increasing the van der Waals radii by 1.4 Ångstrøm, the approximate radius of a water molecule "rolling" over the molecule surface. Tyrosine pairs, i.e. two tyrosine residues in close proximity, were identified to visualize solvent (and catalyst) accessible tyrosine pairs.

5. Dynamic Light Scattering (DLS)

A Zetasizer Nano, Malvern Instruments Ltd., was used for DLS measurements. Protein solutions of 2 g/L in MilliQ water or 4M urea were freshly prepared and filtered prior to measuring the size-dependent intensity distribution in order to demonstrate the influence of urea on ULD tetramer formation. As the intensity distribution is weighted according to the scattering intensity and is proportional to the square of the molecular weight, larger particle species give higher intensity signals. Hence, the intensity distribution is a sensitive detector for the presence of larger particles, in this case ULD tetramers, although it does not give an accurate estimate of the concentrations of differently sized particles.

6. Mass spectrometry

a) LC-MS-MS Analysis of tryptic peptides

The sequence identity of ULD-ELP-ULD fusion proteins as prepared before was confirmed using liquid chromatography mass spectrometry (LC-MS/MS) after tryptic digest. In addition, sequence and purity of full-length intact ULD-(VPGVG)40-ULD and ULD-(VPGVG)20-ULD were confirmed by electrospray ionization (ESI-)LC-MS (Figure 1 F and 3). Corresponding ULD-ELP-ULD monomers could exclusively be detected in the presence of at least 4 M urea and 50 % ethanol. In the absence of urea and ethanol, ULD fusion proteins maintained their tetrameric state, monomers could not be detected in LC-MS. Based on the UV 214 nm chromatogram no other protein or peptide impurity was found, confirming close to 100 % target protein purity. The simulated spectra and the measured spectra of both proteins after deconvolution of the multiple charged protein matched perfectly (Figure 1 F). The slight deviation of the measured mass from the predicted mass lies within an acceptable range of 4.9 ppm for ULD-(VPGVG)40-ULD and 12.3 ppm for ULD-(VPGVG)20-ULD. A slight carbamylation of both proteins could be seen, with about 10 % for ULD-(VPGVG)20-ULD and below 10 % for ULD-(VPGVG)40-ULD . For LC-MS/MS analysis samples were prepared by trypsin digestion using a standard protein digestion protocol. Extracted tryptic peptides were separated by LC chromatography (Agilent 1200 SL G1312B system) on C18 column AdvanceBio Peptide Mapping, 2.1 x 150 mm, 2.7 $\mu$m, LC column, kept at 45°C. Peptides were separated over a linear gradient from 10-45 % acetonitrile in 0.1 % formic acid at a flow rate of 200 $\mu$l/min. Including column loading and washing steps, the total time for an LC-MS/MS run was 50 min. Online MS analysis was performed on Impact HD UHR Time-of Flight Mass Spectrometer System ion trap (Bruker Daltonics) equipped with Apollo II ion funnel electrospray source. Voltage applied to capillary corresponded to 4.5 kV, End Plate offset 500 V, Nebulizer of 1.8 bar dry gas 8 l/min and dry temperature 200°C. Peptides were analyzed in positive MS ion mode using an enhanced resolution scan in a mass range of 150 - 2800 m/z, MS-MS/MS cycle time was set to 3 sec with 0.5 sec for MS and 0.25 sec summation time for MS/MS spectra. Singly-charged ions were excluded from fragmentation. MS/MS spectra were obtained using CID fragmentation, recorded in mass range initiating at 150-2800 m/z. The collision energy was adjusted between 23-65 eV as a function of the m/z value. Absolute threshold for MS/MS fragmentation was set to 1755 counts and active exclusion was set to 2 spectra which were released again after 1 min or if peak intensity increased 3-fold. Internal calibration via infusion of ESI Low Concentration Tune Mix was set at the start of the run.

b) Data Processing and Bioinformatic Analysis

Data were analyzed after recalibration using Data Analysis 4.2 SR2, Biotools 3.2 Peptide Editor 3.2 (Bruker Daltonics, Bremen) and MASCOT 2.5 (Matrix Science, London, UK) search engine. MASCOT 2.5 scored peptides for identification based on a search with an initial allowed mass deviation of the precursor ion of up to 15 ppm and allowed fragment mass deviation of 0.05 Da. The search engine was used for the MS/MS spectra search against the UniProtKB E. coli database (downloaded 21 Dec, 2015, containing 4305 entries, 116 contaminants and the added target proteins). The contaminants list used was downloaded from ftp.the pgm.org as of Jan 21, 2016. To determine the number of false positive peptide hits, data were searched against the full-length decoy database using the MASCOT 2.5 percolator function. The false discovery rate was set below 1 % to exclude false positive hits. Enzyme specificity was set as C-terminal to Arg and Lys, also allowing cleavage at proline bonds and a maximum of two missed cleavages. Deamidation of asparagine and glutamine carbamylation of lysine residues and the N-terminus and methionine oxidation were set as variable modifications.

c) LC-MS-MS Analysis of intact proteins

Intact proteins were separated by LC chromatography (Agilent 1200 SL G1312B system) XDB-C8 Zorbax Eclipse 4.6 mm × 30 mm, 3.5 μm, LC column, kept at 60°C. Proteins were separated over a linear acetonitrile gradient in 0.1 % formic acid from 5-24 % over 3 min and 24 - 40% over 30 min at a flow rate of 250 μl/min. Including column loading and washing steps, the total time for an LC-MS/MS run was 66 min. Online MS analysis was performed on Impact HD UHR Time-of Flight Mass Spectrometer System ion trap (Bruker Daltonics) equipped with Apollo II ion funnel electrospray source. Voltage applied to capillary corresponded to 4.5 kV, End Plate offset 500 V, Nebulizer of 1.6 bar dry gas 8 l/min and dry temperature 200°C. Internal calibration via infusion of ESI Low Concentration Tune Mix was set at the start of each run.

d) Data Processing and Bioinformatic Analysis

After automated internal calibration, data were processed and protein peaks were searched based on the 214 nm UV chromatogram and as extracted ion chromatograms. Subsequently, identified protein peaks were deconvoluted in the range of 30,000 - 40,000 Da using Bruker software Maximum Entropy Data Analysis 4.2 SR 2. Based on the given amino acid sequence the average mass and the isotopic pattern of ULD-(VPGVG)20-ULD and ULD-(VPGVG)40-ULD proteins were generated using Bruker data analysis.

7. Dityrosine content

Dityrosine formation was determined by utilizing its autofluorescence properties. Fluorescence spectroscopy was performed using the SpectraMax iD5 Microplate Reader (Molecular Devices, LCC). 6 μl of pre-gel-solution were pipetted in each well of a 384 well plate and subsequently illuminated to cross-link.

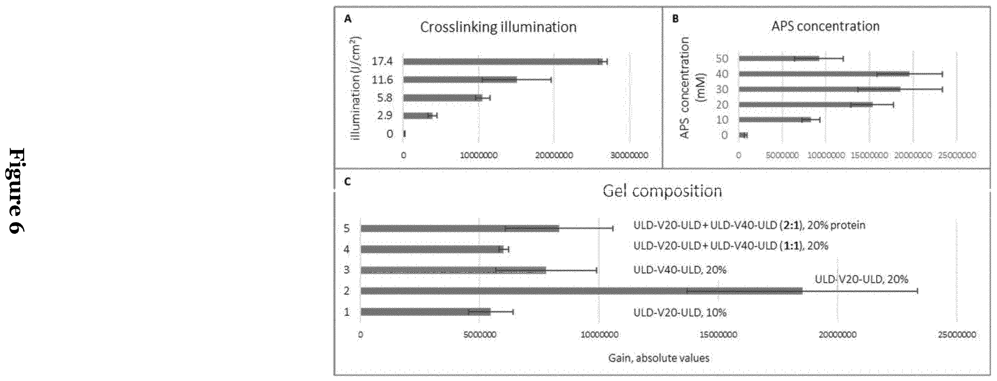

Suitable excitation and emission wavelengths were identified based on a series of emission sweeps at excitation wavelengths of 260 nm to 340 nm with incremental steps of 20 nm (see Figure 6).

8. Characterization of mechanical properties - Dynamic mechanical analysis

One of the major challenges in terms of investigating mechanical properties of hydrogels is the need to perform the respective experiments with the hydrogel being submerged in liquid, as hydrogels become brittle upon drying.

*a) Water content*

19 μl pre-gel-solution was pipetted on a coverslip, placed between two microscope slides with spacers and illuminated from both sides. After letting the gels equilibrate in water for at least one day, we weighed the coverslip with the adherent gel after removing excess water using a lint-free cloth. The gel was then lyophilized, weighed again and removed from the coverslip to determine the coverslip's weight. Water content can be calculated as

$$water\ content\ [\%] = \frac{coverslip\ with\ hydrated\ gel - coverslip\ with\ lyophilized\ gel}{coverslip\ and\ hydrated\ gel - coverslip} * 100\,.$$

*b) Nanoindentation*

For nanoindentation experiments, 19 μl pre-gel-solution was pipetted on a coverslip, placed between two microscope slides with spacers and illuminated from both sides. The fabricated gel pads were 810 μm high and had a diameter of about 5,4 mm. Hydrogel pads on coverslips were measured while being submerged in water at room temperature using the "Bioindenter" (UNHT[3] Bio, Anton Paar GmbH, Peseux, Switzerland) equipped with a ruby spherical tipped indenter (radius 500 μm). A load-controlled mode was chosen with nominal maximum load of 100 - 300 μN and constant loading and unloading rates of 300 μN/min, with or without a hold period of 60 s to observe creep behavior (time-dependent flow of incorporated fluid). The surface contact point was determined automatically, if a stiffness threshold of 2 μN/μm was exceeded and the measured normal force was at least 25 μN at the same time. For every measurement, correct determination of the contact point was confirmed and corrected manually, where necessary. For ULD-(VPGVG)20-ULD (10%, 15% and 20% protein) as well as ULD-(VPGVG)40-ULD and ULD-(VPGVG)80-ULD (20% each), measurements were carried out in triplicates. Every sample was measured at least 15 times in total. For each sample, 5 different surface contact points were determined based on a programmed matrix and 3 indentation measurements were recorded in close proximity of each contact reference (matrix dimensions 1 mm x 1 mm, distance between measurements and respective contact reference 200 $\mu$m).

Measurements were discarded, if no onset was evident (i.e., if measurement started after having already reached the sample surface), or if disturbances were seen (e.g., if measurements were distorted by air bubbles or if the tip slipped off the edge of the gel pad). Young's modulus was analyzed by the built-in Indentation software (v7.2.6) using the Hertz fit method for the loading portion of each load-displacement indentation curve and standard settings (fitting between 10% and 98% of maximum load (Fmax)). The Hertz formula yields the elastic modulus according to the formula:

$$F = \frac{4}{3} E \sqrt{R} * h^{3/2}$$

Where E is the (reduced) elastic modulus, F is the indentation load, R is the radius of the indenter tip, and h is the indentation depth. As the ruby tip is non-compressible, E equals the elastic modulus.

*c) Dynamic Mechanical Analysis*

To determine bulk mechanical properties, uniaxial tensile stretch experiments were carried out for triplicates of ULD-(VPGVG)20-ULD (10% and 20%) and ULD-(VPGVG)40-ULD (20%), cross-linked with 2,5 mM riboflavinphosphate and 30 mM APS. As high amounts of protein were required to cast these gels, proteins were dissolved in 4M urea to ensure fast and homogeneous dissolving. 580 $\mu$m thick gel films were cut to pieces of approx. 10 mm x 5 mm and measured at 37°C in PBS buffer. After placing the gel film between the clamps, the width and length (space between clamps) of the gel were measured. Tensile stretch was then applied in a cyclic manner where the maximum displacement was set to 20% of the measured gel length and the frequency was set to 1 Hz. In order to determine stress and strain at failure, gel pads were stretched until rupture (maximum displacement was set to 5 mm, frequency 0,01 Hz).

Each gel slice underwent cyclic stretching with maximum displacement set to 20 % at a frequency of 1 Hz. Due to the high elasticity of all hydrogels and small dimensions of gel slices, measured force values were low, resulting in a low signal-to-noise ratio. As the recorded signals were of a periodic nature, a denoising process was applied involving fast fourier transform of the recorded force values to identify, separate and remove background and sensor noise. Of the 200 stretching cycles per measurement, subgroups of 50 cycles each were investigated separately to be able to notice changes in time. Young's moduli were calculated based on the slope of the secant connecting the points of minimum stress-strain and maximum stress-strain of each subgroup.

Stress-strain curves for the three hydrogel types are shown in Figure 8. Moreover, the same hydrogel samples were stretched until rupture, where stress and strain were recorded. Extensions to break were recorded at about 250%, showing high elasticity (see Tables S-1 and S-1).

*d) DMA Data Processing and Evaluation*

Both the rupture experiment data and the dynamical stretch data were provided as raw data in csv-format, containing strain data as the independent variable and noisy stress data as the dependent variable. Measured force (stress) values were extremely low, resulting in a low signal-to-noise ratio and requiring a denoising process to allow for data visualization and analysis. Data derived from rupture experiments were denoised using a Savitzky-Golay Filter of window size $n_w$ = 21 and order $n_o$ = 1. This specific filter type was chosen due to its extrema-preserving nature - a desirable characteristic for exact identification of the rupture point. The corresponding exact maximum strain value was then extracted by identifying the maximum index of the denoised stress data and allocating the corresponding strain value.

The sinusoidal dynamic stretch experiment data were denoised by performing a fast fourier transform cancelling out of all spectral parts associated with sensor and background noise. This was achieved by visually inspecting the spectral plots, sorting for descending power density, fixing a small interval of neighboring frequencies around the frequency of highest power magnitude, zeroing all other frequencies and transforming back into time domain. The initial 4000 points were dropped for each data set to discard the mechanical setup phase (including calibration steps) and to allow gel samples to equilibrate in terms of protein chain re-arrangements and entanglements. This resulted in a total of 8000 data points per measurement, summing up to 200 stretching cycles. Subgroups of 50 cycles were isolated from their respective complete datasets and denoised separately.

The fast fourier transformation calculates and returns one single amplitude per sinusoidal frequency. Assuming there was mechanical fatigue present in the unfiltered measurement data, denoising it via fast fourier transformation would have removed any of the corresponding shrinking effects in the measured force data. Therefore, each dataset was split up into 4 subgroups before denoising via fast fourier transformation. This process ensured that possible fatigue, present in the force measurement data, would still be caught. The mean Young's modulus was calculated for each subgroup based on the slope of the secant connecting the zero point and the maximum points in the stress-strain curve. Considering Figure 8, each gel type shows multiple, non-identical ellipsoids, corresponding to the respective subgroups, i.e. "fanning" of ellipsoids around the zero point. This behavior can be explained as follows: real measurement data contains the desired signal of interest, as well as drift and noise. In this specific case, a low frequency response due to the dynamical excitation is of interest. This represents the real mechanical properties of the gel. Even when noise and drift are subtracted, this data can show non-stationary behavior and vary over time, i.e. show small variations in measured stress- values. The DFT decomposes a signal of interest into its spectral parts. It does not preserve the information whether a signal was at some point slightly decaying or increasing, but rather returns an average power density for that spectral part. Applying the DFT to a signal to remove unwanted spectral parts like noise and drift, and transforming back via iDFT will hence eliminate this information and the newly obtained signals will have a constant amplitude.

Therefore, even applying the DFT to two subsequent subgroups will lead to jumps in between subsequent stress-strain curves, i.e. the "fanning" of ellipsoids in Figure 3, if the corresponding real data showed variations in the measured stress values.

Proceeding in time, DMA measurements revealed only slight deviations, both to higher and to lower Young's moduli, giving rise to the assumption that it's not physical fatigue being shown, but rather external effects or a combination thereof. External effects might be caused by gel slices slipping out progressively during the measurement as well as progressively growing fissures, or temperature changes due to insufficient acclimatization of the buffer-filled chamber at measurement start, as the temperature within the chamber cannot be determined. However, after 200 stretch cycles, we did not observe any evidence of material fatigue under the set conditions.

On a macroscopic scale, Young's moduli range from 120 to 137 kPa (with StD of 15 to 54 kPa, see Table S-1 and Table S-2). One reason for the variability between the mechanical properties might be minor posttranslational modifications of ULD monomers. According to MS analysis, the tryptic peptide 67-104 exhibits modifications such as deamidation of the glutamine residues, carbamylation of lysine residues and oxidation of methionine residues to varying extents. These modifications might influence the cross-link reactivity of tyrosine 81 and therefore the homogeneity of the cross-linked protein gel. As presented in Figure 1 B, tyrosine 81 in the USD domain might play the main role in hydrogel cross-linking.

When comparing DMA results, i.e. material properties on a "macroscopic scale", with nanoindentation results ("microscopic scale"), it is evident that elasticity differs considerably between the microscopic and macroscopic scale and material characteristics at both scales describe different processes within the material. Nanoindentation captures very small deformations, mainly attributed to water redistribution within the hydrogel, as well as inhomogeneities within the hydrogel. In contrast, DMA investigates the material's bulk behavior, but is also susceptible to local inhomogeneities potentially leading to micro-cracks that can propagate in time. It should be considered that nanoindentation measurements were performed at ambient temperature, while DMA was performed at 37 °C and respective samples had to be prepared in 4 M urea to ensure fast and homogeneous dissolution of the high amount of protein required.

e) Results

Results are displayed in Tables S-1 and S-2 below:

| | protein conc. | solvent | catalyst | illumination energy (J/cm²) | no. of measurements | Young's modulus (kPa) | StD (kPa) | Young's modulus, average of triplicates (kPa) | StD for average of triplicates (kPa) |
|---|---|---|---|---|---|---|---|---|---|
| ULD-V20-ULD, sample 1 | 10% | water | RF | 25.4 | 15 | 34.35 | 2.33 | | |
| ULD-V20-ULD, sample 2 | 10% | water | RF | 25.4 | 15 | 29.18 | 1.96 | 31.55 | 2.61 |
| ULD-V20-ULD, sample 3 | 10% | water | RF | 25.4 | 15 | 31.12 | 1.83 | | |
| ULD-V20-ULD, sample 1 | 15% | water | RF | 14.1 | 15 | 99.95 | 7.15 | | |
| ULD-V20-ULD, sample 2 | 15% | water | RF | 25.4 | 15 | 85.47 | 3.96 | 100.84 | 15.84 |
| ULD-V20-ULD, sample 3 | 15% | water | RF | 14.1 | 15 | 117.11 | 8.20 | | |
| ULD-V20-ULD, sample 1 | 20% | water | RF | 25.4 | 15 | 134.69 | 7.84 | | |
| ULD-V20-ULD, sample 2 | 20% | water | RF | 25.4 | 14 | 123.85 | 9.58 | 146.62 | 30.54 |
| ULD-V20-ULD, sample 3 | 20% | water | RF | 25.4 | 15 | 181.32 | 10.43 | | |
| ULD-V20-ULD, sample 1 | 10% | water | Ru(II)bpy | 25.4 | 15 | 60.94 | 5.30 | | |

| | | | | | | | | | |
|---|---|---|---|---|---|---|---|---|---|
| ULD-V20-ULD, sample 2 | 10% | water | Ru(II)bpy | 25.4 | 15 | 36.10 | 3.03 | 55.49 | 17.32 |
| ULD-V20-ULD, sample 3 | 10% | water | Ru(II)bpy | 25.4 | 15 | 69.44 | 4.38 | | |
| ULD-V20-ULD, sample 1 | 20% | water | Ru(II)bpy | 25.4 | 15 | 197.19 | 15.49 | | |
| ULD-V20-ULD, sample 2 | 20% | water | Ru(II)bpy | 25.4 | 7 | 214.95 | 40.33 | 210.24 | 11.45 |
| ULD-V20-ULD, sample 3 | 20% | water | Ru(II)bpy | 25.4 | 15 | 218.57 | 16.45 | | |

| | | | | | | | | | |
|---|---|---|---|---|---|---|---|---|---|
| ULD-V40-ULD, sample 1 | 20% | water | RF | 50.7 | 15 | 86.20 | 7.21 | | |
| ULD-V40-ULD, sample 2 | 20% | water | RF | 50.7 | 15 | 71.05 | 11.10 | 82.95 | 10.66 |
| ULD-V40-ULD, sample 3 | 20% | water | RF | 50.7 | 15 | 91.61 | 4.36 | | |
| ULD-V40-ULD, sample 1 | 20% | water | Ru(II)bpy | 50.7 | 15 | 96.46 | 7.77 | | |
| ULD-V40-ULD, sample 2 | 20% | water | Ru(II)bpy | 50.7 | 14 | 108.31 | 5.31 | 100.75 | 6.56 |
| ULD-V40-ULD, sample 3 | 20% | water | Ru(II)bpy | 50.7 | 15 | 97.48 | 5.10 | | |

| | | | | | | | | | |
|---|---|---|---|---|---|---|---|---|---|
| ULD-V80-ULD, sample 1 | 20% | 4M urea | RF | 84.6 | 15 | 107.42 | 4.50 | | |
| ULD-V80-ULD, sample 2 | 20% | 4M urea | RF | 84.6 | 15 | 79.31 | 3.88 | 70.46 | 42.09 |
| ULD-V80-ULD, sample 3 | 20% | 4M urea | RF | 84.6 | 15 | 24.65 | 2.09 | | |
| ULD-V80-ULD, sample 1 | 20% | 4M urea | Ru(II)bpy | 84.6 | 15 | 116.21 | 7.66 | | |
| ULD-V80-ULD, sample 2 | 20% | 4M urea | Ru(II)bpy | 84.6 | 15 | 122.25 | 7.87 | 123.37 | 7.78 |
| ULD-V80-ULD, sample 3 | 20% | 4M urea | Ru(II)bpy | 84.6 | 11 | 131.64 | 30.91 | | |

| | | | | | | | | | |
|---|---|---|---|---|---|---|---|---|---|
| UV20U + UV40U, 1:1, sample 1 | 20% | water | RF | 25.4 | 15 | 98.84 | 4.00 | | |
| UV20U + UV40U, 1:1 sample 2 | 20% | water | RF | 25.4 | 15 | 87.48 | 5.55 | 91.11 | 6.70 |
| UV20U + UV40U, 1:1, sample 3 | 20% | water | RF | 25.4 | 15 | 87.01 | 5.05 | | |
| UV20U + UV40U, 2:1, sample 1 | 20% | water | RF | 25.4 | 15 | 145.81 | 8.21 | | |
| UV20U + UV40U, 2:1, sample 2 | 20% | water | RF | 25.4 | 15 | 149.56 | 10.11 | 136.72 | 19.09 |

| | Protein concentration (%) | prepared in | Illumination energy (J/cm²) | gel dimensions* (mm x mm) | Ultimate tensile strength kPa) | Extension to break | Young's modulus** (kPa) | StD for Young's moduli (kPa) | Young's modulus, average of triplicates (kPa) | StD for average of triplicates (kPa) |
|---|---|---|---|---|---|---|---|---|---|---|
| UV20U + UV40U, 2:1, sample 3 | 20% | water | RF | 25.4 | 15 | 114.78 | 26.96 | | | |

Table S-1: Fabrication parameters of ULD-ELP-ULD hydrogels measured via nanoindentation, number of measurements and resulting Young's moduli.

| | Protein concentration (%) | prepared in | Illumination energy (J/cm²) | gel dimensions* (mm x mm) | Ultimate tensile strength kPa) | Extension to break | Young's modulus** (kPa) | StD for Young's moduli (kPa) | Young's modulus, average of triplicates (kPa) | StD for average of triplicates (kPa) |
|---|---|---|---|---|---|---|---|---|---|---|
| ULD-V20-ULD, sample 1 | 10 | 4M urea | 67.7 | 2 x 5 | | | | 76.23 | 4.15 | | |
| ULD-V20-ULD, sample 2 | 10 | 4M urea | 67.7 | 2 x 5 | | | | 136.32 | 3.50 | 132.08 | 53.86 |
| ULD-V20-ULD, sample 3 | 10 | 4M urea | 67.7 | 1,5 x 5 | 38.16 | 251% | | 183.70 | 3.32 | | |
| ULD-V20-ULD, sample 1 | 20 | 4M urea | 67.7 | 1,5 x 4,5 | | | | 99.68 | 1.30 | | |
| ULD-V20-ULD, sample 2 | 20 | 4M urea | 67.7 | 1,5 x 5 | 85.52 | 185% | | 148.76 | 8.49 | 136.92 | 32.96 |
| ULD-V20-ULD, sample 3 | 20 | 4M urea | 67.7 | 2 x 5 | | | | 162.33 | 8.93 | | |
| ULD-V40-ULD, sample 1 | 20 | 4M urea | 67.7 | 1 x 4 | | | | 125.35 | 4.84 | | |
| ULD-V40-ULD, sample 2 | 20 | 4M urea | 67.7 | 1 x 5 | 70.57 | 256% | | 103.06 | 8.71 | 119.87 | 14.85 |
| ULD-V40-ULD, sample 3 | 20 | 4M urea | 67.7 | 1 x 3,5 | | | | 131.21 | 2.37 | | |

Table S-2: Fabrication parameters of ULD-ELP-ULD hydrogels measured via dynamic mechanical analysis (DMA) and DMA results. *space between the clamps x width of gel strip; ** Mean Young's modulus for each hydrogel sample was calculated as the average mean of Young's moduli of each subgroup of 50 cycles for the respective sample

At the set frequency of 1 Hz, no phase lag could be determined between the sinusoidal functions of force and displacement ($\Delta\Psi \approx 0°$). This finding indicates an excellent gel compliance at a physiologically relevant frequency. After 200 stretch cycles, only slight deviations in the Young's modulus were observed. Proceeding in time, both deviations to higher and to lower Young's moduli have been observed, giving rise to the assumption that it's not physical fatigue being shown, but rather external effects.

Taken together, these results describe a robust, viscoelastic hydrogel material with adequate tensile strength. Material stiffness can be adjusted by varying the applied illumination energy and mixing different building blocks at a given overall protein concentration, in addition to the choice of protein linker sequence and length.

## 9. Sealing of corneal tissue ruptures and stromal defects

For preliminary sealing experiments with human cornea, corneal explants were kindly provided by the Ophthalmology Department of the University Hospital Freiburg. Explants were obtained from donors who had voluntarily agreed to donate organs, including eyes and parts thereof. Explants were only used for these experiments, if their storage time had exceeded the allowed timeframe for subsequent implantation.

## 10. Filling and sealing of cornea defects in porcine eyes

Extracted porcine eyes were obtained from a local butcher and were either frozen and thawn or were used on the same day of slaughter. Fresh eyes were de-epithelialized before the procedure. To prevent liquid from leaking out through the subsequently applied cut, a 1% solution of sodium hyaluronate (Z-HYALIN®) was injected directly underneath the cornea. Next, a paracentesis was set and a small amount of air was injected into the anterior chamber, right behind the incision. Protein aliquots of ULD-(VPGVG)20-ULD and ULD-(VPGVG)40-ULD were dissolved in PBS buffer with 2.5 mM riboflavinphosphate and 30 mM APS, 2 $\mu$l of the freshly prepared pre-gel-solution were pipetted onto the corneal defect and illuminated with a total illumination energy of 5.8 J/cm$^2$, comparable to values that are reported to be applied during keratoconus treatments [9,10]. An infusion bag was connected to an intraocular pressure sensor to measure and control intraocular pressures by varying the height of the infusion bag.

Following in-depth characterization, the protein-based hydrogel demonstrated its power to seal wet body tissues, thereby suggesting its further exploitation as cornea tissue glue, an application with a high medical need.

For corneal sealing experiments, we chose the non-toxic catalyst riboflavinphosphate broadly used for the treatment of keratoconus. UVA irradiation of the photosensitizer riboflavinphosphate results in cross-linking of collagen fibrils and increases corneal rigidity. We demonstrated that irradiation at 460 nm wavelength also yields riboflavin-mediated cross-links, thereby avoiding potential UV-induced cell (DNA) damage.

The inventors inflicted surface defects as well as full-thickness vertical incisions to the de-epithelialized cornea of extracted porcine eyes. An intraocular pressure sensor was installed to control and measure the intraocular pressure. A total illumination energy of 5.8 J/cm$^2$ was applied, an energy comparable to standard keratoconus treatment procedures which are proven to be safe. Of note, hydrogel pads for mechanical characterization were illuminated with up to 50 J/cm$^2$, depending on gel composition. However, exposure of 20 % protein hydrogels to 5.8 J/cm$^2$ with riboflavinphosphate as catalyst sufficed to yield cross-linked, sticky hydrogels. Both ULD-(VPGVG)20-ULD and ULD-(VPGVG)40-ULD hydrogels led to stable fillings and sealings of corneal defects, showing extraordinary adherence to the corneal surface and being able to withstand intraocular pressures of 100 mmHg. ULD-(VPGVG)20-ULD and ULD-(VPGVG)40-ULD hydrogels rather tear apart than detach from the surface once they have been cross-linked (see Figure 13 B (bottom)). At intraocular pressures nearly 100 mmHg, liquid would rather spurt through the paracentesis than breaking the hydrogel sealing. Reference is also made to Figure 14.

The same experiments with ULD-(VPGVG)40-ULD hydrogels were carried in explanted human cornea following the protocol as outlined above (see also Figure 15).

## 11. Re-sealing of human corneal explants

Cornea samples were cut in multiple strips, then each strip was cut in half and both parts were placed a few millimeters apart on a microscope slide or a convex surface. The pre-gel-solution was freshly prepared and 2-4 $\mu$l were pipetted between the both parts of one cornea strip. The pre-gel-solution was illuminated using varying parameters. If the defect was not completely filled, we repeated the procedure with 2-4 $\mu$l pre-gel-solution. The re-sealed cornea strips were incubated in Modified Eagle's Medium (MEM, containing 10% fetal bovine serum and penicillin / streptomycin) for 2 to 3 weeks before preparing cryotome cuts. The cuts underwent hematoxylin-eosin staining and were subsequently scanned to visualize cellular and extracellular matrix structures as well as the hydrogel filling.

Subsequently, we confirmed that ULD-ELP-ULD hydrogels possess the ability to create a bond to human cornea tissue as well. Cornea explants obtained from an eye bank were cut into strips and subsequently re-sealed using hydrogels made of ULD-(VPGVG)20-ULD, ULD-(VPGVG)40-ULD and mixtures thereof. Figure 13 indicates that ULD-(VPGVG)40-ULD hydrogels seem to possess an inner structure that resembles the native corneal structure on the microscopic scale. In contrast, ULD-(VPGVG)20-ULD exhibits a considerably denser inner structure.

After re-sealing of corneal strips, we incubated all samples in Modified Eagle's Medium to determine survival of epithelial and stromal cells (Figure 13). As availability of human cornea from donors is limited, the number of samples for these preliminary experiments is limited and quantitative analyses were not intended. Given the varying shape and height of the cut cornea strips, varying illumination settings had to be employed. The number of epithelial and stromal cells highly varied between different corneas prior to the sealing experiments, probably due to variable storage times. We did not observe any sign of cell toxicity, neither for hydrogels cross-linked with riboflavinphosphate nor with Ru(II)bpy. Figures 13 E and 13 F show a sample with insufficient sealing under these conditions, but a remarkable overgrowth of the ULD-(VPGVG)40-ULD hydrogel by epithelial cells after 2 weeks (Figure 13 E) and 5 weeks of incubation (Figure 13 F). The number of keratinocytes in this sample is also promising, particularly with regard to the high illumination energy used in these experiments.

To determine if the connection between tissue and hydrogel might solely be attributed to riboflavinphosphate and independent from the hydrogel glue, inventors also performed tissue sealing experiments with Ru(II)bpy (Figure 13 D), demonstrating that ULD-(VPGVG)40-ULD coalesces with cornea tissue self-sufficiently. It is noteworthy that multiple layers of ULD-ELP-ULD hydrogels, applied in two steps to fill big gaps between corneal strips and illuminated consecutively, were able to connect to each other (Figure 13 B).

To determine mechanical characteristics of hydrogels cross-linked at the reduced illumination energy of 5.8 $J/cm^2$, inventors compared Young's moduli as determined by nanoindentation. The reduced illumination results in slightly softer hydrogels, with ULD-(VPGVG)20-ULD and ULD-(VPGVG)40-ULD (20% protein each, prepared in PBS buffer, 2.5 mM riboflavinphosphate, 30 mM APS) showing Young's moduli of 96.8 kPa and 45.5 kPa, respectively (StD $\pm$ 22.3 and 25.4 kPa, respectively).

12. Filling and sealing of tissue defects in other organs

Following the same protocol as outlined for Examples 10 and 11 filling and sealing of tissue defects were also carried out in other organs.

Hydrogels based on ULD-(VPGVG)40-ULD (20% protein, prepared in PBS buffer, 2.5 mM riboflavinphosphate, 30 mM APS) were used to seal a lesion in the human kidney and mucosa/stomach. The hydrogel was cross-linked under blue light at 460 nm for 2 minutes (see Figures 16). Moreover, in a further experiment, a lesion in the human kidney and mucosa/stomach was treated with ULD-(VPGVG)40-ULD (2.5 mM riboflavinphosphate, 30 mM APS), wherein the hydrogel was cross-linked under blue light at 460 nm for 30 sec. to 2 minutes (see Figure 17).

Hydrogels based on ULD-(VPGVG)40-ULD (20% protein, prepared in PBS buffer, 2.5 mM riboflavin, 30 mM APS) were also used to sutureless fix/seal a heart valve in the heart of a wild boar with ULD-(VPGVG)40-ULD. The hydrogel was again cross-linked under blue light at 460 nm for 30 sec. to 2 minutes (see Figure 18).

**Claims**

1. Fusion protein for use as an adhesive, tissue glue, filler or sealant for biological tissues, as a vascular sealant, in tissue augmentation, in tissue repair, or as hemostatic agent, the fusion protein having the following formula (I):

$$ULD\text{-}(L)_n\text{-}ULD$$

ULD is an ubiquitin-like-domain selected from any of SEQ ID NO: 1-18 or a sequence, having at least 75% sequence identity to SEQ ID NO: 1-18;
(L) is a peptide or protein linker sequence having 5-1000 aa;
n is an integer selected from 1-100.

2. Fusion protein according to claim 1, wherein (L) is selected from collagen, elastin, elastin-like-protein, fibrin/fibrinogen, HSA, fibronectin, recombinant resilin, spider silk, fluorescent proteins, mEGFP, GFP, monomeric mCherry fluorescent protein, SpyTag/SpyCatcher protein or a peptide or epitope promoting cell adhesion or proteinase-mediated degradation, a protease recognition sequence, a TEVrec protease recognition sequence, a recognition sequence for Protease Factor XA, a recognition sequence for protease Thrombin preferably from a protein sequence of any of SEQ ID NO: 20, 22, 24, 26, 28, 30, 32, 34, 36, 38, 40, or 42 or a sequence, having at least 75% sequence identity, preferably 80 %, 85 %, 90 %, 95 % or even 99 % sequence identity to such a protein sequence.

3. Fusion protein according to claim 1 or 2, wherein (L) is selected from an elastin-like-protein according to SEQ ID NO:

43:

(Val-Pro-Gly-(Xaa)$_m$-Gly)

wherein

m is an integer from 1 to 5, preferably 1-4, more preferably 1-3 or 2, most preferably 1;
Xaa is independently from each other selected from Glu, Asp, Arg, Gly, Ala, Val, Lys, His, Ser, Thr, Asn, Gln, Tyr, Cys, Phe, Ile, Leu, Met or Trp; or is a non- naturally occurring amino acid, or is a mixture thereof.

4. Fusion protein according to claim 3, wherein the elastin-like-protein is selected from any of SEQ ID NO: 44 to SEQ ID NO: 65.

5. Fusion protein according to any of claims 1 to 4, further comprising at the N-terminal and/or C-terminal end of linker sequence (L) a fusion protein linker sequence (FPL), which is an amino acid sequence comprising between 1 and 30 amino acids, preferably, wherein the fusion protein linker sequence (FPL) is selected from a linker sequence sequences, including DPMSSS (SEQ ID NO: 66), GGREASS (SEQ ID NO: 67), (GGGGS)$_{1-4}$ (SEQ ID NO: 68), (Gly$_{1-5,}$), Gly$_1$, Gly$_2$, Gly$_3$, Gly$_4$ or Gly$_5$ (SEQ ID NO: 69), (Gly)$_6$ (SEQ ID NO: 70), (Gly)$_8$ (SEQ ID NO: 71), (EAAAK)$_{1-3}$ (SEQ ID NO: 72), A(EAAAK)$_4$ALEA(EAAAK)$_4$A (SEQ ID NO: 73), PAPAP (SEQ ID NO: 74), AEAAAKEAAAKA (SEQ ID NO: 75), disulfide, VSQTSKLTRAETVFPDV (SEQ ID NO: 76), PLGLWA (SEQ ID NO: 77), RVLAEA (SEQ ID NO: 78), EDVVCCSMSY (SEQ ID NO: 79), GGIEGRGS (SEQ ID NO: 80), TRHRQPRGWE (SEQ ID NO: 81), AGNRVRRSVG (SEQ ID NO: 82), RRRRRRRRR (SEQ ID NO: 83), or GFLGLE (SEQ ID NO: 84), or ENLYFQX, with X = G or S (**SEQ ID NO: 85**), or a sequence, having at least 75% sequence identity, preferably 80 %, 85 %, 90 %, 95 % or even 99 % sequence identity to a nucleic acid sequence according to any of SEQ ID NO: 66 to 85.

6. Fusion protein according to any of claims 1 to 5, wherein the fusion protein further comprises a modification selected from a tag for purification, a protease cleavage site, a chemical cleavage site, interaction or cross-linking sequence, or peptides or epitopes promoting cell adhesion a tag for purification and/or at least one protease cleavage site, preferably selected from a His6-tag (His6), a His10-tag (His10), a FLAG-tag, a Halo-tag, a NE-tag, an ALFA-tag, a Calmodulin-tag, a polyglutamate-tag, an Avi-tag, a C-tag, a polyarginine-tag, an E-tag, an SBP-tag, a TC-tag, an V5-tag, an VSV-tag, a FLAG-tag, a T7-tag, a HA-tag, a Strep-Tag, a MYC tag, a SpyTag/SpyCatcher protein, a SNAP-tag, an isopep-tag, a HUH-tag, a Spot-tag, a Snoop-tag, a Dog-tag, or a Sdy-tag.

7. Fusion protein for use as an adhesive, tissue glue, filler or sealant for biological tissues, as a vascular sealant, in tissue augmentation, in tissue repair, or as a hemostatic agent, the fusion protein having one of the the following formula:

ULD-(Val-Pro-Gly-(Xaa)$_m$-Gly)$_n$-ULD          Formula (Ia)

ULD-(FPL)-(L)$_n$-(FPL)-ULD          Formula (Ib),

or

ULD-(FPL)-(Val-Pro-Gly-(Xaa)$_m$-Gly)$_n$-(FPL)-ULD          Formula (Ic)

wherein

ULD is as defined in any of the claims 1 to 6
(L) is as defined in any of the claims 1 to 6
n is an integer and is selected from 1-100, preferably from 3-90, more preferably from 3-80, even more preferably from 3-65 or from 3-45, e.g. from 10-120, from 15-90, from 15-80, from 15 to 65, 15 to 45 or from 20 to 40;
m is an integer selected from 1 to 5, preferably 1-4, more preferably 1-3 or 2, most preferably 1;
Xaa is independently selected from Glu, Asp, Arg, Gly, Ala, Val, Lys, His, Ser, Thr, Asn, Gln, Tyr, Cys, Phe, Ile, Leu, Met or Trp; or is a non-naturally occurring amino acid, or is a mixture thereof.
(FPL) is an optional amino acid sequence and independently from each other comprises 1 to 30 amino acids, more preferably selected from any of SEQ ID NO: 66 to 85, or a sequence, having at least 75% sequence identity, preferably at least 80 %, at least 85 %, at least 90 %, at least 95 % or even at least 99 % sequence identity to a nucleic acid sequence according to any of SEQ ID NO: 66 to 85.

8. Fusion protein according to claim 7 wherein the fusion protein is a combination of at least two fusion proteins according to any of formulae (I), (Ia), (Ib) and/or (Ic), preferably a combination of a first fusion protein according to any of formulae (I), (Ia), (Ib) and/or (Ic) and a second fusion protein according to any of formulae (I), (Ia), (Ib) and/or (Ic), wherein the first fusion protein and the second fusion protein are present in a ratio of 1000:1-1:1000.

9. Fusion protein according to any of claims 1 to 8, wherein the fusion protein is cross-linked using a cross-linking agent or a cross-linking catalyst, preferably photochemically cross-linked, chemically cross-linked or enzymatically cross-linked, more preferably photochemically cross-linked.

10. Fusion protein according to claim 9, wherein the cross-linking agent or cross-linking catalyst is selected from the group comprising or consisting of riboflavin, riboflavin phosphate, Tris(bipyridine)ruthenium(II) chloride (Ru(II)bpy), 2-hydroxy-4'-(2'-hydroxyethoxy)-2-methylpropiophenone, phenylbis(2,4,6-trimethylbenzoyl)phosphine oxide, methylene blue, multifunctional aldehydes and ketones, glutaraldehyde, haloacetyles, Pyridyl disulfides, NHS-ester, Maleimide crosslinker, peroxidase, laccase, transglutaminase, chlorophyll, preferably wherein the cross-linking reaction is a photochemical cross-linking reaction using as a (photochemical) cross-linking agent or (photochemical) cross-linking catalyst at least one of riboflavin, riboflavin phosphate, Tris(bipyridine)ruthenium(II) chloride (Ru(II)bpy), 2-hydroxy-4'-(2'-hydroxyethoxy)-2-methylpropiophenone, phenylbis(2,4,6-trimethylbenzoyl)phosphine oxide, methylene blue, preferably in the presence of ammonium persulfate (APS).

11. Fusion protein according to any of claims 9 or 10, wherein 1) the fusion protein according to any of formulae (I), (Ia), (Ib) or (Ic), or a combination thereof, and 2) a cross-linking agent or cross-linking catalyst are present in a weight ratio of about 1: 0.000001 to 1:3, e.g. between 1:0.00001 to 1:2, 1:0.0001: to 1:1, or, preferably 1:0.00001: to 1:1.

12. Fusion protein according to any of claims 1 to 11, wherein the fusion protein exhibits a Young's modulus of between 5 kPa and 10 MPa, preferably between 5 kPa and 5 MPa, more preferably between 5 kPa and 1 MPa.

13. Fusion protein according to any of formulae (I), (Ia), (Ib) and/or (Ic), or a combination thereof as defined in any of claims 1 to 12 for use as surgical or medical tool, for use as a (biodegradable) adhesive, tissue glue, filler or sealant for biological tissues, as a vascular sealant, in tissue augmentation, in tissue repair, as hemostatic agents, or for use in open, endoscopic, and laparoscopic surgical procedures, or for use in treating, filling or sealing lesions in a biological tissue or surgical lesions, abrasions, lacerations, or perforations in a patient in need thereof, wherein the fusion protein according to any of formulae (I), (Ia), (Ib) and/or (Ic), or a combination thereof, is preferably cross-linked as defined in any of claims 9-11.

14. Fusion protein for use according to any of claim 13, wherein the fusion protein according to any of formulae (I), (Ia), (Ib) and/or (Ic), or a combination thereof, is mixed prior to application, preferably prior to crosslinking, with a protein sequence as defined for $(L)_n$, preferably selected from collagen, elastin, elastin-like-proteins (ELPs), fibrin, HSA, fibronectin, recombinant resilin, spider silk, mEGFP, GFP, SpyCatcher protein, preferably as defined according to any of SEQ ID NO: 20, 22, 24, 26, 28, 30, 32, 34, 36, 38, 40, or 42 or a sequence, having at least 75% to a protein sequence of any of SEQ ID NO: 20, 22, 24, 26, 28, 30, 32, 34, 36, 38, 40, or 42, or preferably according to a protein sequence selected from any of SEQ ID NO: 43-65.

15. Kit of parts for gluing, sealing or adhering biological tissues together, comprising a fusion protein as defined according to any of claims 1 to 12, a cross-linking agent or cross-linking catalyst as defined according to any of claims 9 to 11, and preferably a medical device for administering the fusion protein and/or the cross-linking agent or cross-linking catalyst, and optionally instructions for use.

**Patentansprüche**

1. Fusionsprotein zur Verwendung als Klebstoff, Gewebekleber, Füllstoff oder Dichtmasse bei biologischen Geweben als vaskuläre Dichtmasse bei der Gewebeaugmentation, Gewebereparatur oder als hämostatisches Mittel, wobei das Fusionsprotein die folgende Formel (I) aufweist:

$$ULD\text{-}(L)_n\text{-}ULD$$

wobei ULD für eine Ubiquitin-ähnliche Domäne steht, die aus einer aus SEQ ID NO: 1-18 oder einer Sequenz mit mindestens 75 % Sequenzidentität zu SEQ ID NO: 1-18 ausgewählt ist;
(L) für eine Peptid- oder Proteinlinkersequenz mit 5-1000 AS steht;
n für eine ganze Zahl steht, die aus 1-100 ausgewählt ist.

2. Fusionsprotein nach Anspruch 1, wobei (L) aus Kollagen, Elastin, Elastinähnlichem Protein, Fibrin/Fibrinogen, HSA, Fibronectin, rekombinantem Resilin, Spinnenseide, Fluoreszenzproteinen, mEGFP, GFP, monomerem mCherry-Fluoreszenzprotein, SpyTag/SpyCatcher-Protein oder einem Zellhaftung oder Proteinase-vermittelten Abbau fördernden Peptid oder Epitop, einer Protease-Erkennungssequenz, einer TEVrec-Protease-Erkennungssequenz, einer Erkennungssequenz für Proteasefaktor XA, einer Erkennungssequenz für Protease-Thrombin, vorzugsweise aus einer Proteinsequenz aus einer aus SEQ ID NO: 20, 22, 24, 26, 28, 30, 32, 34, 36, 38, 40 oder 42 oder einer Sequenz mit mindestens 75 % Sequenzidentität, vorzugsweise 80 %, 85 %, 90 %, 95 % oder sogar 99 % Sequenzidentität zu einer solchen Proteinsequenz ausgewählt ist.

3. Fusionsprotein nach Anspruch 1 oder 2, wobei (L) aus einem Elastin-ähnlichen Protein nach SEQ ID NO: 43:

$$(\text{Val-Pro-Gly-}(\text{Xaa})_m\text{-Gly})$$

ausgewählt ist,
wobei

m für eine ganze Zahl von 1 bis 5, vorzugsweise 1-4, weiter bevorzugt 1-3 oder 2, am meisten bevorzugt für 1 steht;
Xaa unabhängig voneinander aus Glu, Asp, Arg, Gly, Ala, Val, Lys, His, Ser, Thr, Asn, Gln, Tyr, Cys, Phe, Ile, Leu, Met oder Trp ausgewählt ist oder es sich bei Xaa um eine nicht natürlich vorkommende Aminosäure oder eine Mischung davon handelt.

4. Fusionsprotein nach Anspruch 3, wobei das Elastin-ähnliche Protein aus einer aus SEQ ID NO: 44 bis SEQ ID NO: 65 ausgewählt ist.

5. Fusionsprotein nach einem der Ansprüche 1 bis 4, ferner umfassend am N-terminalen und/oder C-terminalen Ende der Linkersequenz (L) eine Fusionsproteinlinkersequenz (FPL), bei der es sich um eine Aminosäuresequenz handelt, die zwischen 1 und 30 Aminosäuren umfasst, vorzugsweise wobei die Fusionsproteinlinkersequenz (FPL) aus Linkersequenzen, einschließlich DPMSSS (SEQ ID NO: 66), GGREASS (SEQ ID NO: 67), $(\text{GGGGS})_{1\text{-}4}$ (SEQ ID NO: 68), $(\text{Gly}_{1\text{-}5},)$, $\text{Gly}_1$, $\text{Gly}_2$, $\text{Gly}_3$, $\text{Gly}_4$ oder $\text{Gly}_5$ (SEQ ID NO: 69), $(\text{Gly})_6$ (SEQ ID NO: 70), $(\text{Gly})_8$ (SEQ ID NO: 71), $(\text{EAAAK})_{1\text{-}3}$ (SEQ ID NO: 72), $\text{A}(\text{EAAAK})_4\text{ALEA}(\text{EAAAK})_4\text{A}$ (SEQ ID NO: 73), PAPAP (SEQ ID NO: 74), AEAAAKEAAAKA (SEQ ID NO: 75), Disulfid, VSQTSKLTRAETVFPDV (SEQ ID NO: 76), PLGLWA (SEQ ID NO: 77), RVLAEA (SEQ ID NO: 78), EDVVCCSMSY (SEQ ID NO: 79), GGIEGRGS (SEQ ID NO: 80), TRHRQPRGWE (SEQ ID NO: 81), AGNRVRRSVG (SEQ ID NO: 82), RRRRRRRRR (SEQ ID NO: 83) oder GFLGLE (SEQ ID NO: 84) oder ENLYFQX, wobei X = G oder S (SEQ ID NO: 85) oder einer Sequenz mit mindestens 75 % Sequenzidentität, vorzugsweise 80 %, 85 %, 90 %, 95 % oder sogar 99 % Sequenzidentität zu einer Nukleinsäuresequenz nach einer aus SEQ ID NO: 66 bis 85 ausgewählt ist.

6. Fusionsprotein nach einem der Ansprüche 1 bis 5, wobei das Fusionsprotein ferner eine Modifikation umfasst, die aus einem Tag zur Aufreinigung, einer Proteasespaltungsstelle, einer chemischen Spaltungsstelle, Interaktions- oder Vernetzungssequenz oder Zellhaftung fördernden Peptiden oder Epitopen, einem Tag zur Aufreinigung und/oder mindestens einer Proteasespaltungsstelle ausgewählt ist, die vorzugsweise aus einem His6-Tag (His6), einem His10-Tag (His10), einem FLAG-Tag, einem Halo-Tag, einem NE-Tag, einem ALFA-Tag, einem Calmodulin-Tag, einem Polyglutamat-Tag, einem Avi-Tag, einem C-Tag, einem Polyarginin-Tag, einem E-Tag, einem SBP-Tag, einem TC-Tag, einem V5-Tag, einem VSV-Tag, einem FLAG-Tag, einem T7-Tag, einem HA-Tag, einem Strep-Tag, einem MYC-Tag, einem SpyTag/SpyCatcher-Protein, einem SNAP-Tag, einem Isopep-Tag, einem HUH-Tag, einem Spot-Tag, einem Snoop-Tag, einem Dog-Tag oder einem Sdy-Tag ausgewählt ist.

7. Fusionsprotein zur Verwendung als Klebstoff, Gewebekleber, Füllstoff oder Dichtmasse bei biologischen Geweben als vaskuläre Dichtmasse bei der Gewebeaugmentation, Gewebereparatur oder als hämostatisches Mittel, wobei das Fusionsprotein eine der folgenden Formeln aufweist:

$$\text{ULD-}(\text{Val-Pro-Gly-}(\text{Xaa})_m\text{-Gly})_n\text{-ULD} \qquad \text{Formula (Ia)}$$

ULD-(FPL)-(L)$_n$-(FPL)-ULD          Formula (Ib)

oder

ULD-(FPL)-(Val-Pro-Gly-(Xaa)$_m$-Gly)$_n$-(FPL)-ULD          Formula (Ic)

wobei

ULD wie in den Ansprüchen 1 bis 6 definiert ist

(L) wie in den Ansprüchen 1 bis 6 definiert ist

n für eine ganze Zahl steht und aus 1-100, vorzugsweise aus 3-90, weiter bevorzugt aus 3-80, noch weiter bevorzugt aus 3-65 oder aus 3-45, z.B. aus 10-120, aus 15-90, aus 15-80, aus 15 bis 65, 15 bis 45 oder aus 20 bis 40 ausgewählt ist;

m für eine ganze Zahl steht, die aus 1 bis 5, vorzugsweise 1-4, weiter bevorzugt 1-3 oder 2, am meisten bevorzugt 1 ausgewählt ist;

Xaa unabhängig voneinander aus Glu, Asp, Arg, Gly, Ala, Val, Lys, His, Ser, Thr, Asn, Gln, Tyr, Cys, Phe, Ile, Leu, Met oder Trp ausgewählt ist oder es sich bei Xaa um eine nicht natürlich vorkommende Aminosäure oder eine Mischung davon handelt.

es sich bei (FPL) um eine optionale Aminosäuresequenz handelt und FPL unabhängig voneinander 1 bis 30 Aminosäuren umfasst, weiter bevorzugt aus einer aus SEQ ID NO: 66 bis 85 oder einer Sequenz mit mindestens 75 % Sequenzidentität, vorzugsweise mindestens 80 %, mindestens 85 %, mindestens 90 %, mindestens 95 % oder sogar mindestens 99 % Sequenzidentität zu einer Nukleinsäuresequenz nach einer aus SEQ ID NO: 66 bis 85 ausgewählt ist.

8. Fusionsprotein nach Anspruch 7, wobei es sich bei dem Fusionsprotein um eine Kombination von mindestens zwei Fusionsproteinen nach einer der Formeln (I), (Ia), (Ib) und/oder (Ic), vorzugsweise eine Kombination von einem ersten Fusionsprotein nach einer der Formeln (I), (Ia), (Ib) und/oder (Ic) und einem zweiten Fusionsprotein nach einer der Formeln (I), (Ia), (Ib) und/oder (Ic) handelt, wobei das erste Fusionsprotein und das zweite Fusionsprotein in einem Verhältnis von 1000:1-1:1000 vorliegen.

9. Fusionsprotein nach einem der Ansprüche 1 bis 8, wobei das Fusionsprotein unter Verwendung eines Vernetzungsmittels oder eines Vernetzungskatalysators vernetzt, vorzugsweise photochemisch vernetzt, chemisch vernetzt oder enzymatisch vernetzt, weiter bevorzugt photochemisch vernetzt wird.

10. Fusionsprotein nach Anspruch 9, wobei das Vernetzungsmittel oder der Vernetzungskatalysator aus der Gruppe ausgewählt ist, die Riboflavin, Riboflavinphosphat, Tris(bipyridin)ruthenium(II)chlorid (Ru(II)bpy), 2-Hydroxy-4'-(2'-hydroxyethoxy)-2-methylpropiophenon, Phenylbis(2,4,6-trimethylbenzoyl)phosphinoxid, Methylenblau, multifunktionelle Aldehyde und Ketone, Glutaraldehyd, Halogenacetyle, Pyridyldisulfide, NHS-Ester, Maleimid-Vernetzer, Peroxidase, Laccase, Transglutaminase, Chlorophyll umfasst oder daraus besteht, vorzugsweise wobei es sich bei der Vernetzungsreaktion um eine photochemische Vernetzungsreaktion unter Verwendung von mindestens einem aus Riboflavin, Riboflavinphosphat, Tris(bipyridin)ruthenium(II)chlorid (Ru(II)bpy), 2-Hydroxy-4'-(2'-hydroxyethoxy)-2-methylpropiophenon, Phenylbis(2,4,6-trimethylbenzoyl)phosphinoxid, Methylenblau, vorzugsweise in Gegenwart von Ammoniumpersulfat (APS), als (photochemisches) Vernetzungsmittel oder (photochemischen) Vernetzungskatalysator handelt.

11. Fusionsprotein nach einem der Ansprüche 9 oder 10, wobei 1) das Fusionsprotein nach einer der Formeln (I), (Ia), (Ib) und/oder (Ic) oder einer Kombination davon und 2) ein Vernetzungsmittel oder Vernetzungskatalysator in einem Gewichtsverhältnis von etwa 1:0,000001 bis 1:3, z.B. zwischen 1:0,00001 bis 1:2, 1:0,0001 bis 1:1 oder vorzugsweise 1:0,00001 bis 1:1 vorliegen.

12. Fusionsprotein nach einem der Ansprüche 1 bis 11, wobei das Fusionsprotein einen Youngschen Modul zwischen 5 kPa und 10 MPa, vorzugsweise zwischen 5 kPa und 5 MPa, weiter bevorzugt zwischen 5 kPa und 1 MPa aufzeigt.

13. Fusionsprotein nach einer der Formeln (I), (Ia), (Ib) und/oder (Ic) oder eine wie in einem der Ansprüche 1 bis 12 definierte Kombination davon zur Verwendung als chirurgisches oder medizinisches Werkzeug, zur Verwendung als (biologisch abbaubaren) Klebstoff, Gewebekleber, Füllstoff oder Dichtmasse bei biologischen Geweben, als vaskuläre Dichtmasse bei der Gewebeaugmentation oder Gewebereparatur, als hämostatisches Mittel oder zur Verwendung bei offenen, endoskopischen und laparoskopischen Operationsverfahren oder zur Verwendung beim Behandeln, Füllen oder Versiegeln von operativen Läsionen, Abschabungen, Platzwunden oder Perforationen bei einem Patienten bzw. einer Patientin, bei dem/der diesbezüglich Bedarf besteht, wobei das Fusionsprotein nach einer der Formeln (I), (Ia), (Ib) und/oder (Ic) oder eine Kombination davon vorzugsweise wie in einem der Ansprüche 9-11 definiert vernetzt wird.

14. Fusionsprotein zur Verwendung nach Anspruch 13, wobei das Fusionsprotein nach einer der Formeln (I), (Ia), (Ib) und/oder (Ic) oder eine Kombination davon vor der Anwendung, vorzugsweise vor dem Vernetzen, mit einer wie für $(L)_n$ definierten Proteinsequenz vermischt wird, die vorzugsweise aus Kollagen, Elastin, Elastin-ähnlichen Proteinen (ELPs), Fibrin, HSA, Fibronectin, rekombinantem Resilin, Spinnenseide, mEGFP, GFP, SpyCatcher-Protein, vorzugsweise wie nach einer aus SEQ ID NO: 20, 22, 24, 26, 28, 30, 32, 34, 36, 38, 40 oder 42 oder einer Sequenz mit mindestens 75 % Sequenzidentität zu einer Proteinsequenz unter einer aus SEQ ID NO: 20, 22, 24, 26, 28, 30, 32, 34, 36, 38, 40 oder 42 oder vorzugsweise nach einer Proteinsequenz, die aus einer aus SEQ ID NO: 43-65 ausgewählt ist, definiert.

15. Kit-of-parts zum Zusammenkleben, zusammen Versiegeln oder Zusammenheften von biologischen Geweben, umfassend ein wie nach einem der Ansprüche 1 bis 12 definiertes Fusionsprotein, ein wie nach einem der Ansprüche 9 bis 11 definiertes Vernetzungsmittel oder einen wie nach einem der Ansprüche 9 bis 11 definierten Vernetzungskatalysator und vorzugsweise eine medizinische Vorrichtung zum Verabreichen des Fusionsproteins und/oder des Vernetzungsmittels oder Vernetzungskatalysators und gegebenenfalls Anweisungen zur Verwendung.

**Revendications**

1. Protéine de fusion pour une utilisation comme adhésif, colle tissulaire, charge ou agent de scellement pour tissus biologiques, comme agent de scellement vasculaire, dans l'augmentation tissulaire, dans la réparation tissulaire ou comme agent hémostatique, la protéine de fusion ayant la formule (I) suivante :

$$\text{ULD-(L)}_n\text{-ULD}$$

ULD est un domaine de type ubiquitine choisi parmi l'une quelconque parmi SEQ ID NO: 1-18 ou une séquence, présentant au moins 75 % d'identité de séquence par rapport à une SEQ ID NO: 1-18 ;
(L) est une séquence de lieur peptidique ou protéique ayant 5-1 000 aa ;
n est un entier choisi parmi 1 à 100.

2. Protéine de fusion selon la revendication 1, dans laquelle (L) est choisi parmi collagène, élastine, protéine de type élastine, fibrine/fibrinogène, HSA, fibronectine, résiline recombinante, soie d'araignée, protéines fluorescentes, mEGFP, GFP, protéine fluorescente mCherry monomérique, protéine SpyTag/SpyCatcher ou un peptide ou épitope favorisant l'adhésion cellulaire ou la dégradation par protéase, une séquence de reconnaissance de protéase, une séquence de reconnaissance de protéase TEVrec, une séquence de reconnaissance pour le facteur XA de protéase, une séquence de reconnaissance pour la protéase thrombine, de préférence parmi une séquence protéique de l'une quelconque parmi SEQ ID NO: 20, 22, 24, 26, 28, 30, 32, 34, 36, 38, 40, ou 42 ou une séquence, ayant au moins 75 % d'identité de séquence, de préférence 80 %, 85 %, 90 %, 95 % ou même 99 % d'identité de séquence avec une telle séquence protéique.

3. Protéine de fusion selon la revendication 1 ou 2, dans laquelle (L) est choisi parmi une protéine de type élastine selon SEQ ID NO: 43:

$$\text{(Val-Pro-Gly-(Xaa)}_m\text{-Gly)}$$

dans laquelle

m est un entier de 1 à 5, de préférence 1 à 4, plus préférablement 1-3 ou 2, de manière préférée entre toutes 1 ;
Xaa est indépendamment les uns des autres choisis parmi Glu, Asp, Arg, Gly, Ala, Val, Lys, His, Ser, Thr, Asn, Gln, Tyr, Cys, Phe, Ile, Leu, Met ou Trp ; ou est un acide aminé d'origine non naturelle ou est un mélange de ceux-ci.

4. Protéine de fusion selon la revendication 3, dans laquelle la protéine de type élastine est choisie parmi l'un quelconque parmi SEQ ID NO: 44 à SEQ ID NO: 65.

5. Protéine de fusion selon l'une quelconque des revendications 1 à 4, comprenant en outre au niveau de l'extrémité N-terminale et/ou C-terminale de la séquence de lieur (I) une séquence de lieur de protéine de fusion (FPL), qui est une séquence d'acides aminés comprenant entre 1 et 30 acides aminés, de préférence, dans laquelle la séquence de lieur de protéine de fusion (FPL) est choisie parmi des séquences de séquence de lieur, y compris DPMSSS (SEQ ID NO: 66), GGREASS (SEQ ID NO: 67), $(GGGGS)_{1-4}$ (SEQ ID NO: 68), $(Gly_{1-5},)$, $Gly_1$, $Gly_2$, $Gly_3$, $Gly_4$ ou $Gly_5$ (SEQ ID NO: 69), $(Gly)_6$ (SEQ ID NO: 70), $(Gly)_8$ (SEQ ID NO: 71), $(EAAAK)_{1-3}$ (SEQ ID NO: 72), $A(EAAAK)_4ALEA(EAAAK)_4A$ (SEQ ID NO: 73), PAPAP (SEQ ID NO: 74), AEAAAKEAAAKA (SEQ ID NO: 75), disulfure, VSQTSKLTRAETVFPDV (SEQ ID NO: 76), PLGLWA (SEQ ID NO: 77), RVLAEA (SEQ ID NO: 78), EDVVCCSMSY (SEQ ID NO: 79), GGIEGRGS (SEQ ID NO: 80), TRHRQPRGWE (SEQ ID NO: 81), AGNRVRRSVG (SEQ ID NO: 82), RRRRRRRRR (SEQ ID NO: 83), ou GFLGLE (SEQ ID NO: 84), ou ENLYFQX, avec X = G ou S (SEQ ID NO: 85), ou une séquence, ayant au moins 75 % d'identité de séquence, de préférence 80 %, 85 %, 90 %, 95 % ou même 99 % d'identité de séquence avec une séquence d'acides nucléiques selon l'une quelconque de SEQ ID NO: 66 à 85.

6. Protéine de fusion selon l'une quelconque des revendications 1 à 5, dans laquelle la protéine de fusion comprend en outre une modification choisie parmi une étiquette pour purification, un site de clivage de protéase, un site de clivage chimique, une séquence d'interaction ou de réticulation, ou des peptides ou épitopes favorisant l'adhésion cellulaire une étiquette pour purification et/ou au moins un site de clivage de protéase, de préférence choisi parmi une étiquette His6 (His6), une étiquette His10 (His10), une étiquette FLAG, une étiquette Halo, une étiquette NE, une étiquette ALFA, une étiquette Calmoduline, une étiquette polyglutamate, une étiquette Avi, une étiquette C, une étiquette polyarginine, une étiquette E, une étiquette SBP, une étiquette TC, une étiquette V5, une étiquette VSV, une étiquette FLAG, une étiquette T7, une étiquette HA, une étiquette Strep, une étiquette MYC, une protéine SpyTag/SpyCatcher, une étiquette SNAP, une étiquette isopep, une étiquette HUH, une étiquette Spot, une étiquette Snoop, une étiquette Dog ou une étiquette Sdy.

7. Protéine de fusion pour une utilisation comme adhésif, colle tissulaire, charge ou agent de scellement pour tissus biologiques, comme agent de scellement vasculaire, dans l'augmentation tissulaire, dans la réparation tissulaire ou comme agent hémostatique, la protéine de fusion ayant l'une des formules suivantes :

$$ULD\text{-}(Val\text{-}Pro\text{-}Gly\text{-}(Xaa)_m\text{-}Gly)_n\text{-}ULD \qquad \text{Formule (Ia)}$$

$$ULD\text{-}(FPL)\text{-}(L)_n\text{-}(FPL)\text{-}ULD \qquad \text{Formule (Ib),}$$

ou

$$ULD\text{-}(FPL)\text{-}(Val\text{-}Pro\text{-}Gly\text{-}(Xaa)_m\text{-}Gly)_n\text{-}(FPL)\text{-}ULD \qquad \text{Formule (Ic)}$$

dans laquelle

ULD est tel que défini dans l'une quelconque des revendications 1 à 6
(L) est tel que défini dans l'une quelconque des revendications 1 à 6
n est un entier et est choisi parmi 1-100, préférablement de 3-90, plus préférablement de 3-80, encore plus préférablement de 3-65 ou de 3-45, p.ex. de 10-120, de 15-90, de 15-80, de 15 à 65, 15 à 45 ou de 20 à 40 ;
m est un entier choisi de 1 à 5, de préférence 1-4, plus préférablement 1-3 ou 2, de manière préférée entre toutes 1 ;
Xaa est indépendamment choisi parmi Glu, Asp, Arg, Gly, Ala, Val, Lys, His, Ser, Thr, Asn, Gln, Tyr, Cys, Phe, Ile, Leu, Met ou Trp ; ou est un acide aminé d'origine non naturelle ou est un mélange de ceux-ci,
(FPL) est une séquence d'acides aminés facultative et comprend indépendamment l'une de l'autre de 1 à 30 acides aminés, plus préférablement choisie parmi l'une quelconque parmi SEQ ID NO: 66 à 85, ou une séquence, ayant au moins 75 % d'identité de séquence, de préférence au moins 80 %, au moins 85 %, au moins 90 %, au moins 95 % ou même au moins 99% d'identité de séquence avec une séquence d'acides nucléiques selon l'une quelconque de SEQ ID NO: 66 à 85.

8. Protéine de fusion selon la revendication 7, dans laquelle la protéine de fusion est une combinaison d'au moins deux protéines de fusion selon l'une quelconque des formules (I), (Ia), (Ib) et/ou (Ic), de préférence une combinaison d'une première protéine de fusion selon l'une quelconque des formules (I), (Ia), (Ib) et/ou (Ic) et d'une seconde protéine de fusion selon l'une quelconque des formules (I), (Ia), (Ib) et/ou (Ic), dans laquelle la première protéine de fusion et la seconde protéine de fusion sont présentes dans un rapport 1 000:1-1:1 000.

9. Protéine de fusion selon l'une quelconque des revendications 1 à 8, dans laquelle la protéine de fusion est réticulée en utilisant un agent de réticulation ou un catalyseur de réticulation, de préférence réticulé photochimiquement, réticulé chimiquement ou réticulé enzymatiquement, de préférence réticulé photochimiquement.

10. Protéine de fusion selon la revendication 9, dans laquelle l'agent de réticulation ou le catalyseur de réticulation est choisi dans le groupe comprenant ou constitué par riboflavine, phosphate de riboflavine, chlorure de tris(bipyridine) ruthénium(II) (Ru(II)bpy), 2-hydroxy-4'-(2'-hydroxyéthoxy)-2-méthylpropiophénone, oxyde de phénylbis(2,4,6-triméthylbenzoyl)phosphine, bleu de méthylène, aldéhydes et cétones multifonctionnel(le)s, glutaraldéhyde, halogénoacétyles, disulfures de pyridyle, ester de NHS, agent de réticulation de maléimide, peroxydase, laccase, transglutaminase, chlorophylle, de préférence dans laquelle la réaction de réticulation est une réaction de réticulation photochimique utilisant comme agent de réticulation (photochimique) ou catalyseur de réticulation (photochimique) au moins l'un parmi riboflavine, phosphate de riboflavine, chlorure de tris(bipyridine)ruthénium(II) (Ru(II)bpy), 2-hydroxy-4'-(2'-hydroxyéthoxy)-2-méthylpropiophénone, oxyde de phénylbis(2,4,6-triméthylbenzoyl)phosphine, bleu de méthylène, de préférence en présence de persulfate d'ammonium (APS).

11. Protéine de fusion selon l'une quelconque des revendications 9 ou 10, dans laquelle 1) la protéine de fusion selon l'une quelconque des formules (I), (Ia), (Ib) ou (Ic), ou une combinaison de celles-ci, et 2) un agent de réticulation ou un catalyseur de réticulation sont présents dans un rapport pondéral d'environ 1:0,000001 à 1:3, par exemple entre 1:0,00001 et 1:2, 1:0,0001 et 1:1, ou, de préférence, 1:0,00001 à 1:1.

12. Protéine de fusion selon l'une quelconque des revendications 1 à 11, dans laquelle la protéine de fusion présente un module de Young compris entre 5 kPa et 10 MPa, de préférence entre 5 kPa et 5 MPa, plus préférablement entre 5 kPa et 1 MPa.

13. Protéine de fusion selon l'une quelconque des formules (I), (Ia), (Ib) et/ou (Ic), ou combinaison de celles-ci telle que définie dans l'une quelconque des revendications 1 à 12, destinée à être utilisée comme outil chirurgical ou médical, pour une utilisation comme adhésif (biodégradable), colle tissulaire, charge ou agent d'étanchéité pour tissus biologiques, comme agent d'étanchéité vasculaire, dans l'augmentation tissulaire, dans la réparation tissulaire, comme agents hémostatiques, ou pour des interventions chirurgicales ouvertes, endoscopiques et laparoscopiques, ou pour une utilisation dans le traitement, le comblement ou la scellement de lésions dans un tissu biologique ou des lésions chirurgicales, des abrasions, des lacérations, ou des perforations chez un patient en ayant besoin, dans laquelle la protéine de fusion selon l'une quelconque des formules (I), (Ia), (Ib) et/ou (Ic), ou une combinaison de celles-ci, est de préférence réticulée tel que défini dans l'une quelconque des revendications 9 à 11.

14. Protéine de fusion destinée à être utilisée selon l'une quelconque de la revendication 13, dans laquelle la protéine de fusion selon l'une quelconque des formules (I), (Ia), (Ib) et/ou (Ic), ou une combinaison de celles-ci, est mélangée avant application, de préférence avant réticulation, avec une séquence protéique telle que définie pour $(L)_n$, de préférence choisie parmi collagène, élastine, protéines de type élastine (ELP), fibrine, HSA, fibronectine, résiline recombinante, soie d'araignée, mEGFP, GFP, protéine SpyCatcher, de préférence tel que défini selon l'une quelconque des SEQ ID NO: 20, 22, 24, 26, 28, 30, 32, 34, 36, 38, 40 ou 42 ou une séquence, ayant au moins 75 % d'identité par rapport à une séquence protéique de l'une quelconque de SEQ ID NO: 20, 22, 24, 26, 28, 30, 32, 34, 36, 38, 40, ou 42, ou de préférence selon une séquence protéique choisie parmi l'une quelconque de SEQ ID NO: 43-65.

15. Kit de pièces pour coller, sceller ou faire adhérer ensemble des tissus biologiques, comprenant une protéine de fusion telle que définie selon l'une quelconque des revendications 1 à 12, un agent de réticulation ou un catalyseur de réticulation tel que défini selon l'une quelconque des revendications 9 à 11, et de préférence un dispositif médical pour administrer la protéine de fusion et/ou l'agent de réticulation ou le catalyseur de réticulation, et éventuellement des instructions d'utilisation.

**Figure 1a**

**Figure 1b**

Figure 1c

| Short name (ULD-ELP-ULD) | | Sequence | Water solubility | MW in kDa |
|---|---|---|---|---|
| ULD-V10-ULD | | ULD-$(VPGVG)_{10}$-ULD-His | +++ | 29,2 |
| ULD-V20-ULD | | ULD-$(VPGVG)_{20}$-ULD-His | ++ | 33,25 |
| ULD-V40-ULD | | ULD-$(VPGVG)_{40}$-ULD-His | + | 41,4 |
| ULD-V80-ULD | | ULD-$(VPGVG)_{80}$-ULD-His | - | 57,8 |
| ULD-D20-ULD | | ULD-$(VPGDG)_{20}$-ULD-His | + | 33,6 |
| ULD-H20-ULD | | ULD-$(VPGHG)_{20}$-ULD-His | ++ | 34,0 |
| ULD-S20-ULD | | ULD-$(VPGSG)_{20}$-ULD-His | ++ | 33,0 |

**Figure 1d, e**

**Figure 1f**

**Figure 2**

**Figure 3**

**Figure 4**

**Figure 5**

**Figure 6**

**Figure 7a, b, c**

Figure 7d, e, f

**Figure 8**

Figure 8 (continued 1)

Figure 8 (continued 2)

**Figure 9**

**Figure 10**

**Figure 11**

**Figure 12**

## Sealing of human cornea explants

| | catalyst | Illum. time | Total illum. energy (J/cm²) |
|---|---|---|---|
| A | riboflavin | 2 min | 68 |
| B | riboflavin | 2 x 1 min | 34 |
| C | riboflavin | 2 x 2 min | 101 |
| D | Ru(II)bpy | 2 x 30 sec | 34 |
| E | riboflavin | 3 min | 101 |
| F | riboflavin | 3 min | 101 |

**Figure 13**

|   | Hydrogel | Catalyst | Illum. time | Illum. energy (J/cm$^2$) |
|---|---|---|---|---|
| A | ULD-V20-ULD, 20% | 0,1 mM ru(II)bpy | 3 min | 101 |
| B | Mix: ULD-V40-ULD & ULD-V20-ULD, 20% (4:1) | 2,5 mM riboflavin | 1 + 1,5 min | 85 |

# Figure 14

**Figure 15**

Figure 16

60

**Figure 17**

**Figure 18**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2018229341 A1
- EP 2668962 A
- EP 2854863 A
- US 10428137 B
- WO 2013178627 A
- WO 201317862 A

**Non-patent literature cited in the description**

- **GE, L.** ; **CHEN, S.** *Polymers (Basel)*, 2020, vol. 12, 939
- **YANG, J. et al.** *Adv. Funct. Mater*, 2020, vol. 30, 1-27
- **BARTON, M. J. et al.** *Neurosurg. Rev*, 2014, vol. 37, 585-595
- **DUARTE, A. P. et al.** *Prog. Polym. Sci.*, 2012, vol. 37, 1031-1050
- **PARK, H. C. et al.** *Expert Rev. Ophthalmol.*, 2011, vol. 6, 631-655
- **PANDEY, N. et al.** *Biomater. Sci.*, 2020, vol. 8, 1240-1255
- **ROSE, J. B. et al.** *Materials (Basel).*, 2014, vol. 7, 3106-3135
- **TONG, A. Y. et al.** *Curr. Opin. Ophthalmol.*, 2018, vol. 29, 14-18
- **WANG, Z. et al.** *Nucleic Acids Res.*, 2012, vol. 40, 4193-4202
- **WANG, Z. et al.** *J. Biol. Chem.*, 2014, vol. 289, 27376-27385
- **KARLIN et al.** *PNAS USA*, 1993, vol. 90, 5873-5877
- **ALTSCHUL et al.** *Nucleic Acids Res.*, 1997, vol. 25, 3389-3402
- **XIAOYING CHEN et al.** *Adv Drug Deliv Rev*, 15 October 2013, vol. 65 (10), 1357-1369
- **HUBER et al.** *Biomaterials*, October 2014, vol. 35 (31), 8767-8779
- **ZHANG X** ; **CHU X** ; **WANG L** ; **WANG H** ; **LIANG G** ; **ZHANG J et al.** Rational Design of a Tetrameric Protein to Enhance Interactions between Self-Assembled Fibers Gives Molecular Hydrogels. *Angew Chemie*, 2012, vol. 124 (18), 4464-8
- **WANG Z** ; **YANG X** ; **CHU X** ; **ZHANG J** ; **ZHOU H** ; **SHEN Y et al.** The structural basis for the oligomerization of the N-terminal domain of SATB1. *Nucleic Acids Res.*, 2012, vol. 40 (9), 4193-202
- **GROTE A** ; **HILLER K** ; **SCHEER M** ; **MÜNCH R** ; **NÖRTEMANN B** ; **HEMPEL DC et al.** JCat: A novel tool to adapt codon usage of a target gene to its potential expression host. *Nucleic Acids Res.*, 2005, vol. 33 (2), 526-31
- **HUBER MC** ; **SCHREIBER A** ; **WILD W** ; **BENZ K** ; **SCHILLER SM.** Introducing a combinatorial DNA-toolbox platform constituting defined protein-based biohybrid-materials. *Biomaterials [Internet*, 2014, vol. 35 (31), 8767-79, http://www.sciencedirect.com/science/article/pii/S0142961214007546
- **QUIROZ, F. G.** ; **CHILKOTI.** *Nat. Mater.*, 2015, vol. 14, 1164-1171